# EUROPEAN PATENT SPECIFICATION

(11) **EP 1 877 403 B1**
(45) Date of publication and mention of the grant of the patent: **30.03.2011**
(21) Application number: 06746245.7
(22) Date of filing: 01.05.2006
(51) Int. Cl.: C07D 405/14, C07D 417/06, C07C 229/26, A61K 31/4196, A61K 31/427, A61P 31/04

(54) **MONO-LYSINE SALTS OF AZOLE COMPOUNDS**
MONOLYSINSALZE VON AZOLVERBINDUNGEN
SELS MONO-LYSINES DE COMPOSES AZOLES

(30) Priority: 03.05.2005 US 676932 P
(43) Date of publication of application: 16.01.2008
(73) Proprietor: Eisai R&D Management Co., Ltd., Tokyo 112-8088 (JP)
(72) Inventor: GAO, Qi, Wallingford, Connecticut 06492 (US); CHEN, Chung-Pin, H., Madison, Connecticut 06443 (US); FAKES, Michael, G., Belle Mead, New Jersey 08502 (US); PENDRI, Yadagiri, R., South Glastonbury, Connecticut 06073 (US); KIAU, Susanne, North Brunswick, New Jersey 08902 (US); VAKKALAGADDA, Blisse, North Brunswick, New Jersey 08902 (US)
(74) Representative: HOFFMANN EITLE
(86) International application number: PCT/JP2006/309435
(87) International publication number: WO 2006/118351

(56) References cited:
- WO-A-01/52852
- WO-A-95/19983
- YASUTSUGA UEDA ET. AL.: "Phosphonooxymethyl Prodrugs of the Broad Spectrum Antifungal Azole, Ravuconazole: Synthesis, and Biological Properties." BIOORGANIC AND MEDICINAL CHEMISTRY LETTERS, vol. 13, 2003, pages 3669-3672, XP002397062
- A. H. GROLL ET. AL.: "Compartmental Pharmacokinetics and Tissue distribution of the Antifungal Triazole Ravuconazole Following Intravenous Administration of its Di-lysine Phosphoester Prodrug (BMS-379224) in Rabbits." JOURNAL OF ANTIMICROBIAL CHEMOTHERAPY, vol. 56, 2005, pages 899-907, XP002397063

## Description

### Technical Field

the present invention relates to novel water-soluble azole compounds which is useful for the treatment of, for instance, serious systemic fungal infections and which is suitable for oral, topical and parenteral administration. More particularly, the present invention relates to novel water-soluble salt prodrugs having the general formula I:

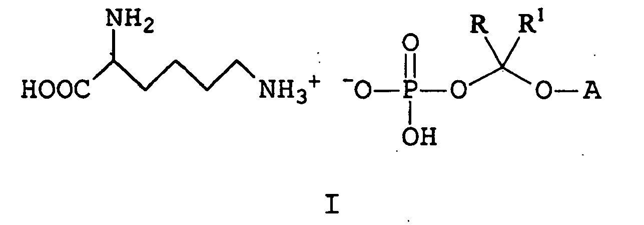

In formula I, each of R and R¹ is a hydrogen or an (C₁-C₆)alkyl group, and A is the non-hydroxy portion of a triazole antifungal compound of the type containing a secondary or tertiary hydroxy group. The present invention also includes pharmaceutically acceptable solvates of the salt compounds of formula I, methods of use and processes of making the same.

### Background Art

Triazole antifungal compounds are known in the art. Of the several classes known, one particularly potent class contains a tertiary hydroxyl group. For example, U.S. Patent No. 5, 648, 372 discloses that the compound of (2R, 3R)-3-[4-(4-cyanophenyl)thiazol-2-yl]-2-(2,4-difluorophenyl)-1-(1*H*-1,2,4-triazol-1-yl)-butan-2-ol has anti-fungal activity. The compound of U.S. Patent No. 5,648,372 is shown below.

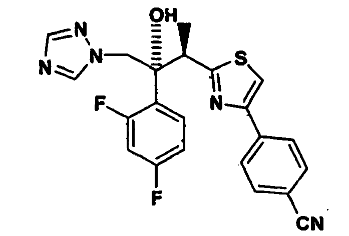

However, the utility of this class of compounds is limited by low water solubility. For example, the solubility of the above triazole compound in water at pH of 6.8 is 0.0006 mg/mL. This greatly impedes developing suitable parenteral dosage forms.

One method of addressing this problem is disclosed in European Patent Application No. 829478, wherein the water solubility of an azole antifungal agent is increased by attaching a linked amino-acid to the azole portion of the molecule (as shown below).

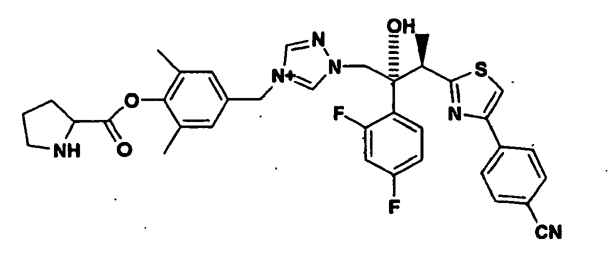

Alternatively, WO 97/28169 discloses that a phosphate moiety can be attached directly to the tertiary hydroxyl portion of the anti-fungal compound, e.g., the compound having the formula shown below.

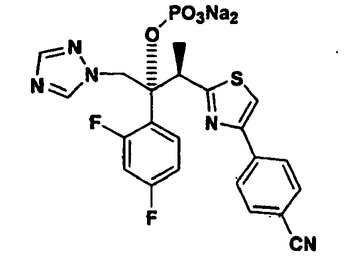

On the other hand, U.S. Patent No. 5,707,977 and WO 95/19983 disclose water-soluble prodrugs having the general formula shown below.

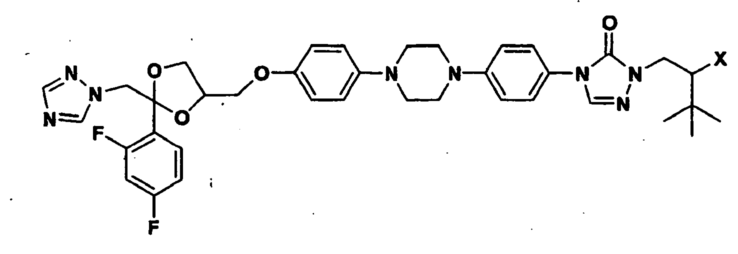

In the above formula, X is OP (O) (OH)₂ or an easily hydrolyzable ester OC (O) RNR¹R².

In contrast, WO 95/17407 discloses water-soluble azole prodrugs of the general formula shown below.

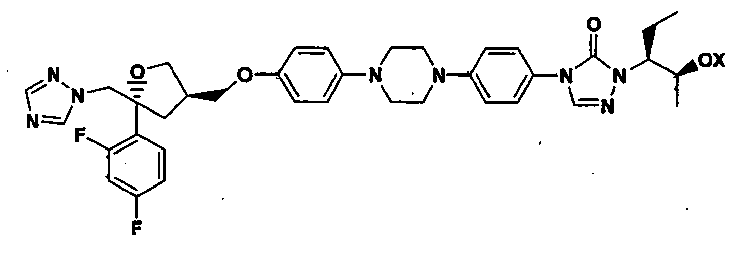

In the above formula, X is P(O) (OH)₂, C (O) -(CHR¹) ₙ-OP (O) (OH)₂ or C (O) - (CHR¹)ₙ- (OCHR¹CHR¹)ₘOR².

Other azole compounds have been proposed. For instance, WO 96/38443 discloses water-soluble azole prodrugs of the general formula shown below.

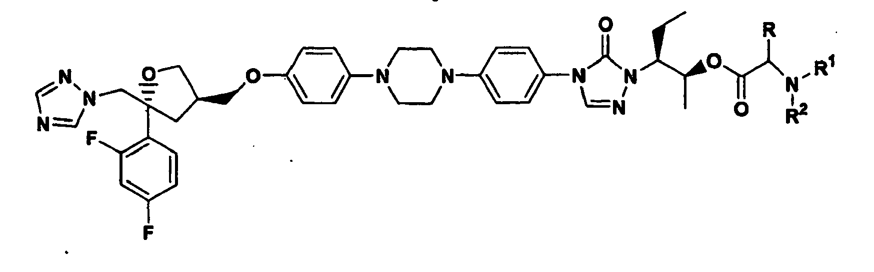

U.S. Patent 5,883,097 discloses water-soluble amino acid azole prodrugs, such as the glycine ester, as shown below.

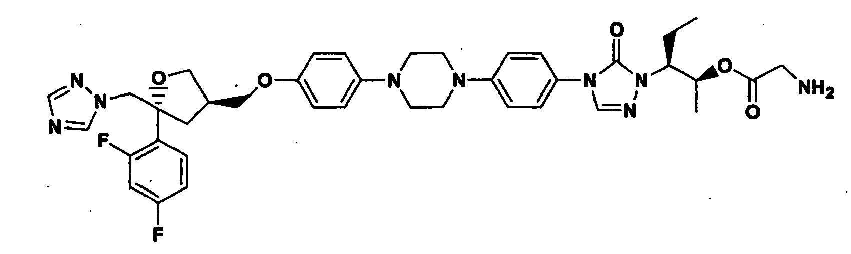

The introduction of the phosphonooxymethyl moiety into hydroxyl containing drugs is disclosed as a method to prepare water-soluble prodrugs of hydroxyl containing drugs.

European Patent Application No. 604910 discloses phosphonooxymethyl taxane derivatives of the general formula as shown below.

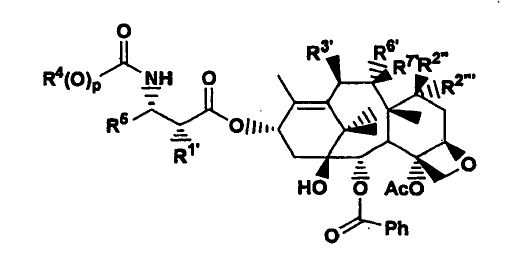

In the above formula, at least one of R^{1'}, R^{2"}, R^{3'}, R^{6'} or R^{7'} is OCH₂OP (O) (OH)₂.

European Patent Application No. 639577 discloses phosphonooxymethyl taxane derivatives of the formula T-[OCH₂(OCH₂)ₘOP(O)(OH)₂]ₙ, wherein T in the formula is a taxane moiety bearing on the C13 carbon atom a substituted 3-amino-2-hydroxypropanoyloxy group, n is 1, 2 or 3, m is 0 or an integer from 1 to 6 inclusive.

WO 99/38873 discloses O-phosphonooxymethyl ether prodrugs of a diaryl 1,3,4-oxadiazolone potassium channel opener.

Golik, J. et al., Bioorganic & Medicinal Chemistry Letters, Vol. 6, pp. 1837-1842 (1996) discloses novel water-soluble prodrugs of paclitaxel, such as the one shown below.

U.S. Patent No.. 6,362,172 discloses water-soluble azole prodrugs having the general formula shown below.

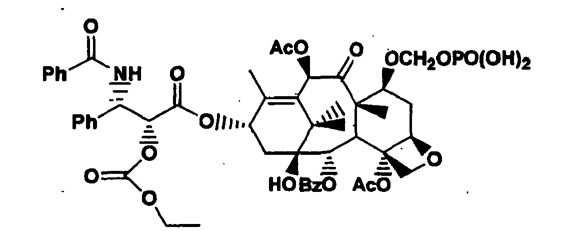

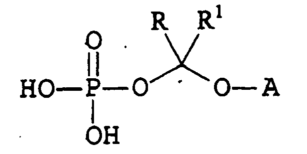

In the above formula, A is the non-hydroxy portion of a triazole antifungal compound of the type containing a secondary or tertiary hydroxy group, R and R¹ are each independently hydrogen or (C₁-C₆)alkyl.

However, the prodrugs of U.S. Patent No. 6,362,172 cannot easily be used for oral administration. WO 01/52852 A1 is directed to water soluble prodrugs of triazole antifungal compounds having a secondary or tertiary hydroxy group. The water soluble triazole antifungal compounds have the general formula I 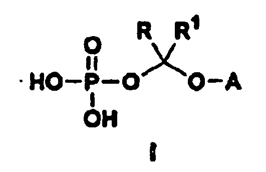 wherein A is the non-hydroxy portion of a triazole antifungal compound of the type containing a secondary or tertiary hydroxy group and R and R1 are each independently hydrogen or (C1-C6)alkyl.

Ueda et al. (Bioorganic & Medicinal Chemistry Letters 13, 2003, 3669-3672) reports about the synthesis and biological properties of phosphonooxymethyl derivatives of Ravuconazole.

### Disclosure OF Invention

It has now been found that mono-lysine salts of triazole anti-fungal phosphate compounds containing a secondary or tertiary hydroxyl group, including ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate, has unexpectedly superior properties to those previously disclosed. Specifically, the present invention relates to mono-lysine salts of compounds, or pharmaceutically acceptable solvates thereof, of the formula I:

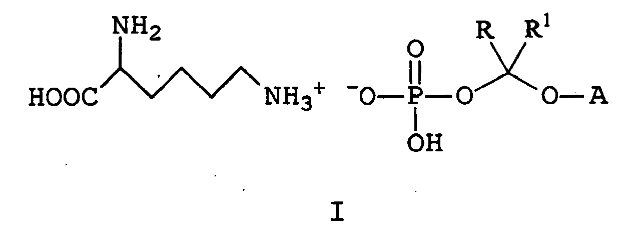

In formula I, each of R and R¹ is a hydrogen atom or a (C₁-C₆)alkyl group, preferably one or both being hydrogen. Also in formula I, A represents the non-hydroxy portion of a triazole antifungal salt compound of the type containing a secondary or tertiary hydroxy group.

Preferred among the compounds of formula I are those wherein A represents the non-hydroxy portion of a triazole antifungal compound of the type containing a tertiary hydroxy group.

In a further embodiment of the above type salt compounds, A can be formula (i): 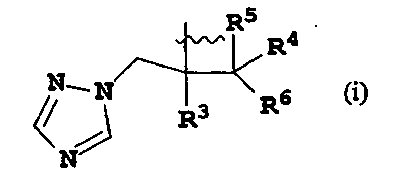 wherein R³ of formula (i) represents a phenyl group substituted by one or more (preferably 1-3) halogen atoms; R⁴ represents a hydrogen or methyl (CH₃); R⁵ represents a hydrogen, or taken together with R⁴ may represent =CH₂; R⁶ represents a 5- or 6-membered nitrogen-containing ring which may be optionally substituted by one or more groups selected from a halogen, =O, CH=CH-(C₆H₄)-OCH₂CF₂CHF₂, and a phenyl substituted by one or more groups selected from CN and OCH₂CF₂CHF₂, or a phenyl substituted by one or more groups selected from a halogen and methylpyrazolyl.

When R⁶ represents a nitrogen-containing heterocycle, such examples include triazolyl, pyrimidinyl, and thiazolyl, wherein each ring is optionally substituted by one or more groups selected from the group consisting of a halogen, =O, CH=CH-(C₆H₄)-OCH₂CF₂CHF₂, and a phenyl substituted by one or more groups selected from the group consisting of CN and OCH₂CF₂CHF₂, or a phenyl substituted by one or more groups selected from the group consisting of a halogen and methylpyrazolyl.

Examples of A include, but are not limited to, the following: 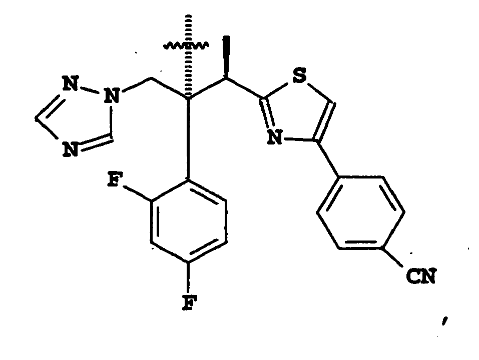 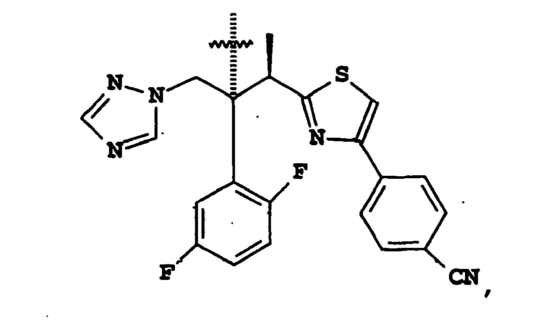 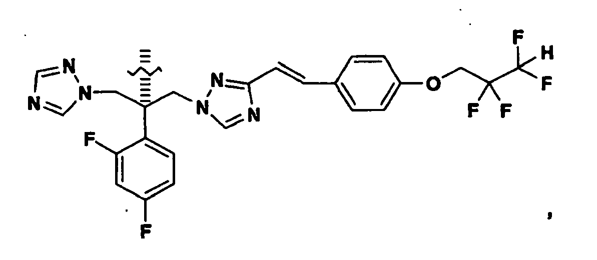 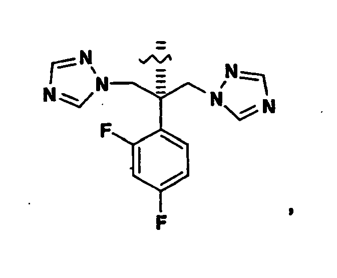 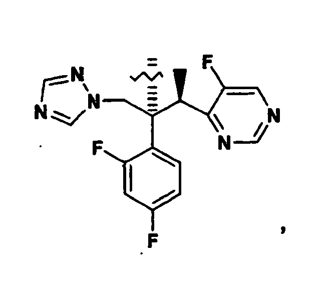 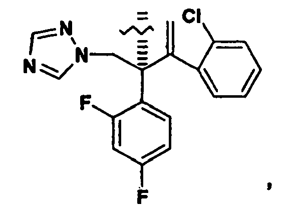 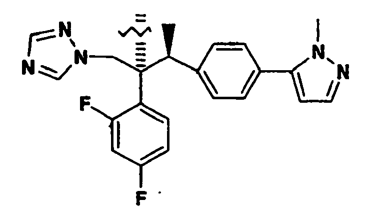 and

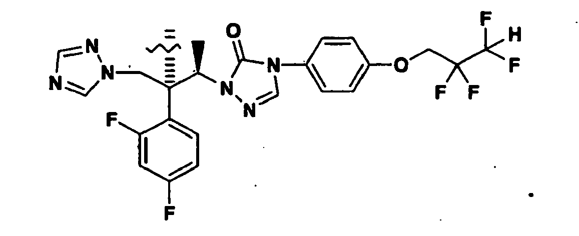

Of those above specific compounds, the following are preferred embodiments: 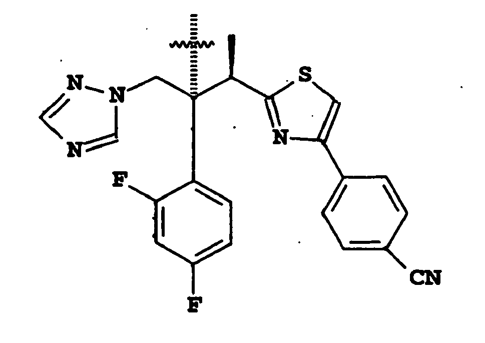 and

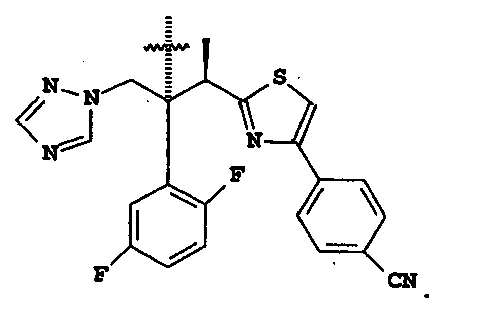

A more preferred embodiment of the mono-lysine salt compound of formula I has the structure as shown below.

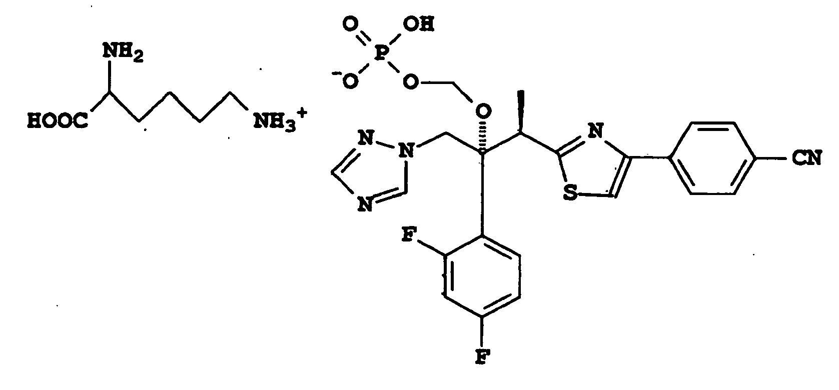

Solvate forms of the salt compounds of formula I are also further embodiments of the present invention.

In addition to the application of the present invention to structures containing a tertiary alcohol, it should also be understood that this discovery can be applied to anti-fungal ingredients which contain secondary alcohols. Some examples of the non-hydroxy portion of triazole antifungal salt compounds of the type containing a secondary hydroxy group include, but are not limited to, the following:

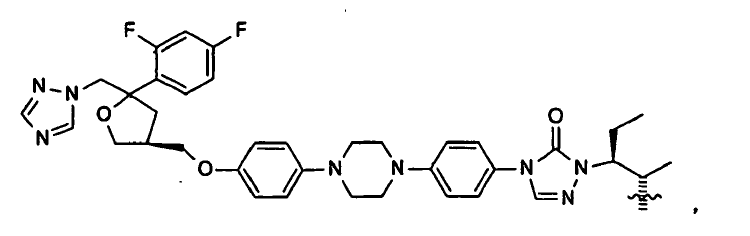

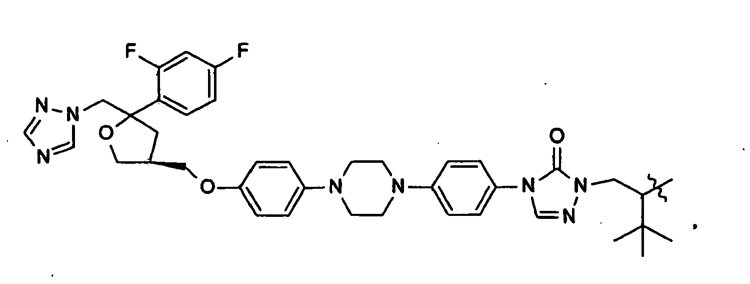

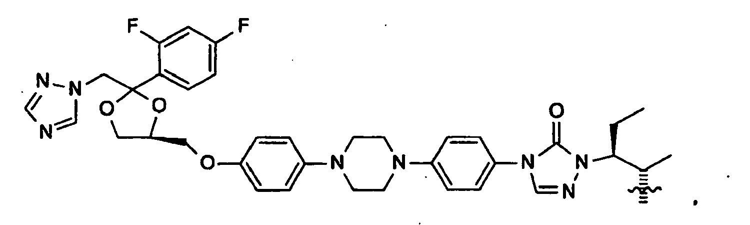

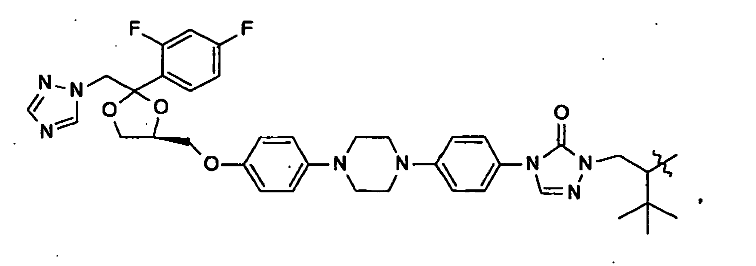

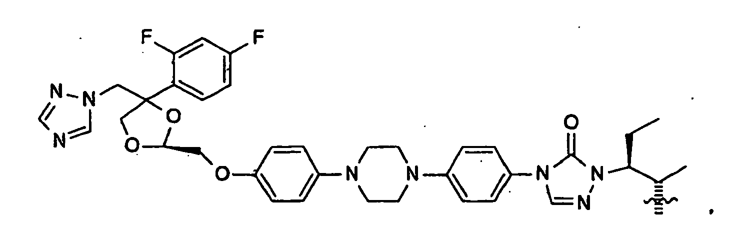

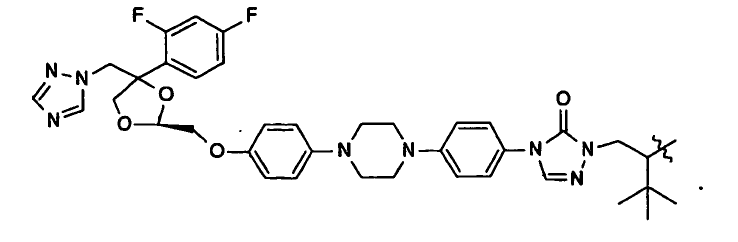

### Brief Description of Drawings

Figure 1 shows moisture uptake properties of bis-lysine ethanol solvate of (2*R*,3*R*)-3-(4-(4-cyanophenyl)thiazol-2-yl)-2-(2,4-difluorophenyl)-1-(1*H*-1,2,4-triazol-1-yl)-2-[(dihydrogen phosphonoxy)methoxy]butane.
Figure 2 shows moisture uptake properties of mono-lysine ethanol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate.
Figure 3 shows moisture uptake properties of mono-lysine isopropyl alcohol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate.
Figure 4 shows moisture uptake properties of a mono-lysine salt relative to a bis-lysine salt.
Figure 5 is a graph for the powder X-ray diffraction (PXRD) data obtained for the mono-lysine ethanol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate.
Figures 6A-6C are graphs pertaining to the NMR data for the mono-lysine ethanol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate.
Figure 7 is a differential scanning calorimetry (DSC) curve the mono-lysine ethanol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate.
Figure 8 is a thermal gravimetric analysis (TGA) curve for the mono-lysine ethanol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate.
Figure 9 is a graph for the PXRD data obtained for the mono-lysine isopropyl alcohol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate.
Figure 10 is a DSC curve for the mono-lysine isopropyl alcohol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate.
Figure 11 is a TGA curve for the mono-lysine isopropyl alcohol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate.
Figure 12 is a graph for the PXRD data obtained for the mono-lysine n-propyl alcohol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate.
Figure 13 is a DSC curve for the mono-lysine n-propyl alcohol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate.
Figure 14 is a TGA curve for the mono-lysine n-propyl alcohol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate.

### Best Mode for Carrying Out the Invention

The mono-lysine salt compounds of general formula I function as "prodrugs" when administered *in vivo*, being converted to the biologically active parent azole in the presence of alkaline phosphatase. Also, the mono-lysine salt compounds of general formula I have unexpectedly improved physical stability with low hygroscopicity, which leads to better handling during manufacture, while maintaining suitable solubility, making the prodrugs suitable for oral, topical and parenteral uses.

The mono-lysine salt compounds of the present invention can be hydrates, solvates or non-solvates. Crystalline structures of several isostructural solvate forms are also possible. For instance, such solvate forms include those derived from water, ethanol, methanol, isopropyl alcohol and n-propyl alcohol. Further, crystal polymorphs of the mono-lysine salt or solvate thereof of the present invention are also possible.

Preferable are solvates of those compounds when A is: 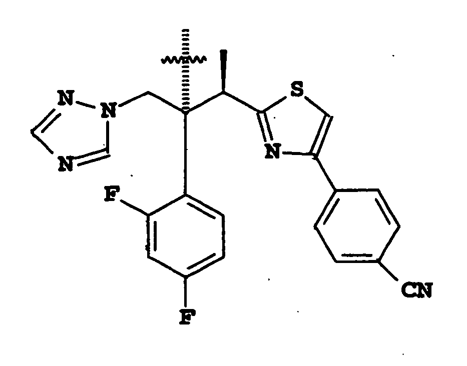 or 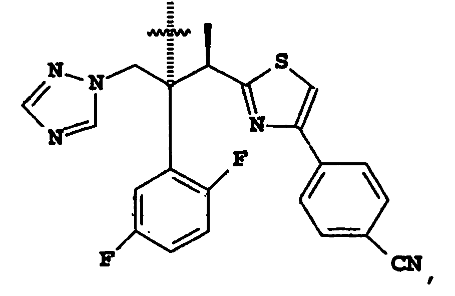 wherein the first of these compounds is most preferred.

The mono-lysine salts of the present invention can be obtained as crystalline solids of high purity with unexpectedly good solubility and low hygroscopicity, which leads to improved handling as compared to bis-lysine salts of the same compounds. For instance, the mono-lysine salt of the present invention can be a crystallized salt of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate.

As used herein "(C₁-C₆)alkyl" refers to a straight or branched chain saturated aliphatic group having 1 to 6 carbon atoms, such as methyl, ethyl, n-propyl, isopropyl, n-butyl, sec-butyl, t-butyl, n-pentyl, n-hexyl, etc.

The term "halogen" as used herein includes chloro, bromo, fluoro and iodo, and is preferably chloro or fluoro, and most preferably fluoro.

As mentioned, each of R and R¹ of formula I can be a hydrogen atom or an alkyl group having one to six carbon atoms in length. For instance, R and/or R¹ can be a methyl or ethyl group. Preferably, each of R and R¹ of formula I represents hydrogen.

Also, R³-R⁶ of formula (i) can be several possible substituents. In one embodiment, the mono-lysine salt or solvate thereof has R³ being 2,4-difluorophenyl. In another embodiment, R⁴ of the mono-lysine salt or solvate thereof is methyl when R⁵ is a hydrogen atom. In still a further embodiment, R⁶ of formula (i) is 4-(4-cyanophenyl)-thiazol-2-yl.

A further embodiment of the present invention is a mono-lysine salt of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate, or a pharmaceutically acceptable solvate thereof, as shown below.

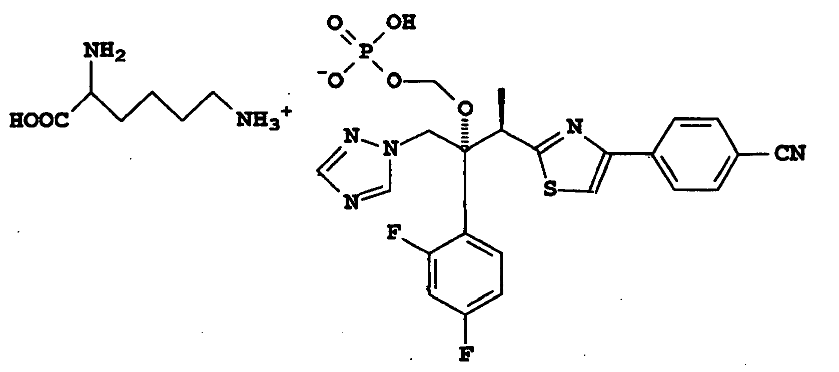

The mono-lysine salts and solvates of the present invention may be in crystalline form, and used in a pharmaceutical composition in the form of a tablet, capsule, powder, solution, suspension, emulsion, ointment, lotion, cream or spray. For instance, a prodrug can comprise the crystalline ethanol solvate of the mono-lysine salt compound.

Also, the mono-lysine salt or solvate of formula I surprisingly maintains its aqueous solubility relative to the bis-lysine salt form thereof, and has unexpectedly improved handling due to low hygroscopicity, which enables it to be used for oral administration as well as parenteral administration.

Further, the mono-lysine salts and solvates thereof exhibit improved handling during manufacture and compaction (compression behavior) relative to bis-lysine salts and are thus suitable for solid dosage forms (i.e., a tablet). It was found that the mono-lysine salt has a lower cohesion index compared to the bis-lysine salt form and similar cohesion index relative to the parent compound (i.e., Ravuconazole). The mono-lysine salts and solvates thereof also exhibit higher bulk and tab density when compared to the bis-lysine form. Thus, the mono-lysine form is viable for compaction (into tablets), has reduced drug loading, and has the further advantage of less sensitivity to high humidity conditions (with or without a coating) versus the bis-lysine form. Further, the mono-lysine salts and solvates thereof are stable in solution (both as a drug substance and in formulation), can be isolated in crystalline form and are readily converted to the parent drug *in vivo*.

The mono-lysine salt and solvate thereof also exhibit better solid state stability. Solid state stability herein means the stability of the API under ambient and/or accelerated storage conditions. For instance, the mono-lysine salt and solvate thereof has improved, better handling, with lower moisture uptake (and its extent) compared to the bis-lysine. Such properties also lead to better handling and long term stability (lower moisture; less degradation; etc.).

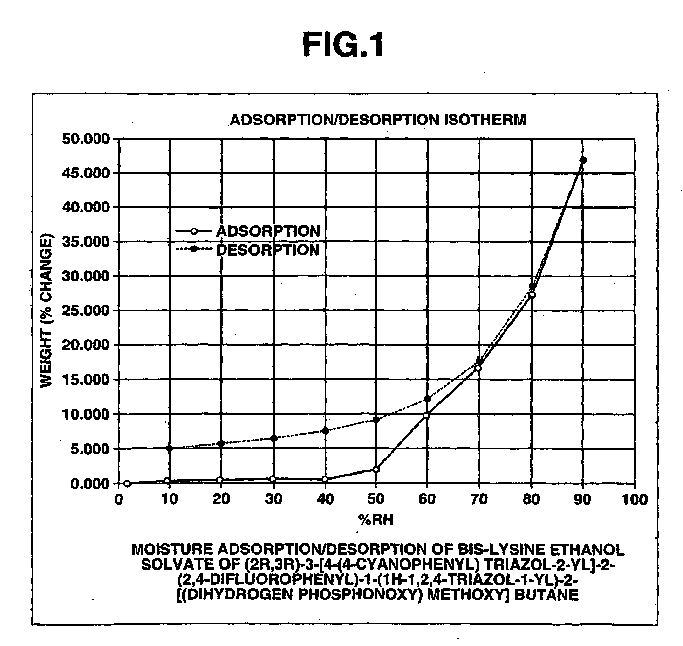

The moisture uptake behavior of the mono-lysine salt or solvate thereof is comparable to that of the corresponding bis-lysine salt or solvate thereof at lower RH values (e.g., 0% up to 50%RH), but is surprisingly much lower at the higher RH values above 50%RH (e.g., 2-3% change in weight at 60%RH for mono-lysine monoethanolate relative to 10% change in weight for bis-lysine monoethanolate). Compared to the bis-lysine salt compound, the mono-lysine salt compound has unexpectedly improved handling and moisture uptake at high relative humidity and at high temperatures as can be seen in Figures 1-4.
Figure 1 shows the moisture uptake of bis-lysine ethanol solvate of (2R,3R)-3-[4-(4-cyanophenyl)thiazol-2-yl]-2-(2,4-difluorophenyl)-1-(1*H*-1,2,4-triazol-1-yl)-2-[(dihydrogen phosphonoxy)methoxy]butane. Weight change (y-axis) versus relative humidity (x-axis) is shown, wherein adsorption is shown by -o-, and desorption by -•-.

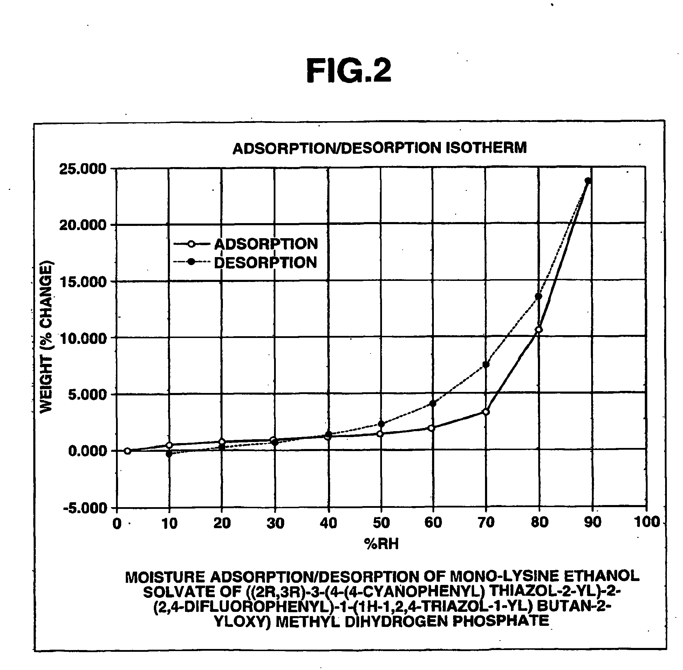

Figure 2 shows moisture uptake of mono-lysine ethanol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate, wherein weight change (y-axis) versus relative humidity (x-axis) is shown, wherein adsorption is shown by -o-, and desorption by -●-.

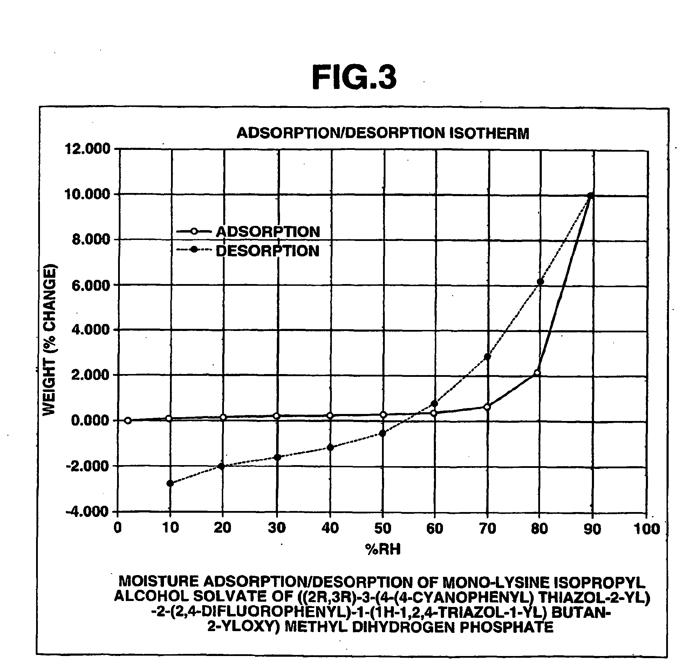

Figure 3 shows moisture uptake of mono-lysine isopropyl alcohol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate, wherein weight change (y-axis) versus relative humidity (x-axis) is shown, wherein adsorption is shown by -o-, and desorption by -●-.

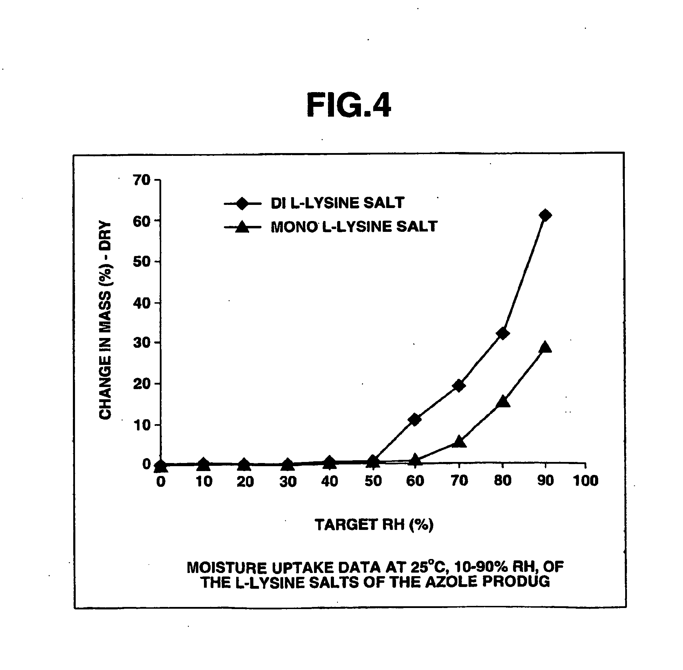

Figure 4 shows moisture uptake data between a mono-lysine salt compared to the bis-lysine form (also known as di-lysine), wherein weight change (y-axis) versus relative humidity (x-axis) is shown, the bis-lysine form represented by -◆-, and the mono-lysine form represented by -▲-.

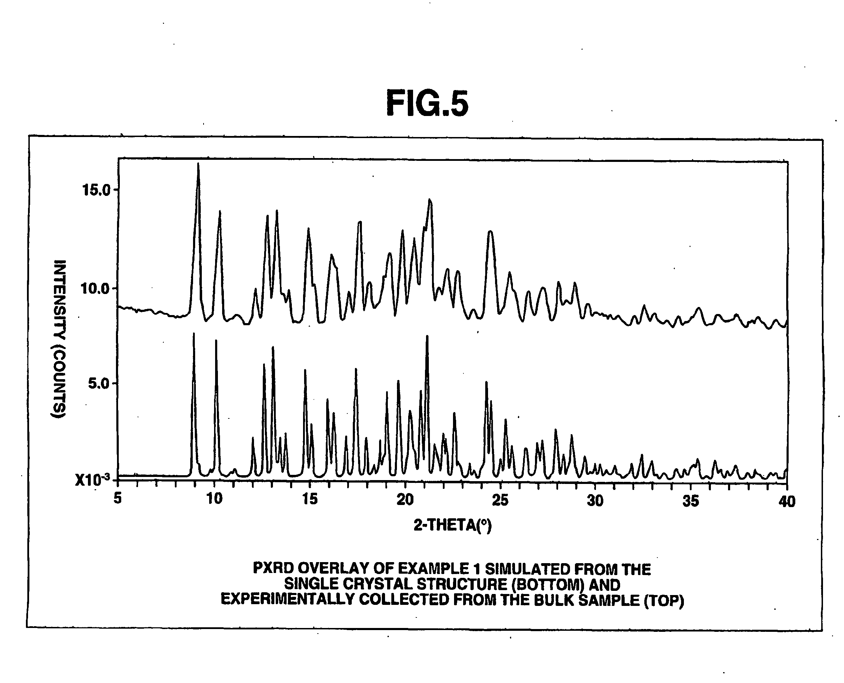

Figure 5 depicts the PXRD overlay of the mono-lysine ethanol solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate (Example 1), simulated from the single crystal structure versus what was experimentally collected from the bulk sample.

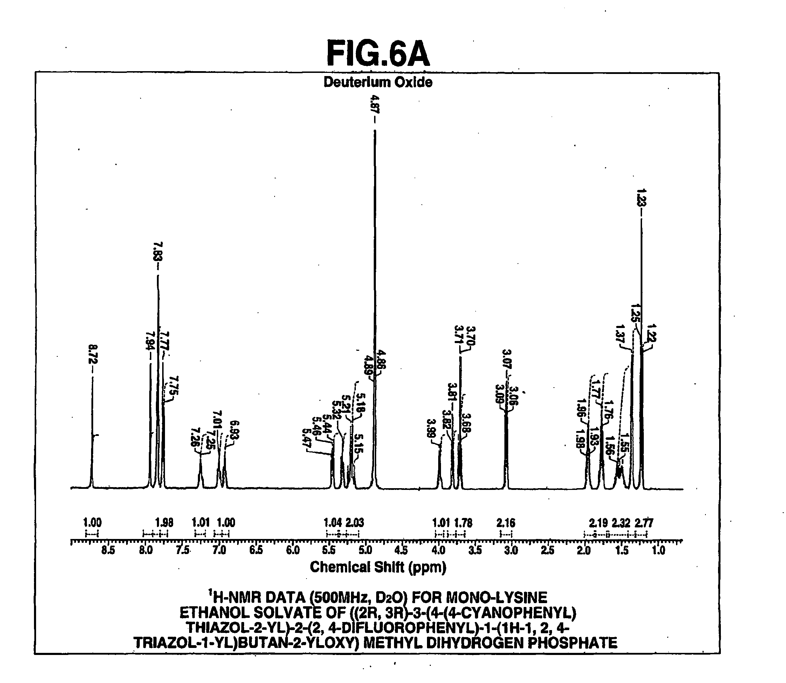

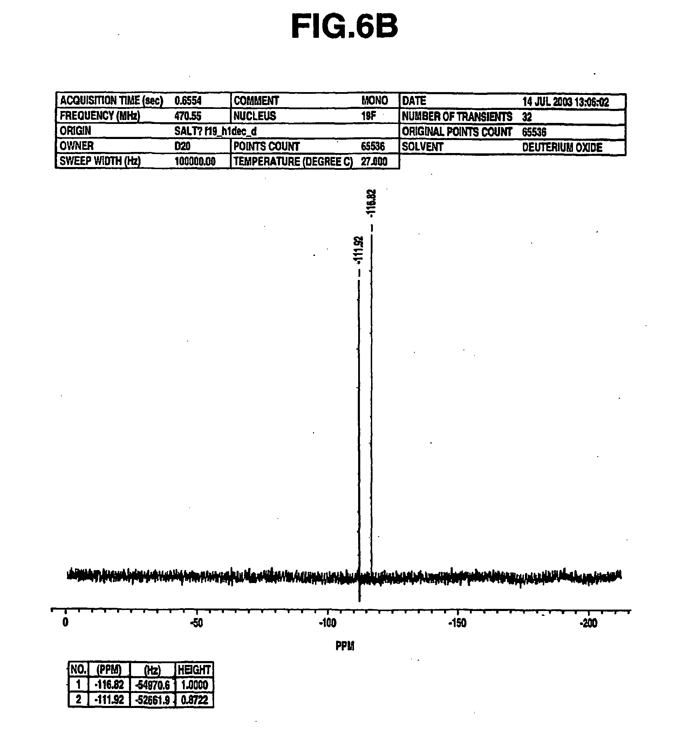

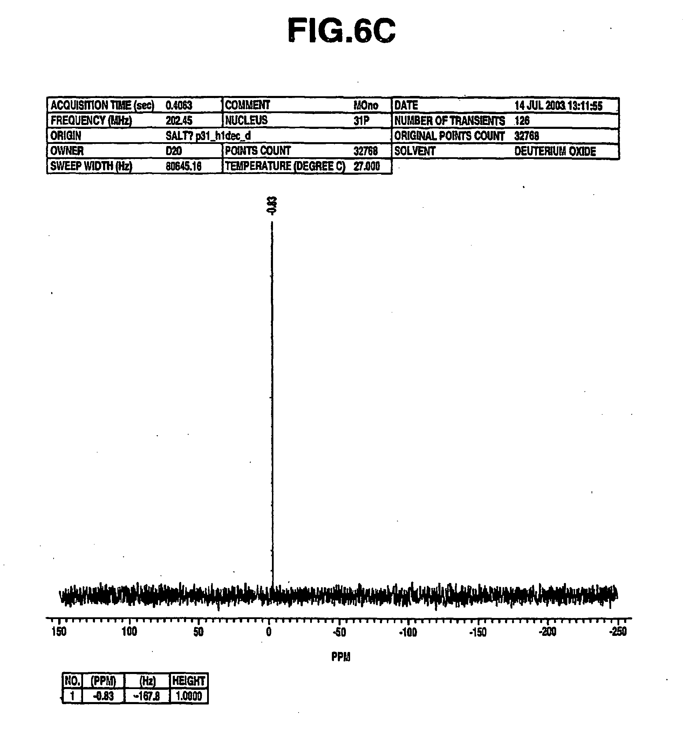

Figures 6A-6C pertain to the nuclear magnetic resonance data for Example 1. Fig. 6A pertains to H-1 NMR data; Fig. 6B pertains to the F-19 NMR data; and Fig. 6C pertains to the P-31 NMR data.

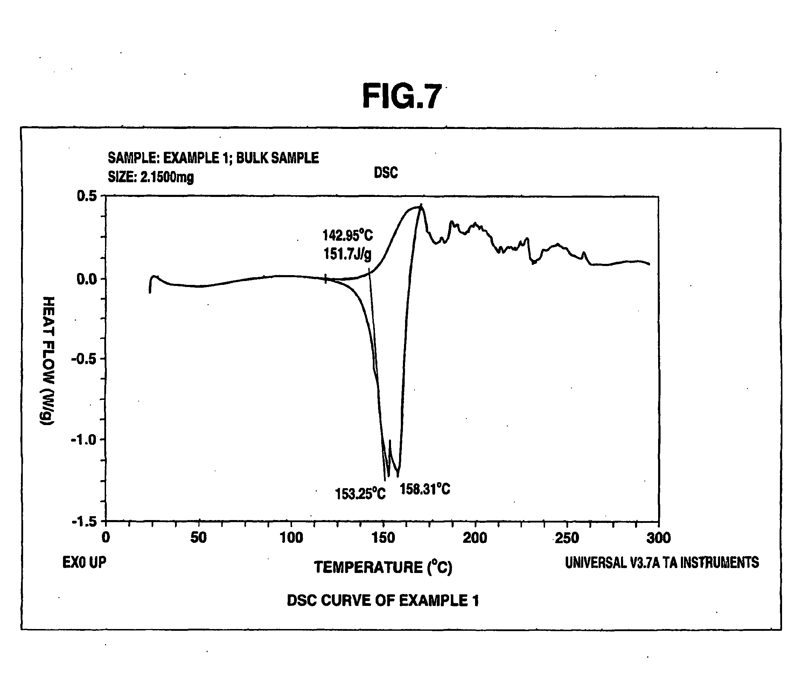

Figure 7 is a DSC curve for Example 1, wherein heat flow (W/g) is the y-axis and temperature (°C) is the x-axis.

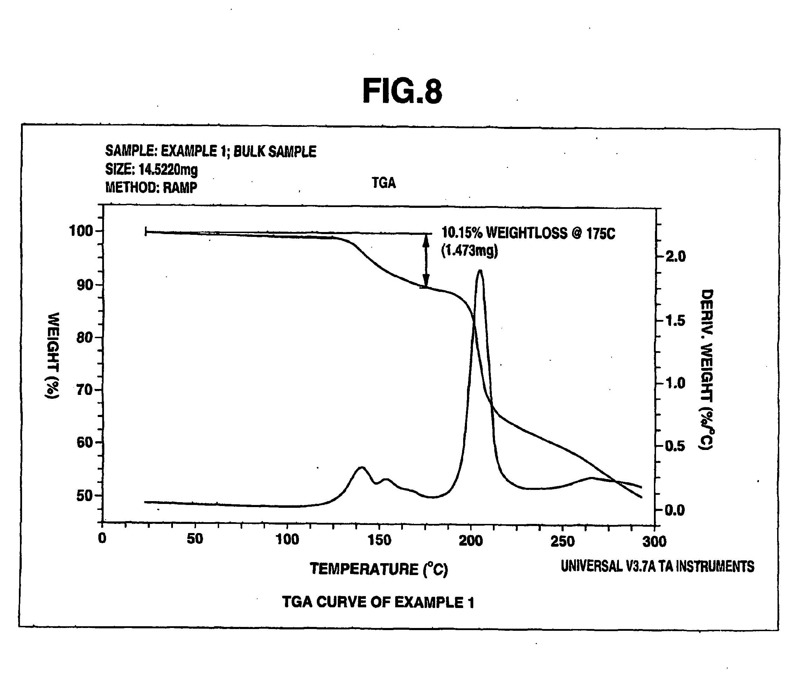

Figure 8 is a TGA curve for Example 1 (weight (%) for the y-axis; temperature (°C) for the x-axis).

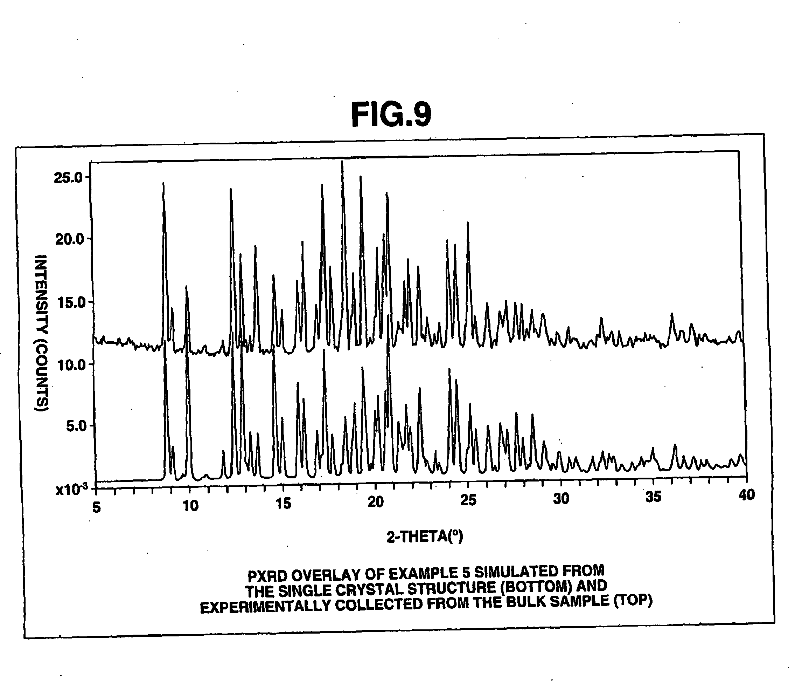

Figure 9 depicts the PXRD overlay of the mono-lysine isopropyl alcohol solvate of ((2*R*,3*R*)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate (Example 5), simulated from the single crystal structure versus what was experimentally collected from the bulk sample.

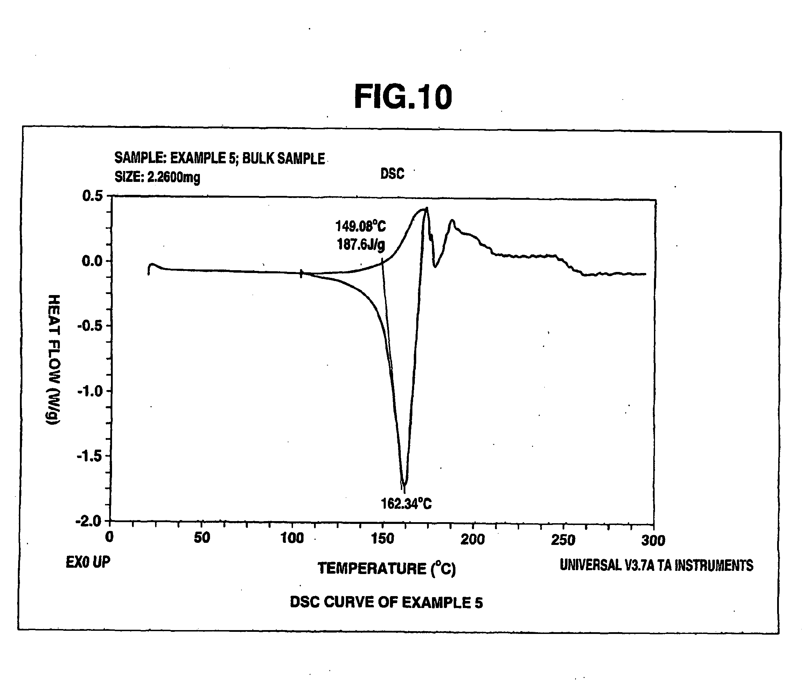

Figure 10 is a DSC curve for Example 5, wherein heat flow (W/g) is the y-axis and temperature (°C) is the x-axis.

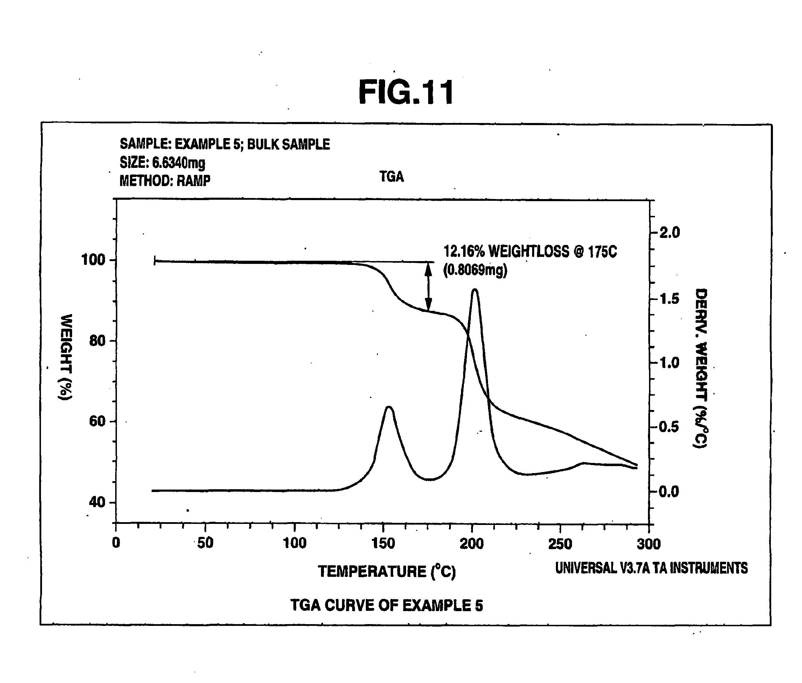

Figure 11 is a TGA curve for Example 5 (weight (%) for the y-axis; temperature (°C) for the x-axis).

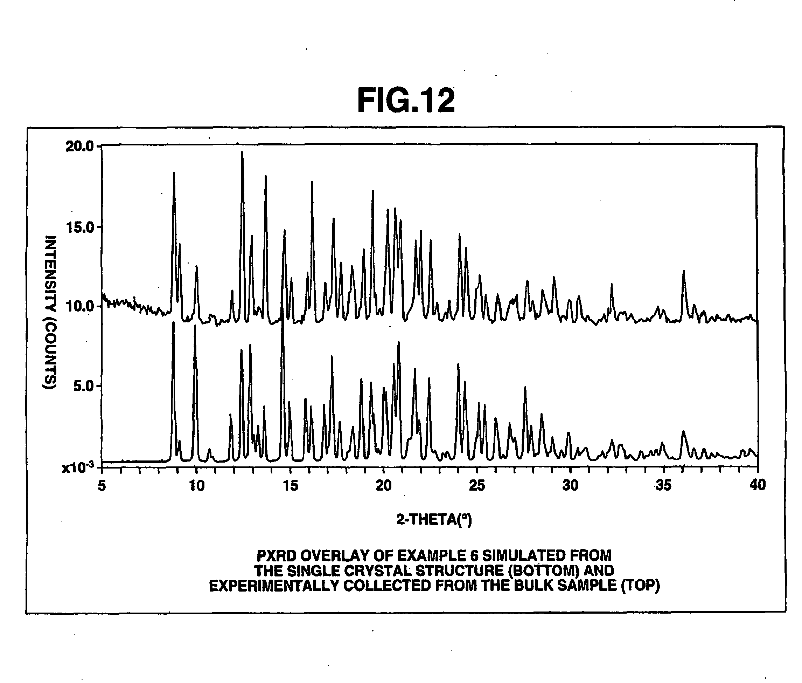

Figure 12 depicts the PXRD overlay of the mono-lysine n-propyl solvate of ((2*R*,3*R*)-3-(4-(4-cyanophenyl)thiazol-2-yl)-2-(2,4-difluorophenyl)-1-(1*H*-1,2,4-triazol-1-yl)butan-2-yloxy)methyl dihydrogen phosphate (Example 6), simulated from the single crystal structure versus what was experimentally collected from the bulk sample.

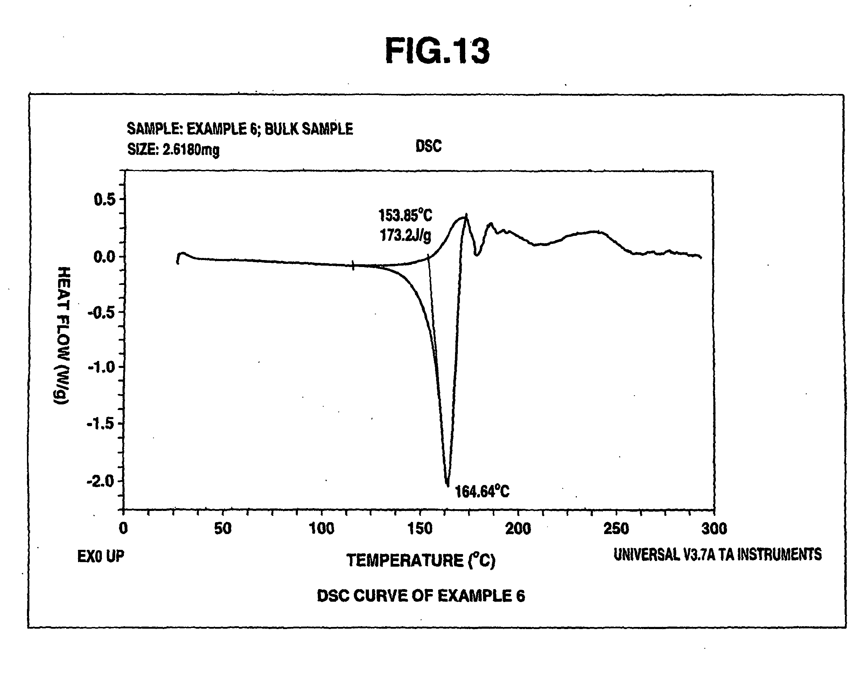

Figure 13 is a DSC curve for Example 6, wherein heat flow (W/g) is the y-axis and temperature (°C) is the x-axis.

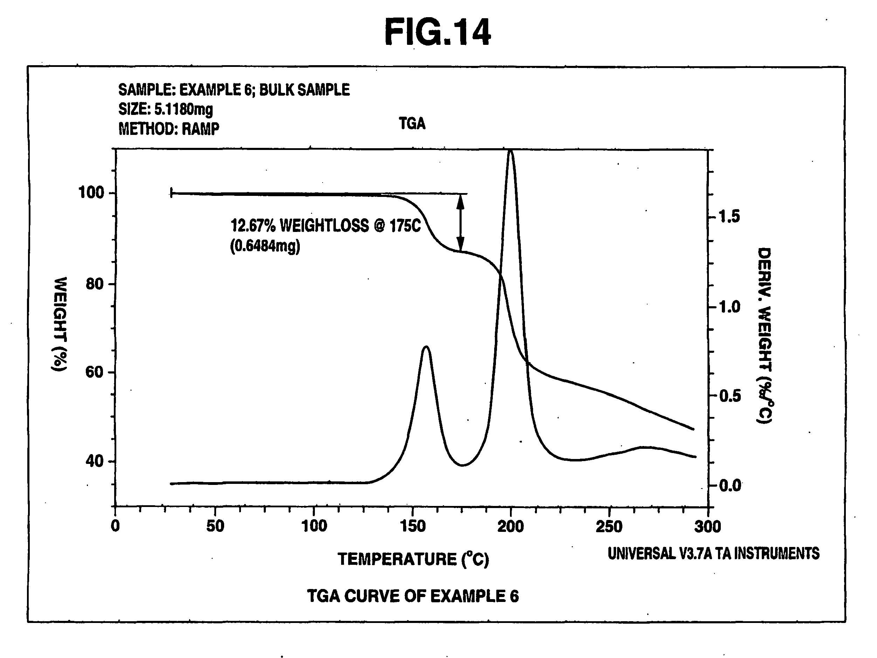

Figure 14 is a TGA curve for Example 6 (weight (%) for the y-axis; temperature (°C) for the x-axis).

The mono-lysine salts and solvates thereof of the present invention may be made by the following general reaction scheme. In this method, A represents the non-hydroxy portion of a triazole antifungal compound of the type containing a tertiary or secondary hydroxyl group, Pr represents a conventional hydroxy-protecting groups such as t-butyl, benzyl or allyl, and R and R¹ are each independently hydrogen or a (C₁-C₆) alkyl group. Most preferably, R and R¹ are both hydrogen.

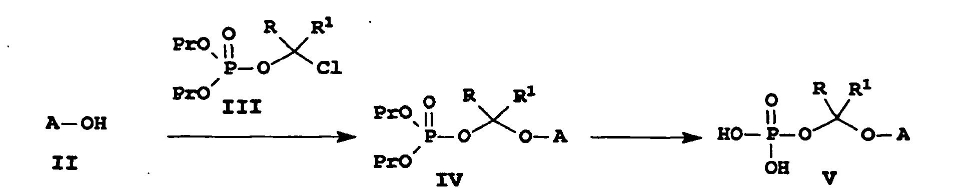

To elaborate on the method, the antifungal parent compound of interest, II, is converted into the ester phosphate intermediate IV (the first intermediate) by O-alkylation with chloride intermediate III in the presence of a suitable base. The suitable base can be sodium hydride, potassium hydride, sodium amide, sodium t-butoxide, potassium t-butoxide, sodium bis(trimethylsilyl)amide, potassium bis(trimethylsilyl)amide, or combinations thereof, such as sodium hydride plus sodium bis(trimethylsilyl)amide. This reaction step may be carried out in an inert organic solvent such as tetrahydrofuran, methyl-tetrahydrofuran, methyl t-butyl ether, diethylether or dimethylacetamide at a temperature of from about 0° to 50°C, more preferably from about 20° to 40°C, and most preferably at about 40°C. The most preferred base is sodium hydride and the most preferred solvent is tetrahydrofuran. The most preferred R is hydrogen, and the most preferred R¹ is also hydrogen.

Ester phosphate intermediate IV is then subjected to a conventional deprotection step to remove the hydroxyl-protecting groups Pr, and then forming the (second) intermediate of formula V (*see* T.W. Greene et al., Protecting Groups in Organic Synthesis, John Wiley & Son (1991); herein incorporated by reference). The reagents used in such step will depend on the particular hydroxyl-protecting group used, but will be well known to those skilled in the art. The most preferred hydroxy protecting group is the t-butyl group that can be removed with trifluoroacetic acid, hydrochloric acid or formic acid in an appropriate inert organic solvent. The inert organic solvent may be, for example, methylene chloride, dichloroethane, methylbenzene or trifluoromethyl benzene. In the case of the preferred deprotection step with the di-tertiary butyl ester, it is preferred to do the deprotection step in trifluoroacetic acid in methylene chloride at a temperature of from about 0° to 40°C, most preferably at a temperature of about 0-5°C.

The intermediate product V may then be recovered and purified by conventional procedures such as reverse phase C-18 column chromatography or solvent extraction. Intermediate product V may be, of course, converted by conventional means to a desired pharmaceutically acceptable salt as described above. Intermediate product V is then mixed with a lysine source to obtain the mono-lysine salt of the present invention.

Specifically, intermediate product V is dissolved in a solvent (e.g., lower alcohol) to form the free acid solution. Then, the free acid solution (containing intermediate product V) is heated and treated with an aqueous solution of lysine (i.e., L-lysine), wherein the pH is adjusted to be between about 3.5 and about 6.0, preferably between about 4.2, and about 5.5, to obtain the final product I. The narrow pH range aids in obtaining the pure mono-lysine salt of formula I without contamination of producing the bis-lysine salt.

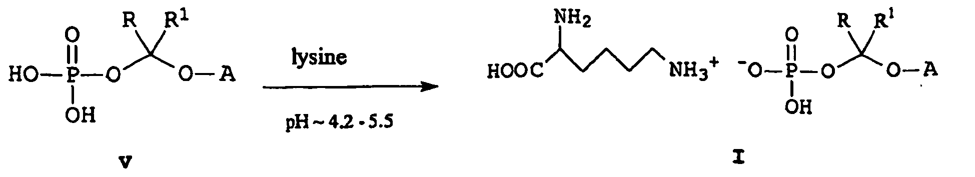

The use of purified reagent III results in fairly low yields of intermediate IV (approximately 10-35% yield) in the above reaction, resulting in low overall yields of product I. However, when a source of iodide ion is added to the O-alkylation step of the above reaction, the yield of intermediate IV is unexpectedly increased to up to about 90%, thus also significantly increasing the yield of intermediate product V. It is believed that the addition of the iodide ion may result in *in situ* formation of the corresponding iodide intermediate IIIa of the formula: 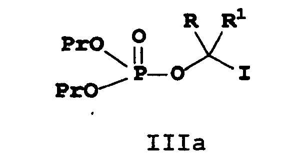 and that use of this reagent results in a large increase in yield of the intermediate IV. An attempt to substitute preformed intermediate IIIa directly for intermediate III in the first step of the above reaction, however, was unsuccessful due to the greatly decreased stability of iodide reagent IIIa compared to the chloride intermediate III. An alternative method that is successful involves using iodine in the O-alkylation step along with chloride intermediate III in the presence of base such as NaH (which also may act as a reducing agent for the iodine). It is believed that the iodine is reduced to iodide ion which then converts chloride intermediate III *in situ* to iodide intermediate IIIa to facilitate this step of the process. The examples below show the o-alkylation step using

elemental iodine that is the preferred method of carrying out this reaction to get intermediate IV.

By forming the iodide reagent IIIa *in situ* by addition of a source of iodide ion or by reaction of iodine and reagent III in the presence of strong base, the greatly increased yield of intermediate IV allows the intermediate product V to be obtained in greatly increased yield. This leads, of course, to greatly increased yield of the mono-lysine salts and solvates thereof of formula I.

The source of iodide ion is preferably sodium iodide, but may also include lithium iodide, cesium iodide, cadmium iodide, cobalt iodide, copper iodide, rubidium iodide, barium iodide, zinc. iodide and calcium iodide. About 2-3 equivalents of the iodide salt are generally used per equivalent of parent compound A-OH.

When elemental iodine is used in the coupling step, about 0.1 to 1.0 equivalent of iodine, preferably 0.5 equivalent, is employed per equivalent of parent compound A-OH.

The bases and solvents that are used when iodine or iodide ion is used are the same as those described above when reagent III is used *per* se.

It will be understood that where the substituent groups used in the above reactions contain certain reaction sensitive functional groups such as amino or carboxylate groups which might result in undesirable side-reactions, such groups may be protected by conventional protecting groups known to those skilled in the art. Suitable protecting groups and methods for their removal are illustrated, for example, in Protective Groups in Organic Synthesis, Theodora W. Greene (John Wiley & Sons, 1991).

It will be appreciated that.certain products within the scope of formula I may have substituent groups which can result in formation of optical isomers. It is intended that the present invention include within its scope all such optical isomers as well as epimeric mixtures thereof, i.e., R- or S- or racemic forms.

The pharmaceutically active salts or solvates thereof of the present invention may be used alone or formulated as medical or pharmaceutical compositions comprising, in addition to the active triazole ingredient, a pharmaceutically acceptable carrier, adjuvant or diluent.

The pharmaceutical compositions may be in solid form such as capsules, tablets, powders, etc., or in liquid form such as solutions, suspensions or emulsions. Such capsules, tablets, etc., may contain a controlled-release formulation. Such solid forms, such as gelatin capsules or compressed tablets, can be prepared in any conventional techniques. For example, the active compounds can be incorporated into a formulation that includes pharmaceutically acceptable carriers such as excipients (e.g., starch, lactose), binders (e.g., gelatin, cellulose, gum), disintegrating agents (e.g., alginate, Primogel, and corn starch), lubricants (e.g., magnesium stearate, silicon dioxide), and sweetening or flavoring agents (e.g., glucose, sucrose, saccharin, methyl salicylate, and peppermint). Various coatings can also be prepared for the capsules and tablets to modify the flavors, tastes, colors, and shapes of the capsules and tablets. In addition, liquid carriers such as fatty oil, sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA. The compositions may be in ready-to-use form or in powder form for reconstitution at the time of delivery with a suitable vehicle such as sterile water.

For example, a tablet may be prepared by compression or molding, optionally with one or more accessory ingredients. Compressed tablets may be prepared by compressing in a suitable machine, the active ingredient in a free-flowing form, including, but not limited to, powder or granules, optionally mixed with a pharmaceutically acceptable carrier, which may comprise one or more of a lubricant, inert diluent, surface active or dispersing agent, or the like. Molded tablets may be made by molding in a suitable machine, a mixture of the powdered compound, moistened with an inert liquid diluent. The amount of active ingredient found in the composition may vary depending on the amount of active ingredient to be administered to the patient.

Also, because the mono-lysine salts and/or solvates thereof of general formula I have improved handling during processing due to low hygroscopicity, and good solubility, the present invention can be administered as a lyophilized formulation. Also, such properties allow the present invention to be administered by a variety of means. Administration herein means several modes thereof. For example, administration can be oral, topical or parenteral (including intravenously, intravascularly, intraperitoneally, subcutaneously, intramuscularly, intrasternally and infusion techniques), wherein the administration employs an effective or therapeutic antifungal amount of the salt compound. The mammalian subject (e.g., human, dog, cat, horse, pig, etc.) can receive such oral, topical or parenteral administration when in need thereof.

The pharmaceutical solutions suitable for injectable use include sterile aqueous solutions or dispersions and sterile powders for the extemporaneous preparation of sterile injectable solutions or dispersions. Additives include a dissolution aid (e.g., sodium salicylate, sodium acetate), buffer (e.g., sodium citrate, glycerine), isotonizing agent (e.g., glucose) and stabilizer (e.g., polyethylene glycol). Solutions or suspensions of the active salt or solvate as a free base can be prepared in glycerol, liquid, polyethylene glycols, mixtures thereof in oils, or some other nontoxic parenterally acceptable diluent or solvent, for example, as a solution in 1, 3-butanediol. Among the acceptable vehicles and solvents that may be employed are water, Ringer's solution, and isotonic sodium chloride solution. In addition, sterile, fixed oils are conventionally employed as a solvent or suspending medium. Under ordinary conditions of storage and use, these preparations contain a preservative. Further, compositions for injection may be prepared in unit dose form in ampules or in multidose containers and may contain additives such as suspending, stabilizing and dispersing agents. In all cases, the form must be sterile and must be fluid to the extent that easy syringability exists. It must be stable under the conditions of manufacture and storage and must be preserved against the contaminating action of microorganisms like bacteria. The carrier can be a solvent or dispersion medium containing, for example, water, ethanol, polyol (e.g., glycerol, propylene glycol and liquid polyethylene glycol), suitable mixtures thereof, and vegetable oil.

Further, the administration dosages can vary. The dosage to be administered depends, to a large extent, on the particular compound being used, the particular composition formulated, the route of administration, the nature and condition of the host and the particular situs and organism being treated. Selection of the particular preferred dosage and route of application, then, is left to the discretion of the physician or veterinarian and can be determined by routine methods.

In addition, the administration times can vary. In general, however, the salt or solvate compounds may be administered parenterally or orally to mammalian hosts in an amount of from about 5 mg/day to about 1.0 g/day. These doses are exemplary of the average case, and there can be individual instances where higher or lower dosages are merited, and such dosages are within the scope of this invention. Furthermore, administration of the compounds of the present inventions can be conducted in either single or divided doses.

When administered orally or parenterally, one of skill in the art can determine suitable amounts of the salt compound and times of administration.

When administered orally, suitable amounts of the salt compound are in the range of 85 mg to 1020 mg, and anywhere from once a day to three times a day.

When administered parenterally, the suitable amounts of the salt compound are in the range of 85 mg to 1020 mg, and anywhere from once a day to three times a day.

Alternatively, the compounds of the present invention can be administered in the form of a suppository or pessary, or they may be applied topically in the form of a lotion, solution, or cream. Additionally, they may be incorporated (at a concentration up to 10%) into an ointment consisting of a white wax or soft, white paraffin base together with the required stabilizers and/or preservatives.

For topical administration, the composition can be applied to the affected areas two to four times a day, or some other variation thereof.

Formulations suitable for topical administration include liquid or semi-liquid preparations suitable for penetration through the skin (e.g., liniments, lotions, ointments, creams, or pastes). Such topical formulations can include one or more thickening agents, humectants, an/or emollients including but not limited to xanthan gum, petrolatum, beeswax, or polyethylene glycol, sorbitol, mineral oil, lanolin, squalene, and the like. For instance, in lotions or creams, the inventive salt or solvate is suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water. For ointment formulations containing the active salt or solvate, the active ingredient is suspended or dissolved in, for example, a mixture with one or more of the following: mineral oil, liquid petrolatum, white petrolatum, propylene glycol, polyoxyethylene polyoxypropylene compound, emulsifying wax and water. A suitable topical amount of active ingredient of the salt or solvate of the present invention is 0.1 mg to 150 mg administered one to four, preferably one or two times daily. For topical administration, the active ingredient may comprise from 0.001% to 10% w/w, e.g., from 1% to 2% by weight of the formulation, although it may comprise as much as 10% w/w., but preferably not more than 5% w/w, and more preferably from 0.1% to 1% of the formulation.

The salts or solvates thereof of the present invention can also be administered intranasally or by inhalation and are conveniently delivered in the form of a dry powder inhaler or an aerosol spray presentation from a pressurized container, pump, spray or nebuliser with the use of a suitable propellant, e.g., dichlorodifluoromethane, trichlorofluoromethane, dichlorotetrafluoroethane, a hydrofluoroalkane such as 1,1,1,2-tetrafluoroethane (HFA 134A [trade mark] or 1,1,1,2,3,3,3-heptafluoropropane (HFA 227EA [trade mark]), carbon dioxide or other suitable gas. In the case of a pressurized aerosol, the dosage unit may be determined by providing a valve to deliver a metered amount. The pressurized container, pump, spray or nebuliser may contain a solution or suspension of the active salt or solvate, e.g., using a mixture of ethanol and the propellant as the solvent, which may additionally contain a lubricant, e.g., sorbitan trioleate. Capsules and cartridges (made, for example, from gelatin) for use in an inhaler or insufflator may be formulated to contain a powder mix of a compound of the present invention and a suitable powder base such as lactose or starch. A spray composition, e.g., would comprise a solution of the novel salt (or solvate thereof) of formula I with a pharmaceutically acceptable liquid carrier as mentioned above. The spray can be used for topical administrations as well. The inhalant composition would also have the novel mono-lysine salt or solvate thereof of formula I, as well as an acceptable propellant as mentioned above.

The mono-lysine salts of the present invention, or solvates thereof, are useful because they possess pharmacological activities in animals, including particularly mammals and most particularly, humans. Specifically, the salt or solvates of the present invention are useful for the treatment or prevention of topical fungal infections, including those caused by species of *Candida, Trichophyton, Microsporum,* or *Epidermophyton.* Additionally, they are useful for the prevention or treatment of mucosal infections caused by Candida albicans. They can also be used in the prevention or treatment of systemic fungal infections caused, for example, by species of *Candida* albicans, *Cryptococcus neoformans, Aspergillus flavus, Aspergillus fumigatus, Coccidioides, Paracoccidiodes, Histoplasma,* or *Blastomyces.*

The use of the salts or solvates thereof of the present invention as pharmaceuticals and the use of the compounds of the invention in the manufacture of a medicament for the treatment of fungal infections are also provided.

The in vitro evaluation of the antifungal activities of the compounds of the present invention can be performed by determining the minimum inhibitory concentration (MIC). The MIC is the concentration of test compound that inhibits the growth of the test microorganism. In practice, a series of agar plates, each having the test compound incorporated at a specific concentration, is inoculated with a fungal strain and each plate is then incubated for 48 hours at 37°C. The plates are examined for the presence or absence of fungal growth, and the relevant concentration is noted. Microorganisms which can be used in the test include Candida *albicans, Asperigillus fumigatus, Trichophyton* spp., *Microsporum spp., Epidermophyton floccosum, Coccidioides* immitis, and *Torulopsos galbrata.* It should be recognized that, as prodrugs, some salt or solvates thereof of the present invention may not be active in the *in vitro* test.

The *in vivo* evaluation of salts or solvates thereof of the present invention can be carried out at a series of dose levels by intraperitoneal or intravenous injection or by oral administration to mice that have been inoculated with a strain of fungus (e.g., *Candida albicans).* Activity is determined by comparing the survival of the treated group of mice at different dosage levels after the death of an untreated group of mice. The dose level at which the test salt or solvate compound provides 50% protection against the lethal effect of the infection is noted.

The mono-lysine salts or solvate thereof of the present invention unexpectedly increase the handling due to low hygroscopicity while maintaining good solubility of the parent triazole antifungal compound and also release the bioactive compound (e.g., function as a prodrug). For example, as shown in Figure 2, there is a <2.5% weight change for adsorption and <5% weight change for desorption at 60%RH for the ethanol solvate form. As another example, there is a <0.5% weight change for adsorption and <1% weight change at 60%RH for the isopropyl alcohol solvate.

### EXAMPLES

The following examples illustrate the present invention, but are not intended as a limitation thereof. The abbreviations used in the examples are conventional abbreviations well-known to those skilled in the art. Some of the abbreviations used are as follows:
- h =: hour(s)
- rt =: room temperature
- mmol =: mmole(s)
- g =: gram(s)
- THF =: tetrahydrofuran
- mL =: milliliter(s)
- L =: liter (s)
- Et2O =: diethyl ether
- EtOAc =: ethyl acetate
- TFA =: trifluoroacetic acid
- CH2Cl2 =: dichloromethane
- CH3CN =: acetonitrile

In the following examples, all temperatures are given in degrees Centigrade (°C). Melting points are determined on an electrothermal apparatus and are not corrected. Proton nuclear magnetic resonance (1H NMR) spectra are recorded on a Bruker -500, Bruker AM-300 or a Varian Gemini 300 spectrometer. All spectra are determined in CDCl3 or D2O unless otherwise indicated. Chemical shifts are reported in δ units (ppm) relative to tetramethylsilane (TMS) or a reference solvent peak and interproton coupling constants are reported in Hertz (Hz). Splitting patterns are designated as follows: s, singlet; d, doublet; t, triplet; q, quartet; m, multiplet; br, broad peak; dd, doublet of doublets; dt, doublet of triplets; and app d, apparent doublet, etc. Mass spectra are recorded on a Kratos MS-50 or a Finnegan 4500 instrument utilizing direct chemical ionization (DCI, isobutene), fast atom bombardment (FAB), or electron ion spray (ESI).

Analytical thin-layer chromatography (TLC) is carried out on precoated silica gel plates (60F-254) and visualized using UV light, iodine vapors, and/or staining by heating with methanolic phosphomolybdic acid. Reverse phase chromatography is performed in a glass column using C18 silica gel (Waters Corporation Preparative C18 125A) at pressures somewhat above atmospheric pressure.

### EXAMPLE 1

### ((2R,3R)-3-(4-(4-cyanophenyl)thiazol-2-yl)-2-(2,4-difluorophenyl)-1-(1H-1,2,4-triazol-1-yl)butan-2-yloxy)methyl dihydrogen phosphate mono-lysine salt ethanol solvate

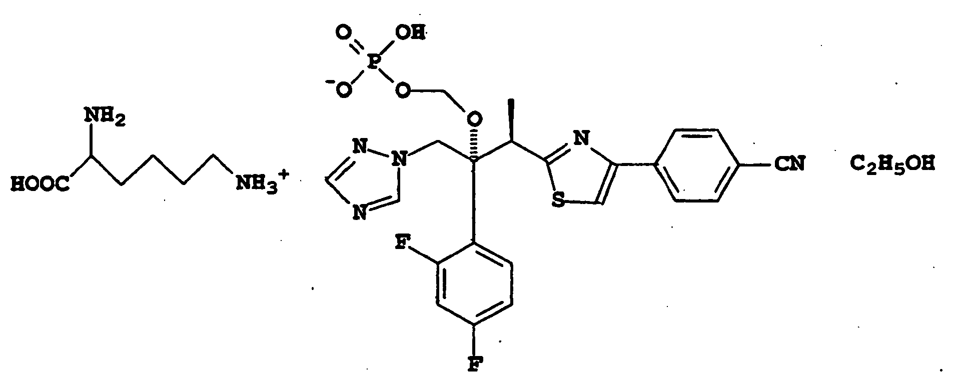

### Step A

An oven dried, 1L round-bottom flask equipped with a mechanical stirrer, nitrogen inlet adapter, pressure-equalizing addition funnel fitted with a rubber septum and temperature probe was charged with sodium hydride (2.89 g, 0.069 mol, 60%) and THF (50 mL). To this stirred suspension, (2R,3R)-3-[4-(4-cyanophenyl)thiazol-2-yl]-2-(2,4-difluorophenyl)-1-(1*H*-1,2,4-triazol-1-yl)butan-2-ol (formula B) (10 g, 0.023 mol) in 30 mL of THF was added dropwise over 20 minutes at room temperature. After stirring for 45 minutes, a solution of iodine (2.99 g, 0.0115 mol) in THF (30 mL)) was added dropwise over 10 minutes followed by dropwise addition of compound di-tert-butyl chloromethyl phosphate (formula III') (13.29 g, 0.035 mol, ∼68% purity) over 15 minutes. The reaction mixture was stirred for 4 hours at about 41°C to complete the reaction. The completion of the reaction was judged by in-process HPLC.

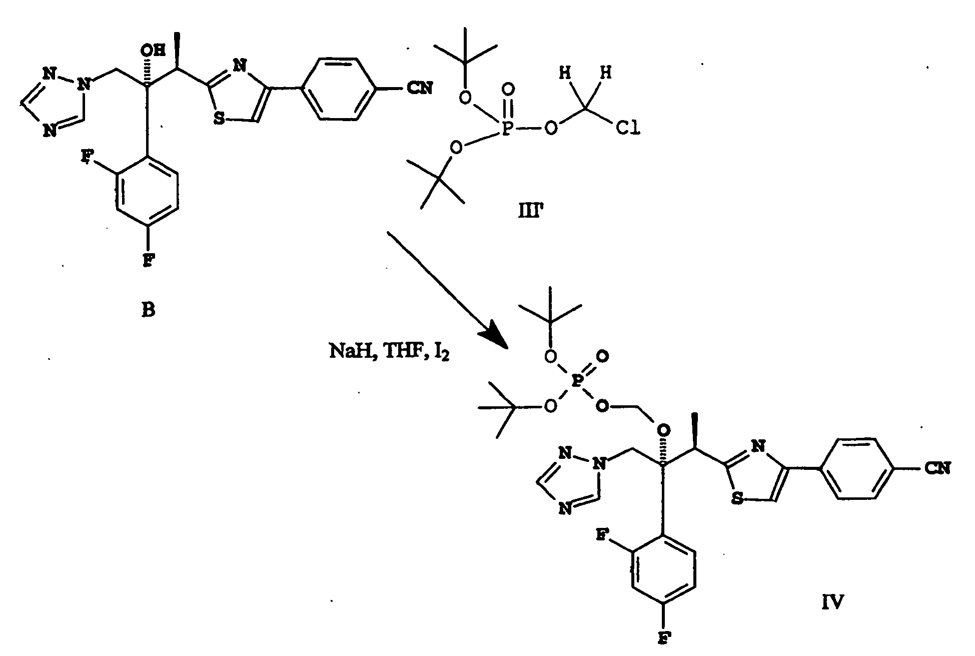

The reaction mixture was poured into ice-cold water (100 mL). The aqueous phase was separated and extracted with ethyl acetate (3 x 50 mL) and the combined organic extract was washed with 10% sodium thiosulfite (50 mL), water (50 mL), brine (50 mL), dried over magnesium sulfate and concentrated under reduced pressure to give pale yellow oil (22.8 g, In-process HPLC: - 97 area percent). The crude product (formula IV) was used "as is" in Step B.

### Step B

To a round-bottom flask equipped with magnetic stirrer, cooling bath, pH probe and N2 inlet-outlet was charged the product of Step A above (formula IV) (7.5 g) in CH2Cl2 (23 mL) and cooled to 0°C. To this stirred solution, trifluoroacetic acid (8.8 mL) was added slowly and stirred for 3 h to complete the reaction. The completion of the reaction was judged by in-process HPLC. The reaction mixture was poured into a cold solution of 2N NaOH (64 mL). The reaction mixture was extracted with t-butyl acetate (2 x 65 mL) to remove all the organic impurities. The aqueous layer containing the bis sodium salt product was treated with activated charcoal (10 g) and filtered through a bed of Celite. The clear filtrate was acidified with 1N HCl to pH 2.5. The free acid product was extracted into ethyl acetate (2 x 50 mL). The combined organic layer was washed with water, dried over MgSO4, filtered, and the filtrate concentrated under reduced pressure to afford 3.39 g of crude product V. Alternatively, in a preferred aspect of the present invention, Step B can be performed as a continuous process, the details of which can be determined by one of ordinary skill in the art.

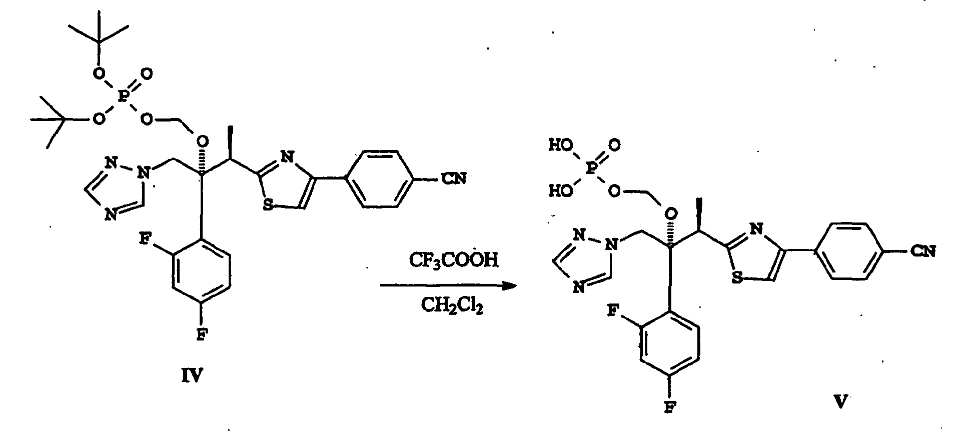

### Step C

The above obtained product V was dissolved in methanol (75 mL). With the free acid solution, L-lysine (1.8 g) was added with the pH maintained at 4.2 to 5.5, and the mixture was heated at 60°C for 4.5 h. The hot reaction mixture was filtered through a bed of Celite. The filtrate was concentrated to about 5 mL, mixed with ethanol (100 mL) and heated to 65°C to crystallize the solvate of the mono-lysine salt. The solvate was collected on a Buchner funnel and dried under vacuum to afford 3.71 g of the title solvate compound as a crystalline solid.

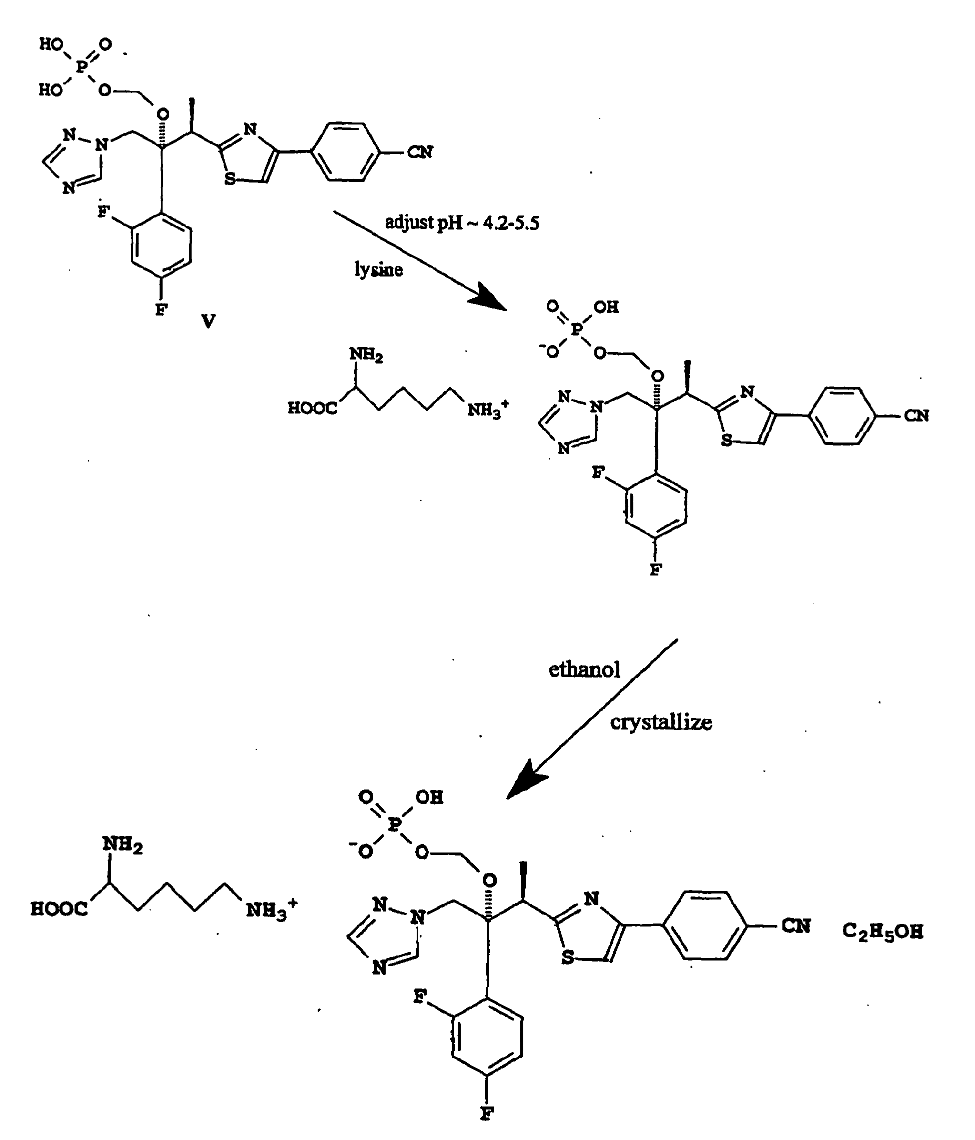

The powder X-ray diffraction data (PXRD) (see Figure 5), X-ray crystallographic data from single crystal (Tables 1-2) and nuclear magnetic resonance spectrum data were collected (Figures 6A, 6B, 6C), and a differential scanning calorimetry curve (Figure 7) and thermal gravimetric analysis (TGA) curve (Figure 8) were obtained for Example 1.

**Table 1: Crystal data and structure refinement for Example 1**

| | |
|---|---|
| Temperature | 293(2) K |
| Wavelength | 1.54178 A |
| Crystal system, space group | Orthorhombic, P2₁2₁2₁ |
| Unit cell dimensions | a = 9.0314(1)A α = 90° |
| | b = 10.2534(1)A β = 90° |
| | c = 38.7048(5)Å γ = 90° |
| Volume | 3584.16(7) Å³ |
| Z, Calculated density | 4, 1.371 Kg/m³ |
| Absorption coefficient | 1.819 mm⁻¹ |
| Crystal size | 0.60 x 0.18 x 0.04 mm |
| θ range for data collection | 2.28 to 65.34° |
| Limiting indices | -9<=h<=10, -11<=k<=12, - |
| | 45<=l<=44 |
| Reflections collected / unique | 19180 / 5953 [R(int) = 0.0673] |
| Completeness to θ = 65.34 | 98.2 % |
| Absorption correction | SADABS |
| Max. and min. transmission | 1.000 and 0.664 |
| Refinement method | Full-matrix least-squares on F² |
| Data / restraints / parameters | 5953 / 0 / 459 |
| Goodness-of-fit on F² | 1.054 |
| Final R indices [I>2σ(I)] | R1 = 0.0409, wR2 = 0.1116 |
| R indices (all data) | R1 = 0.0424, wR2 = 0.1128 |
| Absolute structure parameter | 0.024(18) |
| Largest diff. peak and hole | 0.411 and -0.273 e.Å⁻³ |

**Table 2: Atomic coordinates ( x 10⁴) and equivalent isotropic displacement parameters (Å x 10³). U(eq) is defined as one third of the trace of the orthogonalized Uij tensor**

| | x | y | z | U (eq) |
|---|---|---|---|---|
| S(1) | 6336(1) | 3270(1) | 1117(1) | 56(1) |
| P(1) | 4609(1) | 9112(1) | 606(1) | 32(1) |
| O(1) | 5748(2) | 6356(2) | 1196(1) | 35(1) |
| O(2) | 5450(2) | 9307(2) | 907(1) | 45(1) |
| O(3) | 5863(2) | 10108(2) | 510(1) | 44(1) |
| O(4) | 3344(2) | 9821(2) | 759(1) | 52(1) |
| O(5) | 4260(2) | 8197(2) | 316(1) | 42(1) |
| N(1) | 10040(2) | 7579(3) | 865(1) | 51(1) |
| N(2) | 9863(3) | 6877(3) | 1409(1) | 59(1) |
| N(3) | 8616(2) | 7513(2) | 1315(1) | 38(1) |
| N(4) | /5365(2) | 3376(2) | 1734(1) | 39(1) |
| N(5) | -962(4) | -1128(3) | 2299(1). | 82(1) |
| F(1) | 3340(2) | 5337(2) | 1502(1) | 52(1) |
| F(2) | 1877(2) | 6905(3) | 2576(1) | 84(1) |
| C(1) | 8746(3) | 7918(3) | 993(1) | 48(1) |
| C(2) | 10662(3) | 6949(4) | 1127(1) | 56(1) |
| C(3) | 7379(3) | 7640(3) | 1558(1) | 39(1) |
| C(4) | 6300(3) | 6470(2) | 1544(1) | 34(1) |
| C(5) | 5075(3) | 6636(3) | 1812(1) | 37(1) |
| C(6) | 3697(3) | 6025(3) | 1788(1) | 40(1) |
| C(7) | 2616(3) | 6094(3) | 2038(1) | 52(1) |
| C(8) | 2931(4) | 6811(4) | 2328(1) | 56(1) |
| C(9) | 4238(4) | 7442(4) | 2375(1) | 57(1) |
| C(10) | 5293(3) | 7354(3) | 2116(1) | 47(1) |
| C(11) | 4713(3) | 7273(3) | 1078(1) | 37(1) |
| C(12) | 7153(3) | 5159(3) | 1604(1) | 38(1) |
| C(13) | 7825(4) | 5043(3) | 1964(1) | 53(1) |
| C(14) | 6247(3) | 3974(3) | 1521(1) | 36(1) |
| C(15) | 5129(4) | 2093(3) | 1246(1) | 54(1) |
| C(16) | 4724(3) | 2303(3) | 1578(1) | 41(1) |
| C(17) | 3590(3) | 1526(3) | 1761(1) | 42(1) |
| C(18) | 2757(3) | 2064(3) | 2025(1) | 45(1) |
| C(19) | 1603(3) | 1388(3) | 2171(1) | 49(1) |
| C(20) | 1272(4) | 136(3) | 2056(1) | 50(1) |
| C(21) | 2123(4) | -425(3) | 1800(1) | 60(1) |
| C(22) | 3275(4) | 263(3) | 1654(1) | 55(1) |
| C(23) | 34(4) | -559(3) | 2194(1) | 61(1) |
| O(6) | 8251(2) | 9133(2) | 231(1) | 50(1) |
| O(7) | 8850(2) | 11116(2) | 42(1) | 52(1) |
| N(6) | 11004(2) | 8174(2) | 159(1) | 35(1) |
| N(7) | 16090(3) | 11826(2) | -191(1) | 51(1) |
| C(24) | 9139(3) | 9958(3) | 114(1) | 37(1) |
| C(25) | 10739(3) | 9548(2) | 51(1) | 34(1) |
| C(26) | 11140(3) | 9726(3) | -329(1) | 39(1) |
| C(27) | 12792(3) | 9627(3) | -411(1) | 38(1) |
| C(28) | 13646(3) | 10790(3) | -274(1) | 41(1) |
| C(29) | 15299(3) | 1.0643(3) | -318(1) | 43(1) |
| O(8) | 1443(4) | 11736(3) | 770(1) | 107(1) |
| C(30) | 315(8) | 11509(8) | 1032(2) | 143(3) |
| C(31) | 287(10) | 12513(11) | 1249(2) | 207(5) |
| H(30) | 6577 | 9701 | 417 | 105(17) |
| H(1A) | 8023 | 8379 | 874 | 58 |
| H(2A) | 11602 | 6583 | 1111 | 67 |
| H(3A) | 6838 | 8434 | 1506 | 47 |
| H(3B) | 7770 | 7718 | 1790 | 47 |
| H(7A) | 1711 | 5674 | 2011 | 63 |
| H(9A) | 4420 | 7920 | 2575 | 69 |
| H(10A) | 6187 | 7791 | 2145 | 57 |
| H(11A) | 4152 | 7613 | 1271 | 45 |
| H(11B) | 4027 | 6856 | 920 | 45 |
| H(12A) | 7986 | 5159 | 1441 | 45 |
| H(13A) | 8340 | 4227 | 1984 | 80 |
| H(13B) | 7051 | 5080 | 2134 | 80 |
| H(13C) | 8506 | 5748 | 2001 | 80 |
| H(15A) | 4796 | 1408 | 1108 | 65 |
| H(18A) | 2982 | 2896 | 2105 | 54 |
| H(19A) | 1044 | 1767 | 2346 | 58 |
| H(21A) | 1918 | -1268 | 1725 | 72 |
| H(22A) | 3848 | -123 | 1483 | 66 |
| H(6NA) | 11944 | 7965 | 119 | 67(11) |
| H(6NB) | 10414 | 7645 | 39 | 50(9) |
| H(6NC) | 10812 | 8091 | 384 | 39(8) |
| H(7NA) | 16088 | 12436 | -358 | 105(17) |
| H(7NB) | 17029 | 11620 | -136 | 79(12) |
| H(7NC) | 15625 | 12139 | -3 | 102(16) |
| H(25A) | 11384 | 10114 | 189 | 41 |
| H(26A) | 10788 | 10575 | -404 | 47 |
| H(26B) | 10618 | 9073 | -463 | 47 |
| H(27A) | 13186 | 8834 | -310 | 45 |
| H(27B) | 12924 | 9573 | -660 | 45 |
| H(28A) | 13322 | 11570 | -393 | 49 |
| H(28B) | 13423 | 10900 | -30 | 49 |
| H(29A) | 15637 | 9886 | -190 | 52 |
| H(29B) | 15527 | 10507 | -560 | 52 |
| H(80) | 2012 | 11076 | 777 | 130(20) |
| H(30A) | -645 | 11410 | 923 | 172 |
| H(30B) | 535 | 10713 | 1157 | 172 |
| H(31A) | -447 | 12360 | 1424 | 310 |
| H(31B) | 46 | 13294 | 1124 | 310 |
| H(31C) | 1241 | 12608 | 1355 | 310 |

### EXAMPLE 2

### Mono-lysine salt of 1-((2S,3S)-3-(4-(4-(4-(4-(((3S)-5-((1H-1,2,4-triazol-1-yl)methyl)-5-(2,4-difluorophenyl)-tetrahydrofuran-3-yl)methoxy)phenyl)piperazin-1-yl)phenyl)-5-oxo-4,5-dihydro-1,2,4-triazol-1-yl)pentan-2-yloxy)methyl dihydrogen phosphate

Step A of Example 1 is repeated, except the compound below is used in place of the compound of formula B:

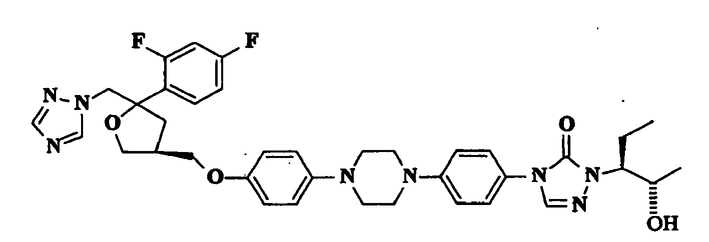

The crude product of the compound of formula IV' is obtained and used "as is" in Step B:

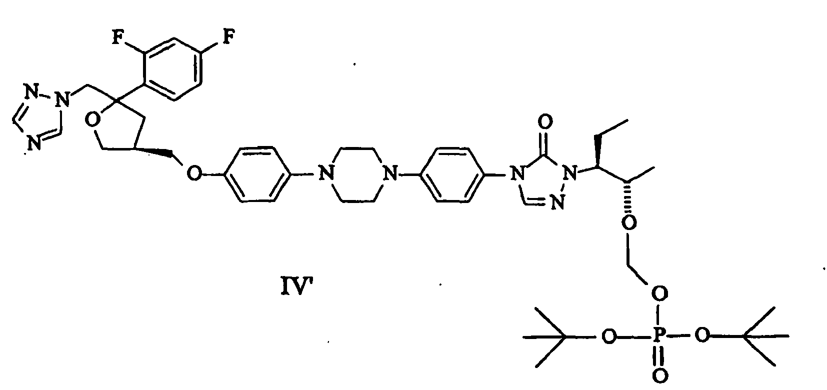

Step B of Example 1 is repeated, except the compound of formula IV' is used in place of the compound of formula IV. Crude product V' is made:

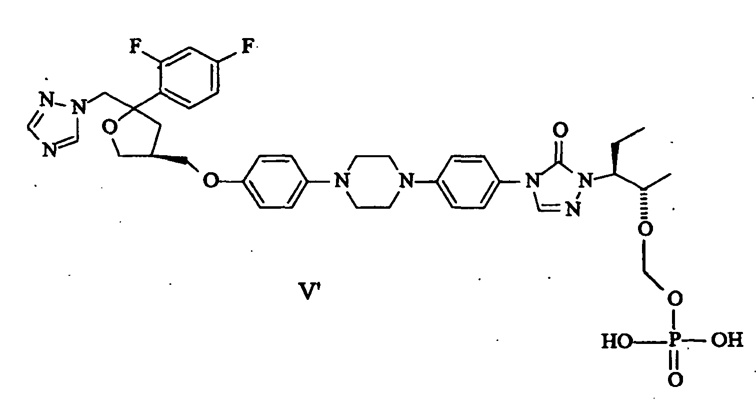

Step C of Example 1 is repeated, except the compound of formula V' is used in place of the compound of formula V to make the mono-lysine salt compound:

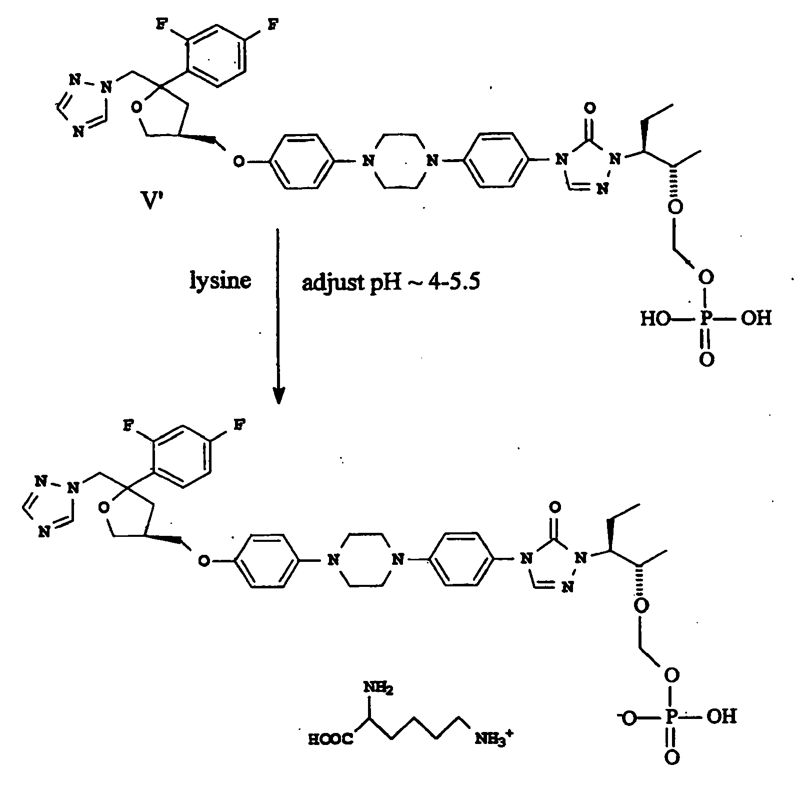

### EXAMPLE 3

### Mono-lysine salt of ((2R,3R)-3-(3-((E)-4-(2,2,3,3,-tetrafluoropropoxy)styryl)-1H-1,2,4-triazol-l-yl)-2-(2,4-difluorophenyl)-1-(1H-1,2,4-triazol-1-yl)butan-2-yloxy)methyl dihydrogen-phosphate

Step A of Example 1 is repeated, except the compound below is used in place of the compound of formula B:

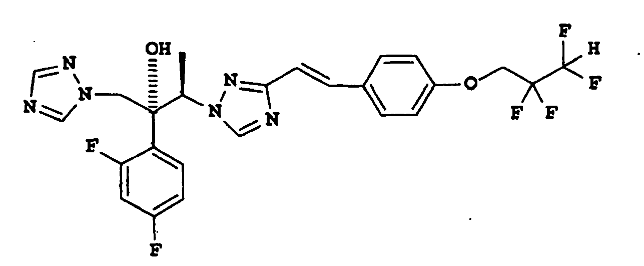

The crude product of the compound of formula IV" is obtained and used "as is" in Step B:

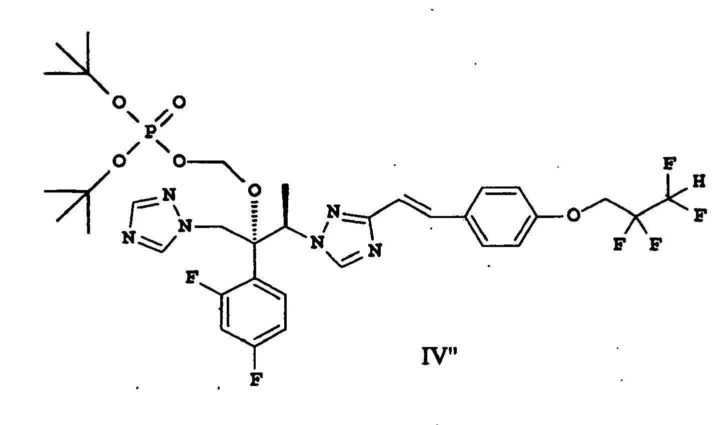

Step B of Example 1 is repeated, except the compound of formula IV" is used in place of the compound of formula IV. Crude product V" is made:

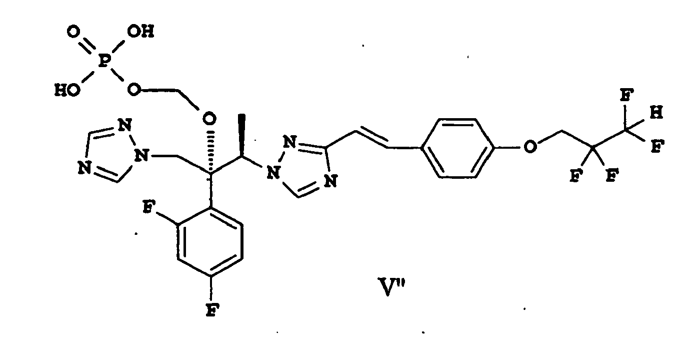

Step C of Example 1 is repeated, except the compound of formula V" is used in place of the compound of formula V to make the mono-lysine salt compound:

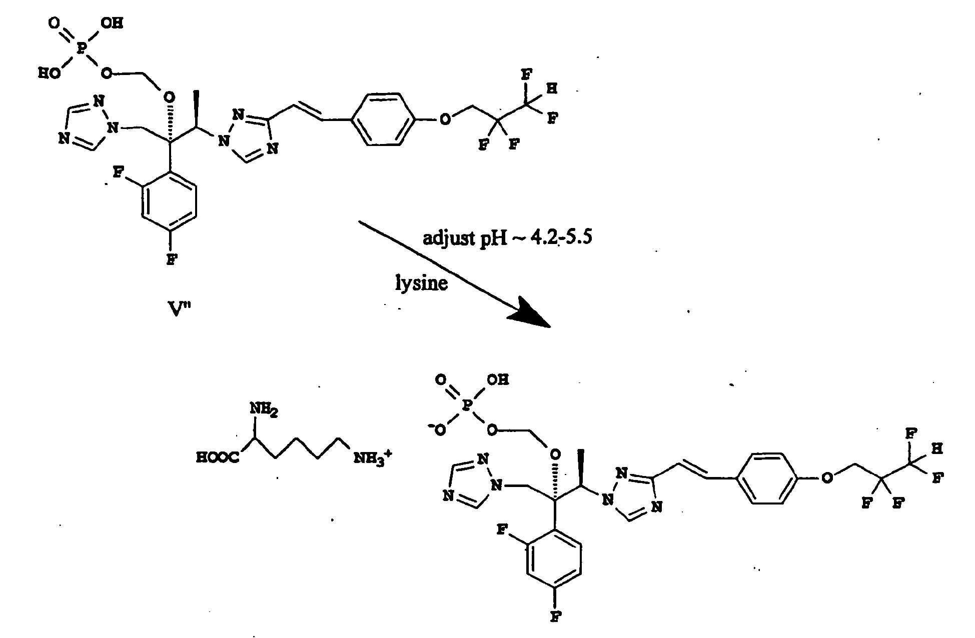

### EXAMPLE 4

### Mono-lysine salt of 1-((2S,3S)-3-(4-(4-(4-(4-(((3S)-5-((1H-1,2,4-triazol-1-yl)methyl)-5-(2,4-difluorophenyl)-tetrahydrofuran-3-yl)methoxy)phenyl)piperazin-1-yl)phenyl)-5-oxo-4,5-dihydro-1,2,4-triazol-1-yl)pentan-2-yloxy)propyl dihydrogen phosphate

Step A of Example 1 is repeated, except the compound below is used in place of the compound of formula B:

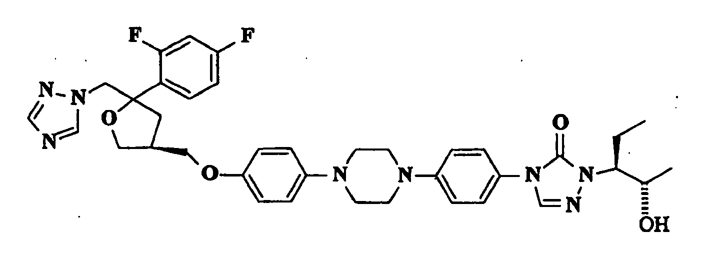

The crude product of the compound of formula IV"' is obtained, wherein R¹ of the chloride intermediate III is ethyl and R is hydrogen, and used "as is" in Step B:

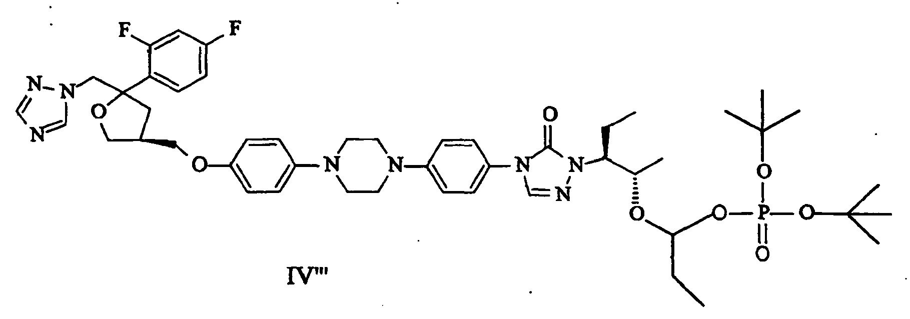

Step B of Example 1 is repeated, except the compound of formula IV"' is used in place of the compound of formula IV. Crude product V"' is made:

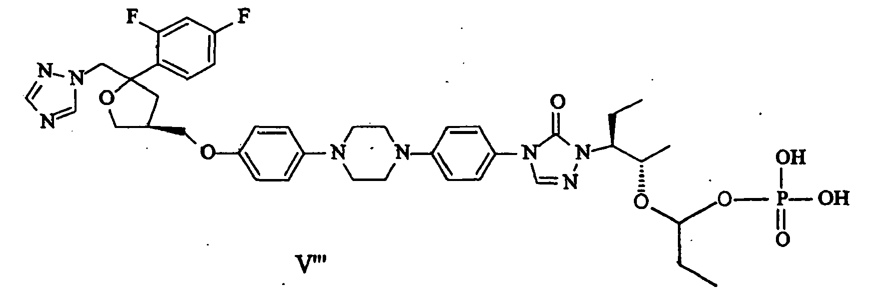

Step C of Example 1 is repeated, except the compound of formula V"' is used in place of the compound of formula V to make the mono-lysine salt compound:

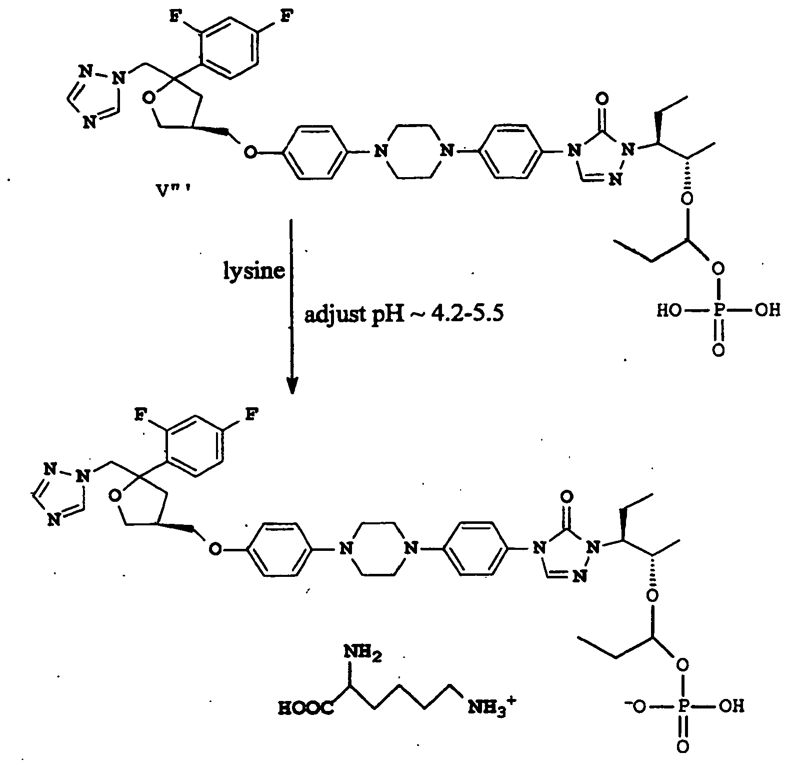

### EXAMPLE 5

### ((2R,3R)-3-(4-(4-cyanophenyl)thiazol-2-yl)-2-(2,4-difluorophenyl)-1-(1H-1,2,4-triazol-1-yl)butan-2-yloxy)methyl dihydrogen phosphate mono-lysine salt isopropyl alcohol solvate

Steps A, B and C of Example 1 were repeated, except the filtrate in Step C was concentrated and mixed with isopropyl alcohol (100 mL) and heated to 65°C to crystallize the solvate of the mono-lysine salt. The solvate was collected on a Buchner funnel and dried under vacuum to obtain the title solvate compound as a crystalline solid.

The PXRD data (see Figure 9), X-ray crystallographic data from single crystal (Tables 3-4) was collected, and a DSC curve (Figure 10) and TGA curve (Figure 11) were obtained for Example 5.

**Table 3: Crystal data and structure refinement for Example 5**

| | |
|---|---|
| Temperature | 293(2) K |
| Wavelength | 1.54178 Å |
| Crystal system, space group | Orthorhombic, P2₁2₁2₁ |
| Unit cell dimensions | a = 9.0716(3)Å α = 90° |
| | b = 10.3611(3) Å β = 90° |
| | c = 38.6521(11)Å γ = 90° |
| Volume | 3632.98(19) Å³ |
| Z, Calculated density | 4, 1.378 Kg/m³ |
| Absorption coefficient | 1.805 mm⁻¹ |
| Crystal size | 0.40 x 0.18 x 0.10 mm |
| θ range for data collection | 2.29 to 65.29° |
| Limiting indices | -10<=h<=10, -12<=k<=11, - |
| | 45<=1<=45 |
| Reflections collected / unique | 19302 / 5801 [R(int) = 0.0757] |
| Completeness to θ = 65.29 | 98.0 % |
| Absorption correction | SADABS |
| Max. and min. transmission | 1.000 and 0.631 |
| Refinement method | Full-matrix least-squares on F² |
| Data / restraints / parameters | 5801 / 0 / 468 |
| Goodness-of-fit on F² | 1.078 |
| Final R indices [I>2σ(I)] | R1 = 0.0493, wR2 = 0.1354 |
| R indices (all data) | R1 = 0.0522, wR2 = 0.1375 |
| Absolute structure parameter | 0.03(2) |
| Largest diff. peak and hole | 1.253 and -0.371 e.Å⁻³ |

**Table 4: Atomic coordinates ( x 10⁴) and equivalent isotropic displacement parameters (Å x 10³). U(eq) is defined as one third of the trace of the orthogonalized Uij tensor**

| | x | y | z | U (eq) |
|---|---|---|---|---|
| S(1) | 6247(1) | 3328(1) | 1114(1) | 48(1) |
| P(1) | 4521(1) | 9094(1) | 613(1) | 29(1) |
| O(1) | 5671 (2) | 6364(2) | 1202 (1) | 31(1) |
| O(2) | 5343(3) | 8315(2) | 921(1) | 40(1) |
| O(3) | 5766(3) | 10084(2) | 519(1) | 40(1) |
| O(4) | 3249(3) | 9801(3) | 760(1) | 47(1) |
| O(5) | 4198(3) | 8179(2) | 325(1) | 40(1) |
| N(1) | 9942(3) | 7464(3) | 862(1) | 41(1) |
| N(2) | 9831(3) | 6952(4) | 1425(1) | 53(1) |
| N(3) | 8541(3) | 7483(3) | 1317(1) | 33(1) |
| N(4) | 5383(3) | 3368(3) | 1740(1) | 34(1) |
| N(5) | -796(5) | -1240(5) | 2303(1) | 86(1) |
| F(1) | 3316(3) | 5308(3) | 1514(1) | 66(1) |
| F(2) | 1871(3) | 6753(3) | 2596(1) | 83(1) |
| C(1) | 8637(4) | 7775(4) | 984(1) | 40(1) |
| C(2) | 10609(4) | 6972(5) | 1139(1) | 53(1) |
| C(3) | 7316(4) | 7631(3) | 1559(1) | 34(1) |
| C(4) | 6229(3) | 6476(3) | 1550(1) | 28(1) |
| C(5) | 5032(3) | 6616(3) | 1821(1) | 31(1) |
| C(6) | 3676(4) | 5989(4) | 1798(1) | 37(1) |
| C(7) | 2606(4) | 6016(4) | 2051(1) | 49(1) |
| C(8) | 2911(5) | 6711(5) | 2344(1) | 53(1) |
| C(9) | 4180(4) | 7359(5) | 2388(1) | 51(1) |
| C(10) | 5241(4) | 7331(4) | 2130(1) | 45(1) |
| C(11) | 4637(4) | 7271(3) | 1085(1) | 34(1) |
| C(12) | 7096(4) | 5183(3) | 1604(1) | 34(1) |
| C(13) | 7783(4) | 5075(4) | 1964(1) | 48(1) |
| C(14) | 6207(4) | 3996(3) | 1520(1) | 31(1) |
| C(15) | 5132(5) | 2120(4) | 1248(1) | 45(1) |
| C(16) | 4761(4) | 2293(3) | 1586(1) | 36(1) |
| C(17) | 3677(4) | 1497(3) | 1775(1) | 36(1) |
| C(18) | 2777(4) | 2025(4) | 2025(1) | 42(1) |
| C(19) | 1640(5) | 1323(4) | 2169(1) | 45(1) |
| C(20) | 1400(5) | 69(4) | 2063(1) | 44 (1) |
| C(21) | 2327(5) | -495(4) | 1822(1) | 49(1) |
| C(22) | 3453(5) | 219(4) | 1679(1) | 46(1) |
| C(23) | 170(5) | -667(4) | 2200(1) | 57(1) |
| O(6) | 8154(3) | 9113(3) | 240(1) | 45(1) |
| O(7) | 8763(3) | 11091(2) | 58(1) | 47(1) |
| N(6) | 10897(3) | 8162(3) | 162(1) | 32(1) |
| N(7) | 16016(3) | 11822(3) | -191(1) | 48(1) |
| C(24) | 9044(4) | 9935(3) | 126(1) | 33(1) |
| C(25) | 10647(4) | 9530(3) | 64(1) | 28(1) |
| C(26) | 11053(4) | 9738(3) | -318(1) | 34(1) |
| C(27) | 12697(4) | 9652(3) | -396(1) | 34(1) |
| C(28) | 13566(4) | 10807(3) | -257(1) | 37(1) |
| C(29) | 15198(4) | 10632(3) | -300(1) | 39(1) |
| O(8) | 1614(4) | 11897(3) | 792(1) | 83(1) |
| C(30) | 829(6) | 11841(5) | 1115(1) | 75(2) |
| C(31) | 497(7) | 13146(6) | 1234(2) | 91(2) |
| C(32) | -444(9) | 10990(7) | 1078(3) | 134(3) |
| H(30) | 6716 | 9809 | 413 | 61(13) |
| H(1A) | 7885 | 8148 | 855 | 47 |
| H(2A) | 11570 | 6660 | 1130 | 64 |
| H(3A) | 6783 | 8416 | 1504 | 40 |
| H(3B) | 7706 | 7720 | 1791 | 40 |
| H(7A) | 1716 | 5582 | 2024 | 58 |
| H(9A) | 4345 | 7823 | 2591 | 62 |
| H(10A) | 6112 | 7792 | 2160 | 54 |
| H(11A) | 4065 | 7586 | 1279 | 40 |
| H(11B) | 3966 | 6860 | 924 | 40 |
| H(12A) | 7918 | 5199 | 1439 | 41 |
| H(13A) | 8297 | 4268 | 1984 | 72 |
| H(13B) | 7021 | 5115 | 2136 | 72 |
| H(13C) | 8463 | 5773 | 1999 | 72 |
| H(15A) | 4819 | 1436 | 1111 | 54 |
| H(18A) | 2938 | 2869 | 2098 | 50 |
| H(19A) | 1037 | 1693 | 2336 | 55 |
| H(21A) | 2190 | -1351 | 1757 | 59 |
| H(22A) | 4071 | -159 | 1515 | 55 |
| H(6NA) | 10559 | 8028 | 378 | 20(8) |
| H(6NB) | 11867 | 7983 | 155 | 35(10) |
| H(6NC) | 10409 | 7646 | 14 | 59(13) |
| H(7NA) | 16843 | 11601 | -77 | 42(11) |
| H(7NB) | 16251 | 12304 | -377 | 73(16) |
| H(7NC) | 15435 | 12288 | -50 | 140(30) |
| H(25A) | 11288 | 10076 | 207 | 34 |
| H(26A) | 10541 | 9098 | -456 | 40 |
| H(26B) | 10701 | 10581 | -389 | 40 |
| H(27A) | 12835 | 9600 | -644 | 41 |
| H(27B) | 13087 | 8867 | -295 | 41 |
| H(28A) | 13259 | 11582 | -378 | 44 |
| H(28B) | 13344 | 10921 | -13 | 44 |
| H(29A) | 15524 | 9904 | -162 | 47 |
| H(29B) | 15415 | 10445 | -541 | 47 |
| H(8O) | 2172 | 11183 | 752 | 95(19) |
| H(30A) | 1489 | 11449 | 1286 | 90 |
| H(31A) | 1398 | 13621 | 1261 | 137 |
| H(31B) | -8 | 13106 | 1452 | 137 |
| H(31C) | -118 | 13570 | 1066 | 137 |
| H(32A) | -116 | 10144 | 1012 | 201 |
| H(32B) | -1090 | 11326 | 903 | 201 |
| H(32C) | -962 | 10939 | 1294 | 201 |

### EXAMPLE 6

### ((2R,3R)-3-(4-(4-cyanophenyl)thiazol-2-yl)-2-(2,4-difluorophenyl)-1-(1H-1,2,4-triazol-1-yl)butan-2-yloxy)methyl dihydrogen phosphate mono-lysine salt n-propyl alcohol solvate

Steps A, B and C of Example 1 were repeated, except the filtrate in Step C was concentrated and mixed with n-propyl alcohol (100 mL) and heated to 65°C to crystallize the solvate of the mono-lysine salt. The solvate was collected on a Buchner funnel and dried under vacuum to obtain the title solvate compound as a crystalline solid.

The PXRD data (see Figure 12), X-ray crystallographic data from single crystal (Tables 5-6) was collected, and a DSC (Figure 13) and TGA curve (Figure 14) were obtained for Example 6.

**Table 5: Crystal data and structure refinement for Example 6**

| | |
|---|---|
| Temperature | 293(2) K |
| Wavelength | 1.54178 A |
| Crystal system, space group | Orthorhombic, P2₁2₁2₁ |
| Unit cell dimensions | a = 9.0728(1)Å α = 90° |
| | b = 10.3764(1)Å β = 90° |
| | c = 38.7396(5)Å γ = 90° |
| Volume | 3647.06(7) Å³ |
| Z, Calculated density | 4, 1.373 Kg/m³ |
| Absorption coefficient | 1.798 mm⁻¹ |
| Crystal size | 0.25 x 0.15 x 0.10 mm |
| θ range for data collection | 2.28 to 65.20° |
| Limiting indices | -9<=h<=10, -11<=k<=11, - |
| | 45<=1<=41 |
| Reflections collected / unique | 19537 / 6047 [R(int) = 0.0742] |
| Completeness to θ = 65.34 | 98.0 % |
| Absorption correction | SADABS |
| Max. and min. transmission | 1.000 and 0.740 |
| Refinement method | Full-matrix least-squares on F² |
| Data / restraints / parameters | 6047 / 0 / 465 |
| Goodness-of-fit on F² | 1.050 |
| Final R indices [I>2<σ(I)] | R1 = 0.0526, wR2 = 0.1449 |
| R indices (all data) | R1 = 0.0539, wR2 = 0.1462 |
| Absolute structure parameter | 0.04(2) |
| Largest diff. peak and hole | 0.910 and -0.312 e.Å⁻³ |

**Table 6: Atomic coordinates ( x 10⁴) and equivalent isotropic displacement parameters (Å x 10³). U(eq) is defined as one third of the trace of the orthogonalized Uij tensor**

| | x | y | z | U (eq) |
|---|---|---|---|---|
| S(1) | 6078(1) | 3387(1) | 1093(1) | 50(1) |
| P(1) | 4526(1) | 9111(1) | 606(1) | 29(1) |
| O(1) | 5639(2) | 6391(2) | 1196(1) | 32(1) |
| O(2) | 5337(3) | 8336(2) | 918(1) | 42(1) |
| O(3) | 5784(3) | 10080(2) | 506(1) | 41(1) |
| O(4) | 3260(3) | 9840(3) | 752(1) | 48(1) |
| O(5) | 4181(3) | 8185(2) | 320(1) | 40(1) |
| N(1) | 9928(3) | 7531(3) | 862(1) | 45(1) |
| N(2) | 9784(3) | 6867(4) | 1416(1) | 56(1) |
| N(3) | 8526(3) | 7473(3) | 1314(1) | 35(1) |
| N(4) | 5304(3) | 3359(3) | 1727(1) | 35(1) |
| N(5) | -775(5) | -1299(4) | 2310(1) | 82(1) |
| F(1) | 3256(3) | 5349(2) | 1502(1) | 60(1) |
| F(2) | 1865(3) | 6725(3) | 2594(1) | 81(1) |
| C(1) | 8632(4) | 7860(4) | 989(1) | 41(1) |
| C(2) | 10577(4) | 6925(5) | 1128(1) | 55(1) |
| C(3) | 7292(4) | 7610(3) | 1558(1) | 36(1) |
| C(4) | 6205(3) | 6466(3) | 1542(1) | . 29(1) |
| C(5) | 4990(3) | 6613(3) | 1816(1) | 33(1) |
| C(6) | 36355(4) | 6002(3) | 1790(1) | 37(1) |
| C(7) | 2571(4) | 6012(4) | 2042(1) | 50(1) |
| C(8) | 2883(4) | 6693(5) | 2339(1) | 54(1) |
| C(9) | 4161(5) | 7343(5) | 2388(1) | 56(1) |
| C(10) | 5201(4) | 7306(4) | 2123(1) | 44(1) |
| C(11) | 4612(4) | 7294(3) | 1080(1) | 33(1) |
| C(12) | 7030(3) | 5164(3) | 1592(1) | 34(1) |
| C(13) | 7723(5) | 5021(4) | 1952(1) | 50(1) |
| C(14) | 6101(4) | 4001(3) | 1506(1) | 33(1) |
| C(15) | 4935(5) | 2194(4) | 1222(1) | 49(1) |
| C(16) | 4622(4) | 2330(3) | 1566(1) | 35(1) |
| C(17) | 3582(4) | 1499(3) | 1759(1) | 36(1) |
| C(18) | 2760(4) | 1997(3) | 2031(1) | 41(1) |
| C(19) | 1681(4) | 1265(4) | 2184(1) | 47(1) |
| C(20) | 1400(4) | 8 (3) | 2064 (1) | 43 (1) |
| C (21) | 2230 (5) | -496 (4) | 1802 (1) | 51(1) |
| C(22) | 3313(5) | 234(3) | 1646(1) | 49(1) |
| C(23) | 203(5) | -729(4) | 2209(1) | 56(1) |
| O(6) | 8147(3) | 9104(3) | 230(1) | 46(1) |
| O(7) | 8741(3) | 11066(2) | 42(1) | 50(1) |
| N(6) | 10896(3) | 8158(2) | 159(1) | 32(1) |
| N(7) | 15977(3) | 11804(3) | -178(1) | 50(1) |
| C(24) | 9035(3) | 9923(3) | 114(1) | 33(1) |
| C(25) | 10645(3) | 9514(3) | 54(1) | 29(1) |
| C(26) | 11055(4) | 9707(3) | -326(1) | 36(1) |
| C(27) | 12704(4) | 9646(3) | -403(1) | 35(1) |
| C(28) | 13546(4) | 10785(3) | -260(1) | 38(1) |
| C(29) | 15176(4) | 10662(3) | -317(1) | 40(1) |
| O(8) | 1348(4) | 11714(3) | 751(1) | 81(1) |
| C(30) | 88 (7) | 11246(6) | 952 (2) | 90(2) |
| C(31) | -80(8) | 11882(8) | 1265(2) | 105(2) |
| C (32) | -1357(11) | 11214 (9) | 1478 (2) | 144(4) |
| H(30) | 6537 | 9874 | 388 | 120(30) |
| H(1A) | 7901 | 8299 | 869 | 49 |
| H(2A) | 11516 | 6570 | 1113 | 66 |
| H(3A) | 6765 | 8400 | 1507 | 43 |
| H(3B) | 7682 | 7679 | 1791 | 43 |
| H(7A) | 1681 | 5580 | 2014 | 60 |
| H(9A) | 4338 | 7796 | 2590 | 67 |
| H(10A) | 6075 | 7762 | 2151 | 53 |
| H(11A) | 4040 | 7610 | 1274 | 40 |
| H(11B) | 3942 | 6888 | 918 | 40 |
| H(12A) | 7849 | 5167 | 1427 | 41 |
| H(13A) | 8219 | 4206 | 1967 | 75 |
| H(13B) | 6964 | 5064 | 2124 | 75 |
| H(13C) | 8419 | 5705 | 1989 | 75 |
| H(15A) | 4573 | 1543 | 1080 | 59 |
| H(18A) | 2941 | 2829 | 2110 | 49 |
| H(19A) | 1136 | 1599 | 2366 | 56 |
| H(21A) | 2061 | -1335 | 1727 | 61 |
| H(22A) | 3866 | -112 | 1466 | 59 |
| H(6NA) | 10650 | 8060 | 380 | 20(7) |
| H(6NB) | 11844 | 7963 | 131 | 58(13) |
| H(6NC) | 10348 | 7637 | 29 | 41(10) |
| H(7NA) | 15617 | 12000 | 32 | 61 |
| H(7NB) | 15847 | 12478 | -321 | 61 |
| H(7NC) | 16945 | 11626 | -160 | 61 |
| H(25A) | 11282 | 10068 | 194 | 35 |
| H(26A) | 10561 | 9052 | -462 | 43 |
| H(26B) | 10685 | 10538 | -401 | 43 |
| H(27A) | 13104 | 8859 | -305 | 42 |
| H(27B) | 12846 | 9612 | -651 | 42 |
| H(28A) | 13196 | 11568 | -369 | 45 |
| H(28B) | 13354 | 10855 | -14 | 45 |
| H(29A) | 15533 | 9888 | -205 | 48 |
| H(29B) | 15371 | 10583 | -562 | 48 |
| H(80) | 2096 | 11228 | 727 | 140 (30) |
| H(30A) | -804 | 11354 | 817 | 108 |
| H(30B) | 213 | 10332 | 996 | 108 |
| H(31A) | -322 | 12780 | 1225 | 127 |
| H(31B) | 834 | 11846 | 1395 | 127 |
| H(32A) | -1497 | 11663 | 1692 | 216 |
| H(32B) | -1095 | 10334 | 1524 | 216 |
| H(32C) | -2253 | 11238 | 1346 | 216 |

### COMPARATIVE EXAMPLE 1

### Bis-lysine salt of (2R,3R)-3-[4-(4-cyanophenyl)thiazol-2-yl]-2-(2,4-difluorophenyl)-1-(1H-1,2,4-triazol-1-yl)-2-[(dihydrogen phosphonoxy)methoxy]butane

The above obtained product of formula V from Example 1, after Steps A and B, was dissolved in methanol (75 mL). To this, L-lysine (1.8 g) was added, the pH maintained at 7.0 to 9.0, and the mixture heated at 60°C for 4.5 h. The hot reaction mixture was filtered through a bed of Celite. The filtrate was concentrated to about 5 mL, mixed with ethanol (100 mL) and heated to 65°C to crystallize the bis lysine salt. The salt was collected on a Buchner funnel and dried under vacuum to afford 3.71 g of the title compound as an off white crystalline solid.

### COMPARATIVE EXAMPLE 2

### Di-tris salt of (2R,3R)-3-[4-(4-cyanophenyl)thiazol-2-yl]-2-(2,4-difluorophenyl)-1-(1H-1,2,4-triazol-1-yl)-2-[(dihydrogen phosphonoxy)methoxy]butane

Comparative Example 1 (10 g, 11.3 mmol) was dissolved in water. 22.6 mL 1N HCl is added to pH 2.65, and 70 mL of ethyl acetate for extraction. The mixture was washed with 70 mL water. The free acid in the EtOAc layer was separated, wherein the aqueous layer was extracted with EtOAc (30 mL X 2), the EtOAc layer was concentrated in vacuo to afford 380 mg of glassy solid. 2.596 g of tris amine salt in 3.6 mL water (70-80°C) was added. A milky suspension was obtained. The reaction mixture was heated to 50-55°C for 2h, cooled to rt and stirred for 18 h. Filtration and rinsing with EtOAc followed. The di-tris salt was collected on a Buchner funnel and dried under vacuum to afford 7.92 g of the compound as an off white crystalline solid.

### COMPARATIVE EXAMPLE 3

### Tert-butyl amine salt of (2R,3R)-3-[4-(4-cyanophenyl)thiazol-2-yl]-2-(2,4-difluorophenyl)-1-(1H-1,2,4-triazol-1-yl)-2-[(dihydrogen phosphonoxy)methoxy]butane

A solution of product IV of Example 1, after Steps A and B, was dissolved in 50 mL of ethyl acetate and to this was added t-butyl amine (5.3 mL) under nitrogen. The reaction mixture was stirred at 40°C for about 1 hour to crystallize the product. The bis t-butyl amine salt was collected on a Buchner funnel and dried under vacuum to afford 2.21 g of the compound as an off white crystalline solid.

### COMPARATIVE EXAMPLE 4

### (2R, 3R)-3-[4-(4-cyanophenyl)thiazol-2-yl]-2-(2,4-difluorophenyl)-1-(1H-1,2,4-triazol-1-yl)-2-[(dihydrogen phosphonoxy)methoxy]butane, sodium salt

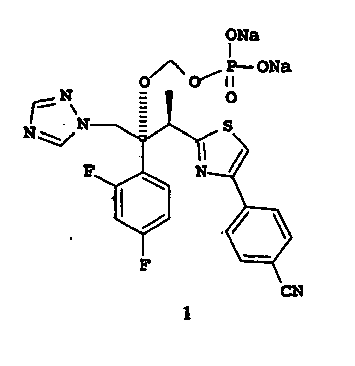

### Step A

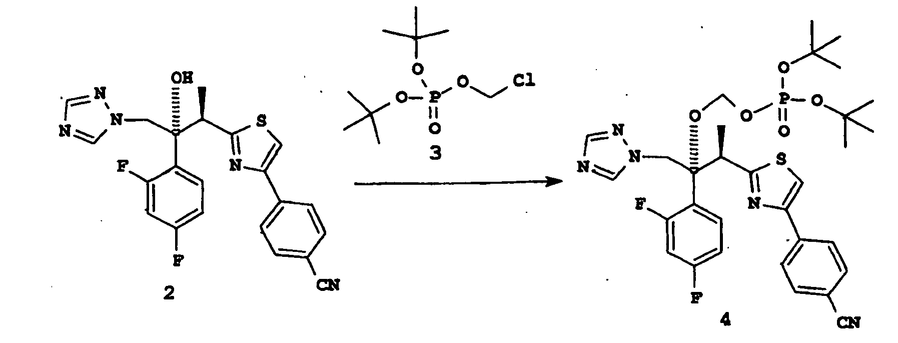

To a solution of (2R,3R)-3-[4-(4-cyanophenyl)thiazol-2-yl]-2-(2,4-difluorophenyl)-1-(1H-1,2,4-triazol-1-yl)butan-2-ol, 2, (8.74 g, 20 mmol) in THF (40 mL) under a nitrogen atmosphere was added sodium hydride (0.80 g, 60% in oil, 20 mmol) at rt. The resulting mixture was stirred at rt for 0.25 h and then di-tert-butyl chloromethyl phosphate, 3 (10.3 g, 40 mmol) is added. The reaction mixture was heated at 50°C for 16 h. The reaction mixture was then allowed to cool to rt and was concentrated under reduced pressure. The residue was dissolved in Et20 and is washed with H2O and brine. The organic layer was dried over MgSO4 and is concentrated under reduced pressure to obtain 17.0 g of crude compound, 4, as a gum. A small portion of this crude compound was purified by reverse phase chromatography on C-18. The column was eluted with 30% CH3CN/H2O, 38% CH3CN/H2O, 45% CH3CN/H2O and then 50% CH3CN/H2O. The product containing fractions are concentrated under reduced pressure in order to remove CH3CN. The resulting aqueous layer was then extracted with Et20. The Et20 layers are washed with brine, dried and concentrated under reduced pressure to afford purified compound, 4, as a white solid. The spectra data is as follows: 1H NMR (300 MHz, CDCl3): 8 8.35 (s, 1H), 7.98 (d, 2H, J=9), 7.76 (s, 1H), 7.71 (d, 2H, J=9), 7.63 (s, 1H), 7.36-7.27 (m, 1H), 6.86-6.78 (m, 2H), 5.53 (dd, 1H, J=28,6), 5.53 (dd, 1H, J=9,6), 5.17 (d, 1H, J=15), 5.03 (d, 1H, J=15), 4.01 (q, 1H, J=7), 1.47 (s, 9H), 1.45 (s, 9H), 1.37 (d, 3H, J=7). MS [ESI+ (M+H)+] 660.2 obs.

### Step B

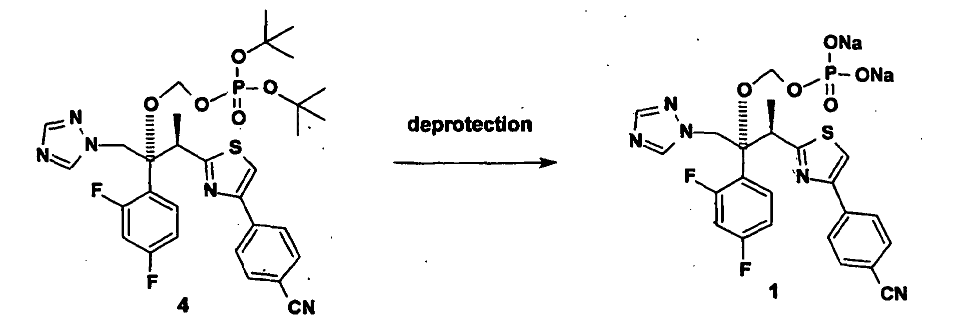

The crude (2R,3R)-3-[4-(4-cyanophenyl)thiazol-2-yl]-2-(2,4-difluorophenyl)-1-(1*H-*1,2,4-triazol-1-yl)-2-[(di-tert-butyl phosphonoxy)methoxy]butane, 4, (17 g) was dissolved in CH2Cl2 (100 mL). To this solution was added TFA (50 mL) and the reaction mixture was stirred at rt for 0.25 h. The reaction mixture was then concentrated under reduced pressure. To the residue was added H2O (200 mL), Et2O (100 mL) and EtOAc (100 mL). The pH of the aqueous layer was adjusted to 7.6 by addition of solid Na2CO3 and then the organic and aqueous layers are separated. The aqueous layer was then subjected to reverse phase chromatography on 400 g of C-18 eluted with H2O to 5% CH3CN/H2O. The product containing fractions are concentrated under reduced pressure, frozen and lyophilized to afford 1.5 g of the compound, 1, as a white solid. (1.5 g, 12% over two steps). The spectra data is as follows: 1H NMR (500 MHz, D2O) δ 8.91 (s, 1H), 7.92 (s, 1H), 7.81 (d, 2H, J=8), 7.80 (s, 1H), 7.77 (d, 2H, J=8), 7.21 (dd, 1H, J=15,9), 6,99 (ddd, 1H, J=9,9,2), 6.91 (ddd, 1H, J=9,9,2), 5.35 (dd, 1H, J=6,6), 5.29 (d, 1H, J=15), 5.21 (dd, 1H, J=6,6), 5.19 (d, 1H, J=15), 3.86 (q, 1H, J=7), and 1.35 (d, 3H, J=7) ; MS [(ESI- (M-H)- 546.1]; Anal. Calcd for C23H18F2N505S1P1/Na2/3.5 H2O: C, 42.21: H, 3.85: N, 10.70: Na, 7.03. Found: C, 42.32: H, 3.83: N, 10.60: Na, 7.04.

The di-tert-butyl chloromethyl phosphate, 3, may be made by any of the following methods.

### Method 1

Silver di-t-butyl phosphate (6.34 g, 20 mmol), which is prepared by mixing di-t-butyl phosphate (obtained from di-t-butyl phosphite by the method of Zwierzak and Kluba, Tetrahedron, Vol. 27, 3163 (1971)) with one equivalent of silver carbonate in 50% aqueous acetonitrile and by lyophilizing to dryness, is placed together with chloroiodomethane (35 g, 200 mmol) in benzene and stirred at room temperature for 18 hrs. The reaction mixture is filtered and the filtrate concentrated under reduced pressure. The residue is chromatographed on silica and eluted with 2:1 hexanes-ethyl acetate. Appropriate fractions are concentrated to dryness - to obtain the subtitled compound 3 (3.7 g, 71% yield): 1H NMR (CDCl3) δ 5.63 (d, 2H, J=17), 1.51 (s, 18H); MS (MH+ = 259).

### Method 2

Tetrabutylammonium di-t-butyl phosphate is prepared by dissolving di-t-butyl phosphate [20g, 94 mmol (obtained from di-t-butyl phosphite by the method of Zwierzak and Kluba, Tetrahedron, Vol. 27, 3163 (1971)] in methanolic tetrabutylammonium hydroxide (47 mL of 1M solution, 47 mmol). The reaction mixture has a temperature of 23°C and pH of 4.33. The pH of the reaction mixture is adjusted to 6.5-7.0 by addition of methanolic tetrabutylammonium hydroxide (48 mL of 1M solution, 48 mmol) over 0.2 h. The reaction mixture is stirred for 0.5 h at approximately 26°C and then is concentrated under reduced pressure at a bath temperature below 40°C. The crude residue is azeotroped three times by adding toluene (3x100 mL) and then the mixture is concentrated under reduced pressure. The crude residue is then triturated in cold hexanes (0°C) for 1 h and then the solid is collected by filtration, washed with a minimum amount of cold hexanes and dried to give a first crop of tetrabutylammonium di-t-butyl phosphate as a white solid. (24.0 g). The mother liquor is concentrated under reduced pressure and then triturated in cold hexanes (20 mL) for 1h. The solid is collected by filtration, washed with a minimum amount of cold hexanes and dried to give a second crop of tetrabutylammonium di-t-butyl phosphate as a white solid. [(8.5g), 32.5 g total (77%)]. A solution of tetrabutylammonium di-t-butyl phosphate (218 g, 480 mmol) in benzene (200 mL) is added dropwise to stirred chloroiodomethane (800 g, 4535 mmol) over 1.5 h at rt. The reaction mixture is stirred an additional 1.5 h at rt and then is concentrated under reduced pressure. The oily residue is dissolved in Et2O and filtered to remove white solids that precipitates. The organic layer is washed with saturated NaHCO3 and H2O/brine (1/1). The organic layer is then dried over magnesium sulfate, filtered and concentrated under reduced pressure to yield a red brown oil (320 g). The red brown oil is subjected to chromatography on silica gel (800 g) eluted with 20% EtOAc/Hexanes, 25% EtOAc/Hexanes then 30% EtOAc/Hexanes. The product containing fractions are concentrated under reduced pressure to yield a golden oil. The oil is diluted with CH2Cl2 (30 mL), concentrated under reduced pressure and then dried under vacuum to yield the compound 3 (61.3g, 49% yield). 1H NMR (Benzene-d6) 8 5.20 (2H, d, J=15), 1.22 (18H, s).

### Method 3

Iodochloromethane (974 g, 402 mL, 5.53 mol) at 25°C is treated with tetrabutylammonium di-t-butylphosphate (250 g, 0.553 mol). The phosphate is added portionwise over 10 minutes. The heterogeneous mixture becomes a clear pink solution after approximately 15 minutes. The mixture is stirred for three hours, and the iodochloromethane is then removed by rotary evaporation with a bath temperature of <30°C. The residue is taken up in 1 L t-butyl methyl ether and stirred for 15 minutes to precipitate tetrabutylammonium iodide by-product. Tetrabutylammonium iodide is removed by vacuum filtration through a sintered glass funnel. The filtrate is concentrated by rotary evaporation to an oil which contains a 5:1 mixture of 3" and undesired dimer impurity:

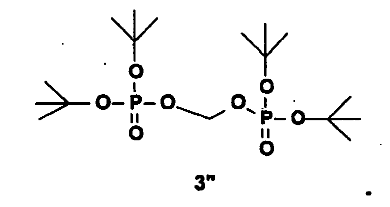

The mixture can be purified by a silica gel chromatography to obtain 3 as pure compound in ∼60% yield as an oil.

### Crystalline Data and Physical-Chemical Properties

Single-crystals of solvate forms for the mono-lysine salts are analyzed by crystallography, and the ethanol solvate and n-propyl alcohol solvate are found to be isomorphous with the isopropyl alcohol solvate (Table 7).

**Table 7: Crystalline Data**

| Solvate Compound | Solvent Sites for Z' | Sol vent % (w/w) | Z' | Vm | Space Group | d_{calc} g/cc | Solu bility (mg/ ml) |
|---|---|---|---|---|---|---|---|
| Mono-lysine salt (ethanol) | EtOH | 5.9 | 4 | 896 | P2₁2₁2₁ | 1.371 | >200 |
| Mono-lysine salt (isopropyl alcohol) | iPA | 7.5 | 4 | 908 | P2₁2₁2₁. | 1.378 | - |
| Mono-lysine salt (n-propyl alcohol) | nPA | 7.5 | 4 | 911 | P2₁2₁2₁ | 1.373 | - |

The obtained solvate of the mono-lysine salt of ((2R,3R)-3-(4-(4-cyanophenyl)thiazol-2-yl)-2-(2,4-difluorophenyl)-1-(1*H-*1,2,4-triazol-1-yl)butan-2-yloxy)methyl dihydrogen phosphate is scaled up and fully characterized. Physical-chemical properties including solubility, stability, moisture uptake, compaction, etc., are evaluated and compared to the bis-lysine salt of Comparative Example 1.

The mono-lysine salt or solvate thereof demonstrates greatly improved hygroscopicity (Figures 1, 2), especially at higher RH values (50% uptake relative to the bis-lysine salt of Comparative Example 1 at 90% RH). For instance, Comparative Example 1 has about 10% change in weight at 60% RH (Fig. 1), whereas Example 1 has about 2-2.5% change in weight at the same humidity level (Fig. 2). On average, the mono-lysine salt or solvate thereof has better stability over the bis-lysine form (Fig. 4).

Moisture-uptake data for with the isopropyl alcohol solvate shows greater improvement (Figure 3). For instance, there is less than 1% change in weight at 60% RH (Fig. 3).

Also, Example 1 retains a high aqueous solubility (>200 mg/mL) similar to that of the bis-lysine salt of Comparative Example 1.

Crystals of mono-lysine salt or solvate thereof are grown with ease and exhibit a more desired morphology. Physicochemical studies illustrate superior solution and physical stability comparing to bis-lysine salt. Based on 2-week stability data collected with material stored under stressed stability conditions, crystalline ethanol solvate shows good stability at low relative humidity and at high temperatures (Table 8) with minimal degradation at 40°C/75% RH (open and closed). No degradation is seen under any other storage conditions.

The low hygroscopicity of the present invention in turn affords physical stability and material handling that makes the mono-lysine salts of formula I and solvates thereof suitable as oral solids as well as an intravenous dosage forms (Table 8). Another major advantage of the mono-lysine salt or solvate, in addition to its enhanced physical stability, is the reduced drug loading and consequentially better processability.

**Table 8: Physical stability of Solvate of Example 1**

| Storage conditions | Initial | 5 Days | 1 Week | 2 Weeks |
|---|---|---|---|---|
| | Area Percent (AP as phosphonoxoxymethyl ether derivative of ravuconazole) | | | |
| | 99.56 | | | |
| 5° C | | 99.59 | 98.47 | 99.60 |
| 25° C/60% RH (Open) | | 99.61 | 99.65 | 99.42 |
| 25° C/60% RH (Closed | | 99.59 | 99.66 | 99.21 |
| 40° C/75% RH (Open) | | 98.97 | 98.76 | 96.90 |
| 40° C/75% RH (Closed | | 99.20 | 99.05 | 97.90 |
| 60° C | | 99.70 | 99.70 | 99.73 |
| HIL/UV (Controlled) | | 99.60 | 99.64 | 99.68 |
| HIL/UV (Exposed) | | 99.61 | 98.68 | 99.68 |

### EXAMPLES 7-9

Examples 1-3 are used to make pharmaceutical compositions Examples 4-6 as follows.

### EXAMPLE 7

2.5 mg of the compound of Example 1 is mixed with starch, mannitol, microcrystalline cellulose and magnesium stearate, wherein suitable ingredients and amounts can be determined by one of ordinary skill in the art, and then compacted to form a tablet.

### EXAMPLE 8

2.5 mg of the compound of Example 2 is converted into lyophilized form, mixed with sterile water, vegetable oil and polyethylene glycol, wherein suitable ingredients and amounts can be determined by one of ordinary skill in the art, to produce a pharmaceutical solution.

### EXAMPLE 9

2.5 mg of the compound of Example 3 is mixed with mineral oil, propylene glycol, liquid petrolatum, emulsifying wax and water (active ingredient is about 0.01% w/w of 1% by weight of the formulation), wherein suitable ingredients and amounts can be determined by one of ordinary skill in the art, to produce a waxy ointment.

### EXAMPLES 10-12

Examples 7-9 are administered to a subject as follows.

### EXAMPLE 10

Example 7 is orally administered twice a day to a first set of mice that are systemically infected with *Candida albicans* for the duration of two weeks. Another set of mice also systemically infected with *Candida albicans* is treated but four times a day for two weeks. Observation of infection is carried out each day for 7 days for each group.

### EXAMPLE 11

Example 8 is intravenously administered twice a day to mice systemically infected with Cryptococcus *neoformans* for the duration of one week. Observation of infection is carried out each day for 14 days.

### EXAMPLE 12

The cream of Example 9 is topically administered to mice infected with *Trichophyton* species twice a day for one week. Observation of infection is carried out each day for 7 days.

The salts and solvates thereof of the invention exhibit excellent antifungal activity whether administered orally, parenterally or topically.

### Industrial Applicability

According to the present invention, there is provided mono-lysine salts of azole compounds, or pharmaceutically acceptable solvates thereof, of the formula I: 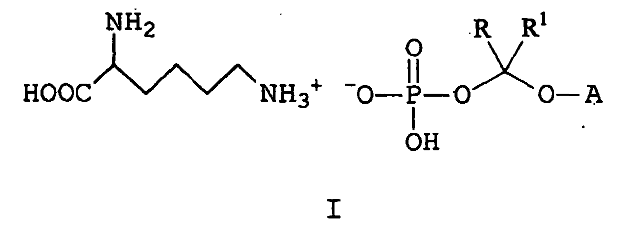 wherein each of R and R1 is a hydrogen atom or a (C1-C6)alkyl group, and A represents the non-hydroxy portion of a triazole antifungal salt compound of the type containing a secondary or tertiary hydroxyl group. These mono-lysie salts or pharmaceutically acceptable solvates thereof according to the present invention are useful for the treatment of, for instance, serious systemic fungal infections.

## Claims

1. A mono-lysine salt of a compound of formula I, or a solvate thereof: 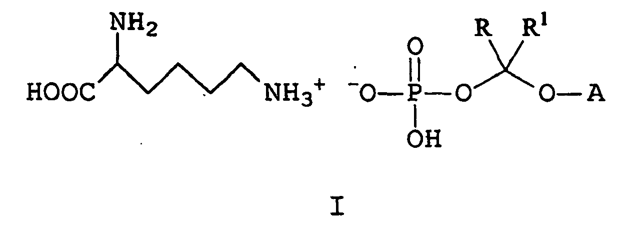 wherein each of R and R¹ is a hydrogen or (C₁-C₆) alkyl: and
A is selected from the group consisting of: 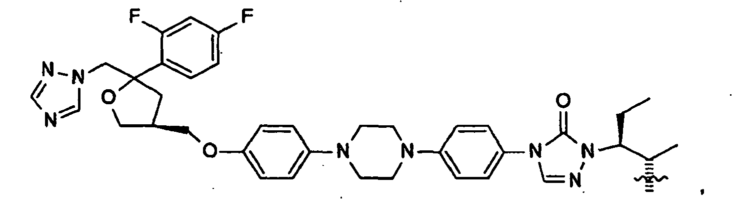 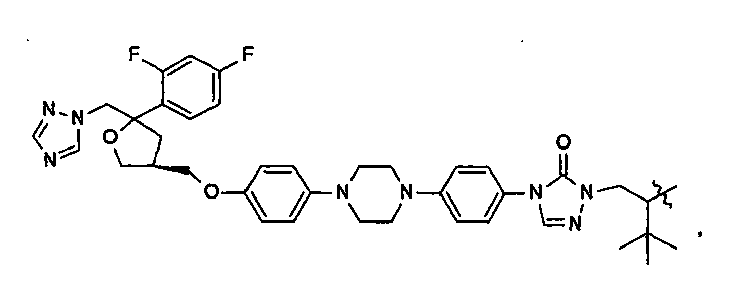 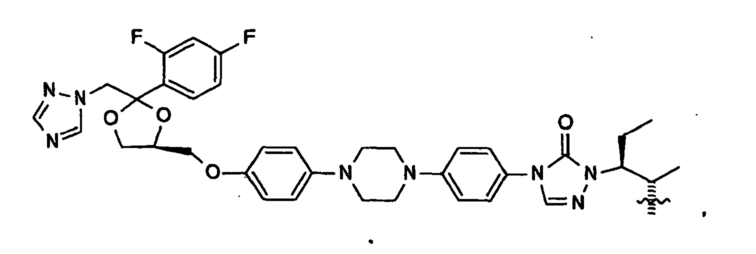 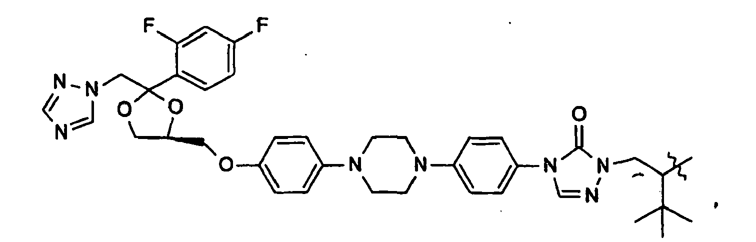 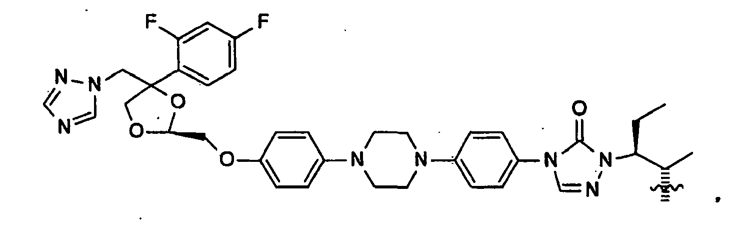 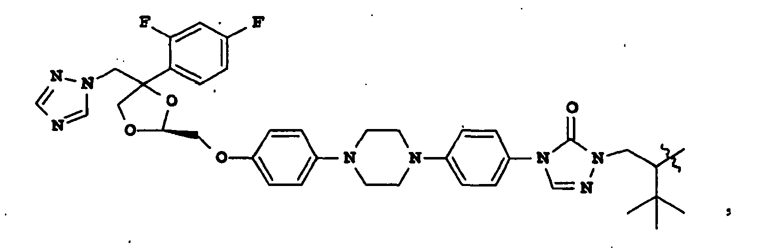 and a formula (i): 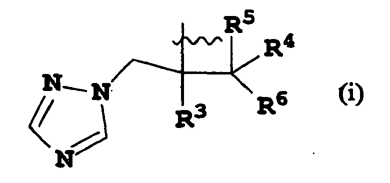 wherein in formula (i)
R³ represent a phenyl group substituted by one or more halogen atoms;
R⁴ represents a hydrogen or CH₃;
R⁵ represents a hydrogen, or taken together with R⁴ represent =CH₂; and
R⁶ represents a thiazolyl, pyrimidinyl or triazolyl wherein each ring is optionally substituted by one or more groups selected from the group consisting of a halogen, =O, CH=CH- (C₆H₄) -OCH₂CF₂CHF₂, and a phenyl substituted by one or more groups selected from the group consisting of CN and OCH₂CF₂CHF₂, or a phenyl substituted by one or more groups selected from the group consisting of a halogen and methylpyrazolyl.

2. The mono-lysine salt or solvate thereof according to claim 1, wherein A represents the formula (i): 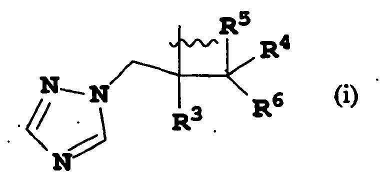 wherein
R³ represents a phenyl group substituted by one or more halogen atoms;
R⁴ represents a hydrogen or CH₃;
R⁵ represents a hydrogen, or taken together with R4 represent =CH₂; and
R⁶ represents a thiazolyl, pyrimidinyl or triazolyl wherein each ring is optionally substituted by one or more groups selected from the group consisting of a halogen, =O, CH=CH- (C₆H₄) -OCH₂CF₂CHF₂, and a phenyl substituted by one or more groups selected from the group consisting of CN and OCH₂CF₂CHF₂, or a phenyl substituted by one or more groups selected from the group consisting of a halogen and methylpyrazolyl.

3. The mono-lysine salt or solvate thereof according to claim 1 or 2, wherein R³ of formula (i) is 2,4-difluorophenyl.

4. The mono-lysine salt or solvate thereof according to any one of claims 1 to 3, wherein R⁴ of formula (i) is methyl and R⁵ is hydrogen.

5. The mono-lysine salt or solvate thereof according to any one of claims 1 to 4, wherein R⁶ of formula (i) is 4-(4-cyanophenyl)-thiazol-2-yl.

6. The mono-lysine salt or solvate thereof according to any one of claims 1 to 5, wherein each of R and R¹ of formula I is hydrogen.

7. The mono-lysine salt or solvate thereof according to claim 1 or 2, wherein A is 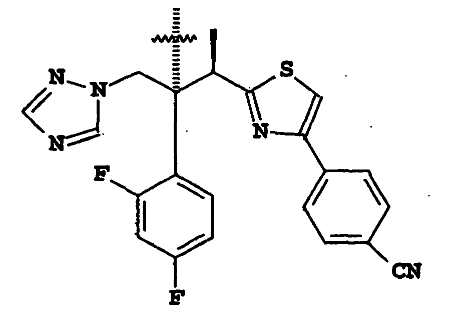 or

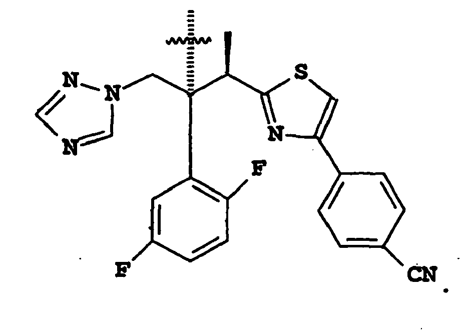

8. The mono-lysine salt or solvate thereof according to claim 1, wherein A is selected from the group consisting of 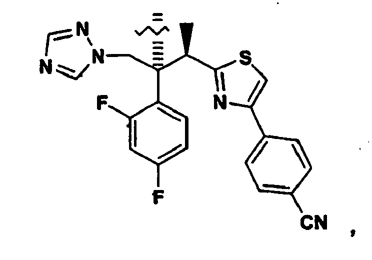 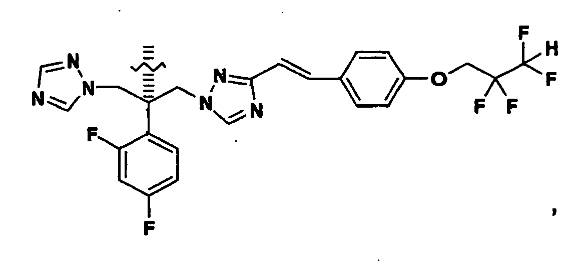 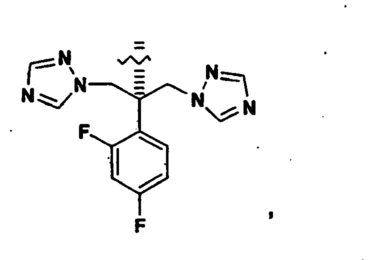 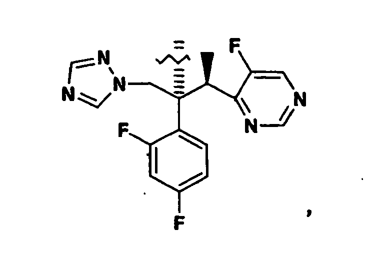 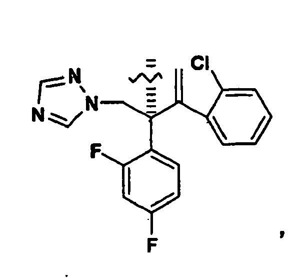 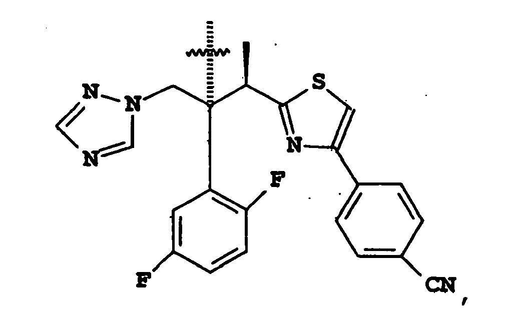 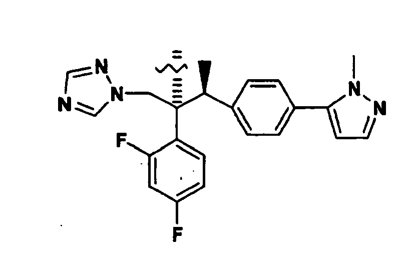 and

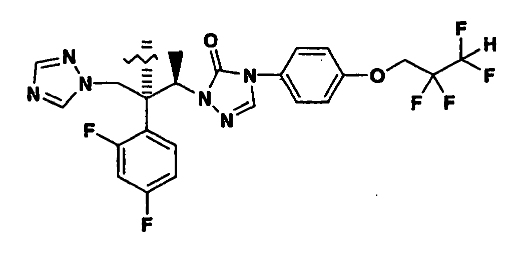

9. The mono-lysine salt or solvate thereof according to claim 1, wherein said A is selected from the group consisting of: 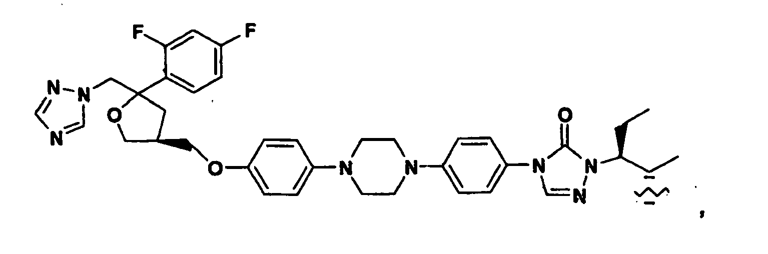 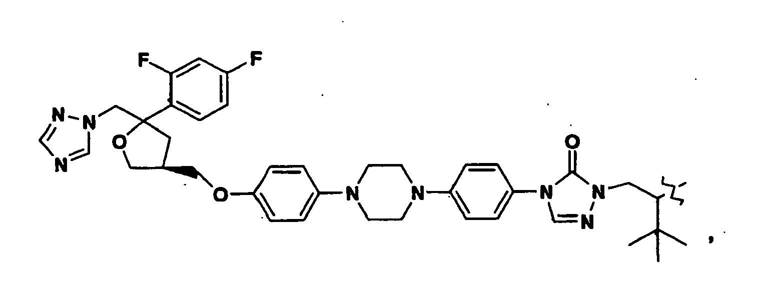 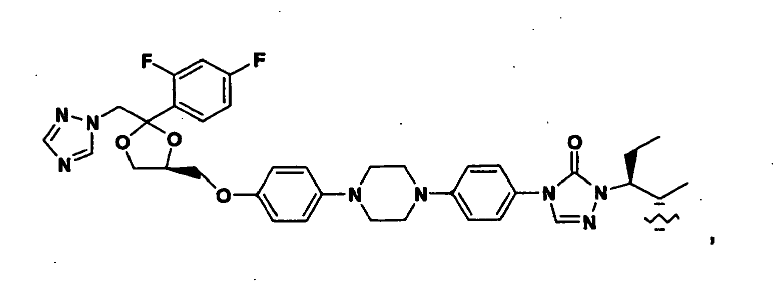 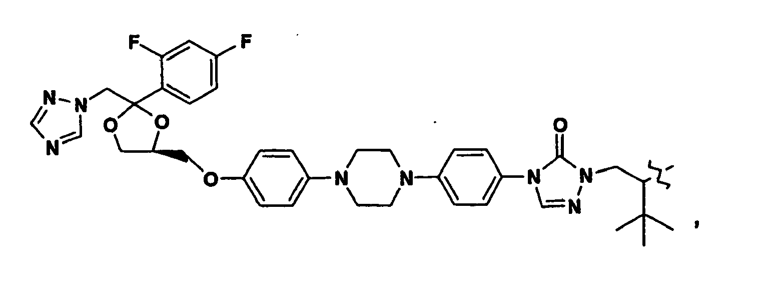 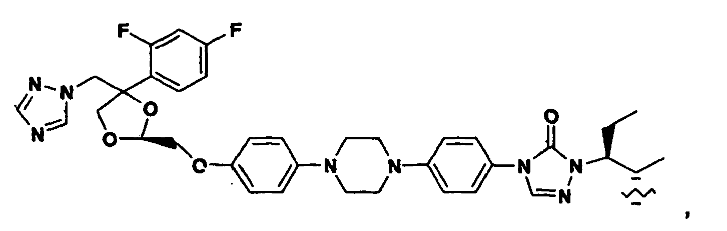 and

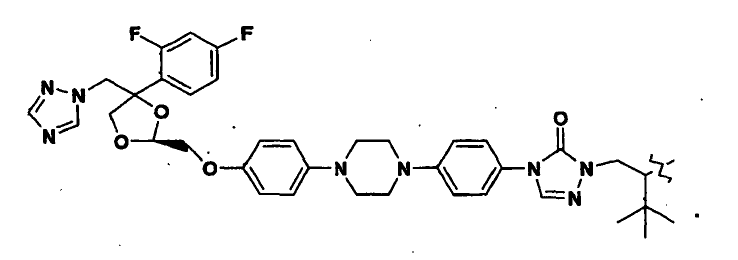

10. A solvate of the mono-lysine salt according to any one of claims 1 to 9, wherein said solvate thereof is an ethanol solvate.

11. A solvate of the mono-lysine salt according to any one of claims 1 to 9, wherein said solvate thereof is an isopropyl alcohol solvate.

12. A solvate of the mono-lysine salt according to any one of claims 1 to 9, wherein said solvate thereof is a n-propyl alcohol solvate.

13. A pharmaceutical composition comprising:
the mono-lysine salt according to any one of claims 1 to 9, or a pharmaceutically acceptable solvate thereof; and
a pharmaceutically acceptable adjuvant, diluent, or carrier.

14. Mono-lysine salt according to any one of claims 1 to 9, or a pharmaceutically acceptable solvate thereof for use in a method for treatment of fungal infections.

15. A process for the preparation of a water-soluble mono-lysine salt of the formula I: 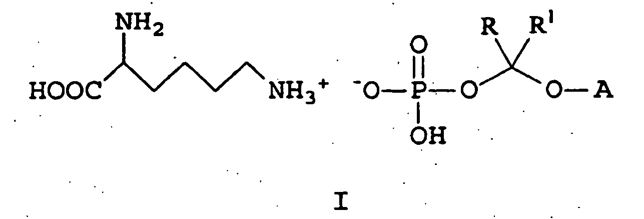 wherein each of R and R¹ is a hydrogen or (C₁-C₆) alkyl ; and
A is selected from the group consisting of: 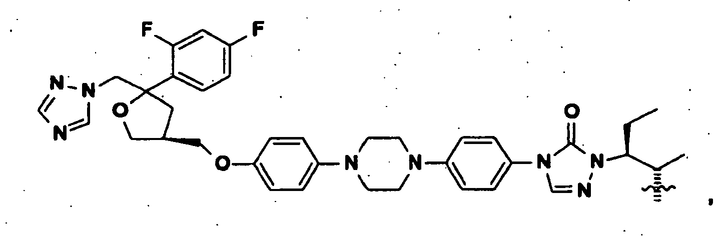 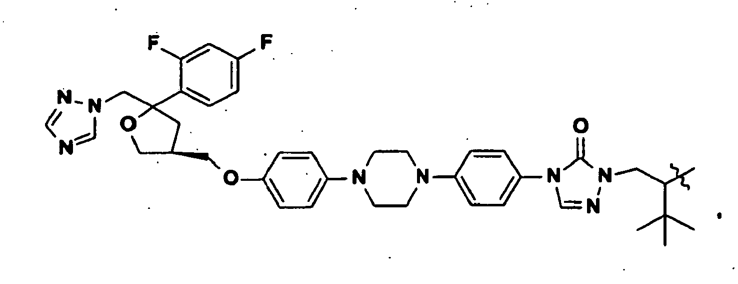 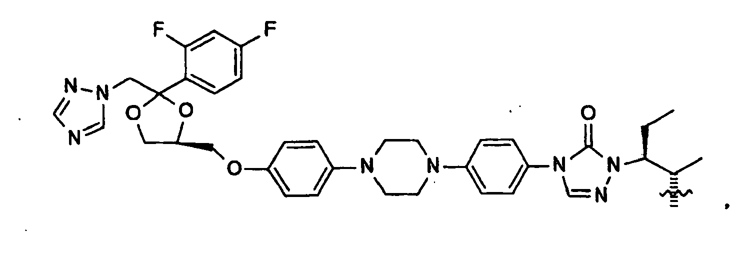 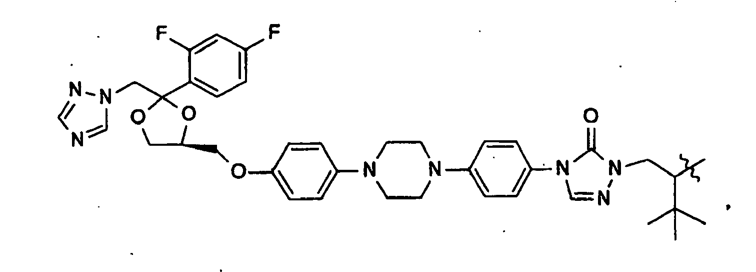 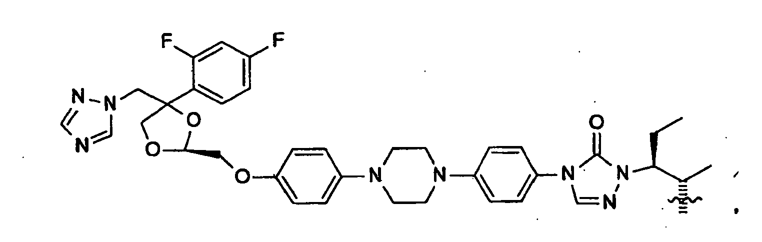 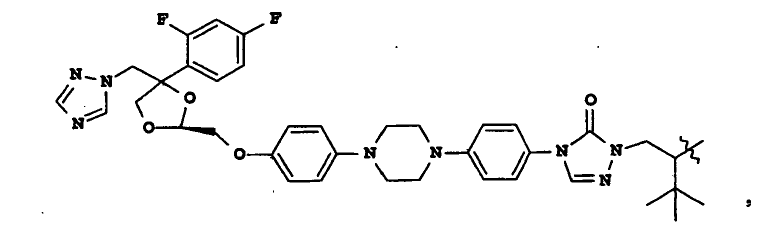 and a formula (i): 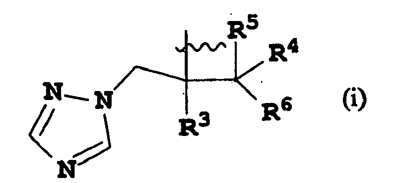 wherein in formula (i)
R³ represents a phenyl group substituted by one or more halogen atoms;
R⁴ represents a hydrogen or CH₃;
R⁵ represents a hydrogen, or taken together with R⁴ represent =CH₂; and
R⁶ represents a thiazolyl, pyrimidinyl or triazolyl wherein each ring is optionally substituted by one or more groups selected from the group consisting of a halogen, =O, CH=CH- (C₆H₄) -OCH₂CF₂CHF₂, and a phenyl substituted by one or more groups selected from the group consisting of CN and OCH₂CF₂CHF₂, or a phenyl substituted by one or more groups selected from the group consisting of a halogen and methylpyrazolyl;
said method comprising:
(a) reacting a compound of formula A-OH wherein A is as defined above in formula I with a compound of formula III: 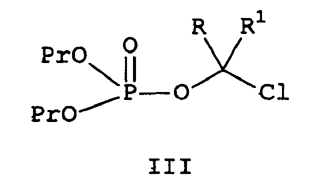 wherein R and R¹ in formula III are each independently hydrogen or (C₁-C₆)alkyl, and Pr represents a hydroxyl-protecting group; said reaction is in an inert organic solvent in the presence of base at a temperature of from about 25°C to 50°C to form a first intermediate of formula IV: 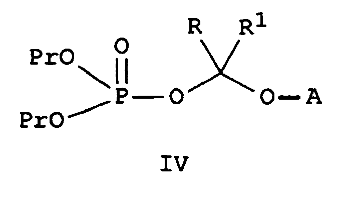 wherein R and R¹ in formula IV are each independently hydrogen or (C₁-C₆)alkyl, Pr represents a hydroxyl-protecting group, and A is as defined in formula I;
(b) removing the protecting groups Pr of formula IV with an organic solvent to form a second intermediate of formula V: 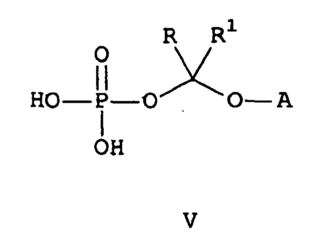 wherein R and R¹ in formula V are each independently hydrogen or (C₁-C₆)alkyl, and A is as defined in formula I; and
(c) reacting said second intermediate of formula V with lysine in a solvent at a pH in the range of 4.2-5.5 to produce said mono-lysine salt of formula I.

16. The process according to claim 15, wherein the protecting group of Pr is tertiary-butyl.

17. The process according to claim 15 or 16, wherein the solvent in step (a) is tetrahydrofuran.

18. The process according to any one of claims 15 to 17, wherein the base used in step (a) is sodium hydride.

19. A process for the preparation of a water-soluble solvate of a mono-lysine salt, said mono-lysine salt having the formula I: 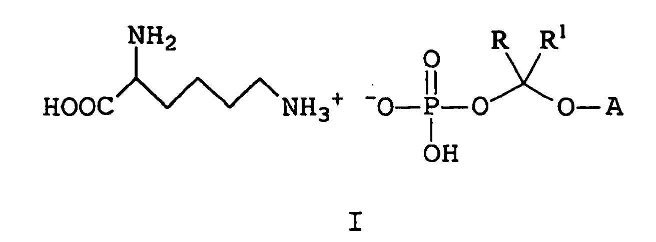 wherein each of R and R¹ is a hydrogen or (C₁-C₆) alkyl: and
A is selected from the group consisting of: 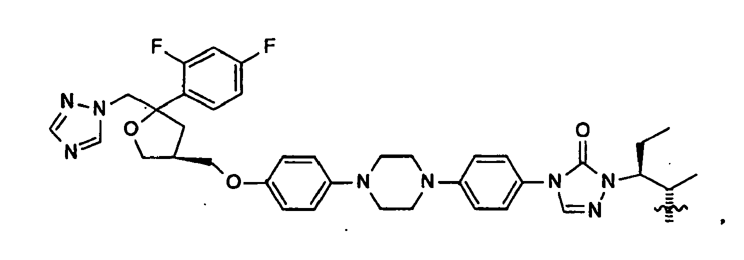 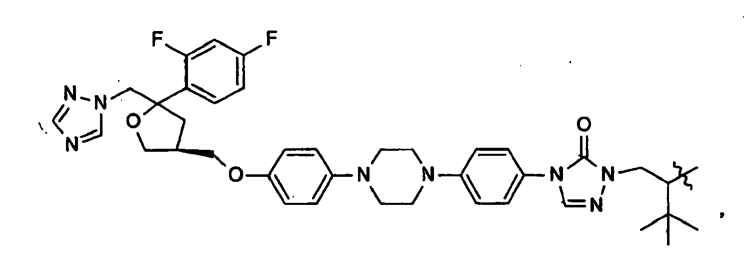 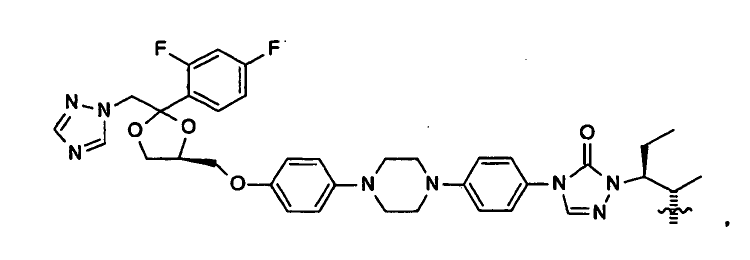 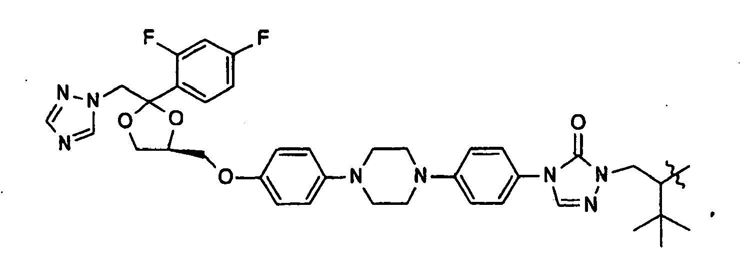 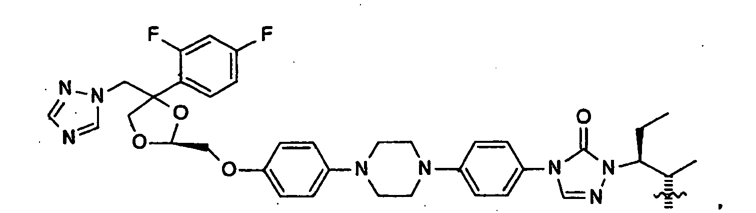 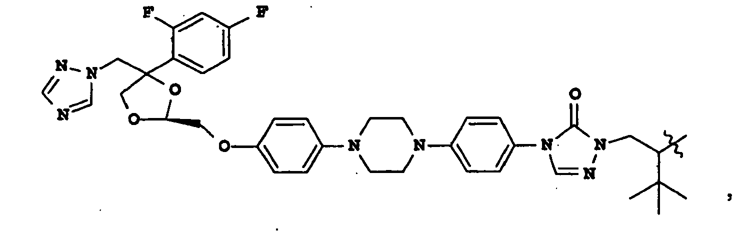 and a formula (i): 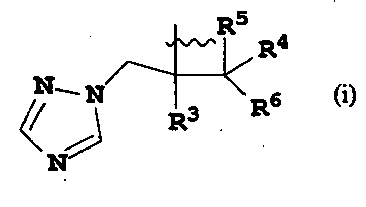 wherein in formula (i)
R³ represents a phenyl group substituted by one or more halogen atoms;
R⁴ represents a hydrogen or CH₃;
R⁵ represents a hydrogen, or taken together with R⁴ represent =CH₂; and
R⁶ represents a thiazolyl, pyrimidinyl or triazolyl wherein each ring is optionally substituted by one or more groups selected from the group consisting of a halogen, =O, CH=CH- (C₆H₄) -OCH₂CF₂CHF₂, and a phenyl substituted by one or more groups selected from the group consisting of CN and OCH₂CF₂CHF₂, or a phenyl substituted by one or more groups selected from the group consisting of a halogen and methylpyrazolyl;
said method comprising:
(a) reacting a compound of formula A-OH wherein A is as defined above in formula I with a compound of formula III: 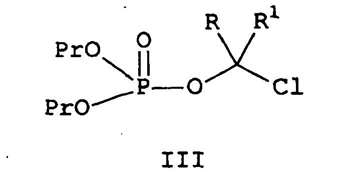 wherein R and R¹ in formula III are each independently hydrogen or (C₁-C₆)alkyl, and Pr represents a hydroxyl-protecting group; said reaction is in an inert organic solvent in the presence of base at a temperature of from about 25°C to 50°C to form a first intermediate of formula IV: 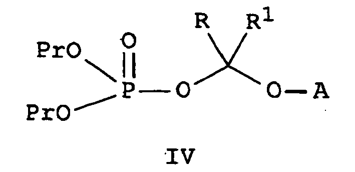 wherein R and R¹ in formula IV are each independently hydrogen or (C₁-C₆)alkyl, Pr represents a hydroxyl-protecting group, and A is as defined in formula I;
(b) removing the protecting groups Pr of formula IV with an organic solvent to form a second intermediate of formula V: 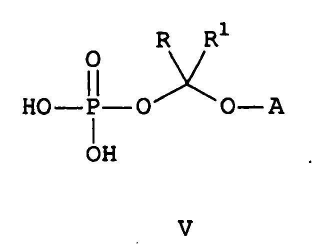 wherein R and R¹ in formula V are each independently hydrogen or (C₁-C₆)alkyl, and A is as defined in formula I;
(c) reacting said second intermediate of formula V with lysine in a solvent at a pH in the range of 4.2-5.5 to produce said mono-lysine salt of formula I; and
(d) crystallizing said mono-lysine salt in a solvent to produce the solvate of said mono-lysine salt.

20. The process according to claim 19, wherein the solvent in step (d) is aqueous ethanol.

21. The process according to claim 19 or 20, wherein the solvent in step (d) is aqueous isopropyl alcohol.

22. The process according to any one of claims 19 to 21, wherein the solvent in step (d) is aqueous n-propyl alcohol.

23. The process according to any one of claims 15 to 22, wherein A of starting material A-OH is the formula (i):

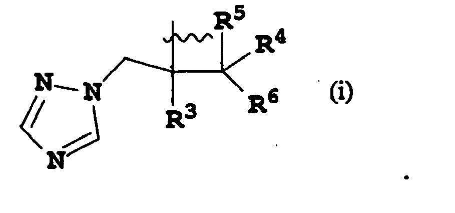

24. A mono-lysine salt of ((2R, 3R)-3-(4-(4-cyanophenyl)thiazol-2-yl)-2-(2,4-difluoroahenyl)-1-(1*H-*1,2,4-triazol-1-yl)butan-2-yloxy)methyl dihydrogen phosphate having the structure: 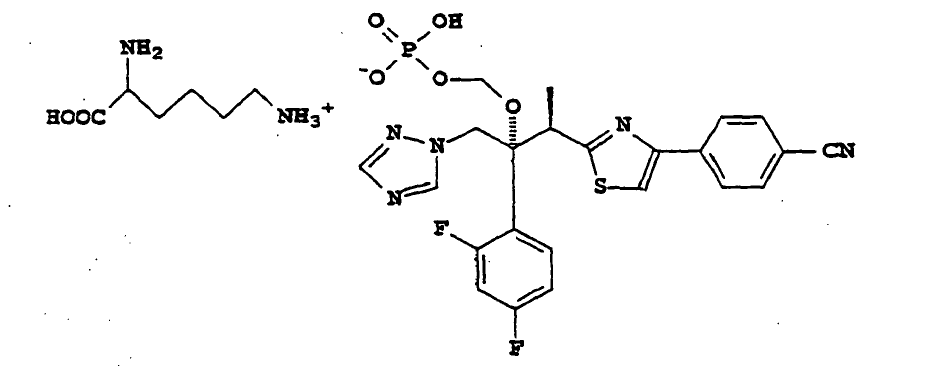 or a pharmaceutically acceptable solvate thereof.

25. The mono-lysine salt or solvate thereof according to claim 24, said salt or solvate thereof is in crystalline form.

26. A solvate of the mono-lysine salt according to claim 24, wherein said solvate thereof is an ethanol solvate.

27. A solvate of the mono-lysine salt according to claim 24, wherein said solvate thereof is an isopropyl alcohol solvate.

28. A solvate of the mono-lysine salt according to claim 24, wherein said solvate thereof is a n-propyl alcohol solvate.

29. A pharmaceutical composition comprising:
an effective amount of the mono-lysine salt according to claim 24, or the pharmaceutically acceptable solvate thereof; and
a pharmaceutically acceptable adjuvant, diluent, or carrier.

30. The pharmaceutical composition according to claim 29, wherein said composition is a tablet, capsule, powder, solution, suspension, emulsion, ointment, lotion, cream or spray.

31. Use of the mono-lysine salt according to claim 24, or the pharmaceutically acceptable solvate thereof for the preparation of a medicament for the treatment of fungal infections.

32. Use according to claim 31, wherein the medicament is administered only.

33. Use according tao claim 31, wherein the medicament is administered parenterally.

34. A process for the preparation of a water-soluble mono-lysine salt of the following formula: 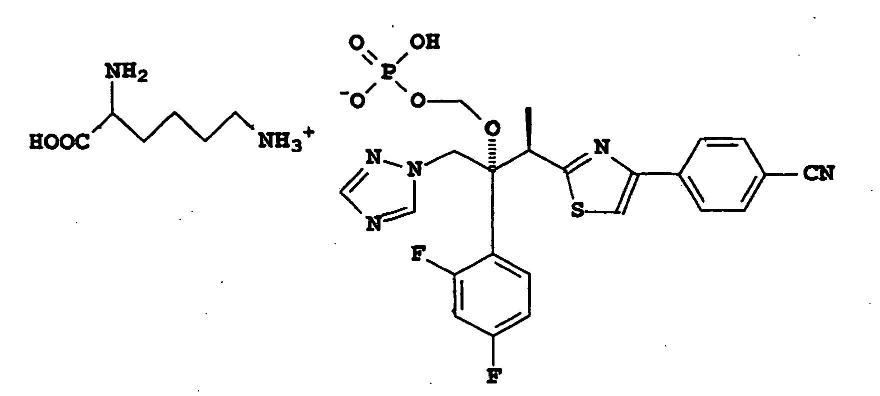 said method comprising:
(a) reacting a compound of formula B: 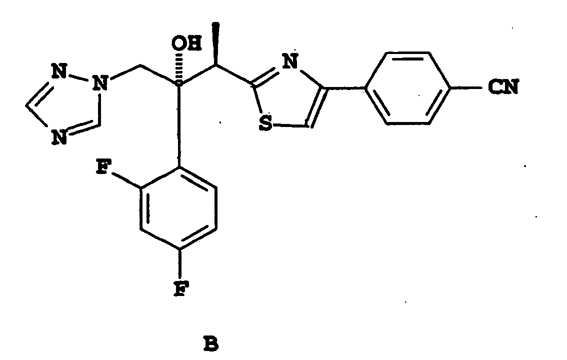 with a compound of formula III': 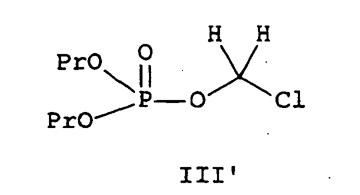 wherein Pr of formula III' represents a hydroxyl-protecting group;
said reaction is in an inert organic solvent in the presence of base at a temperature of from about 25°C to 50°C to form a first intermediate of formula IV': 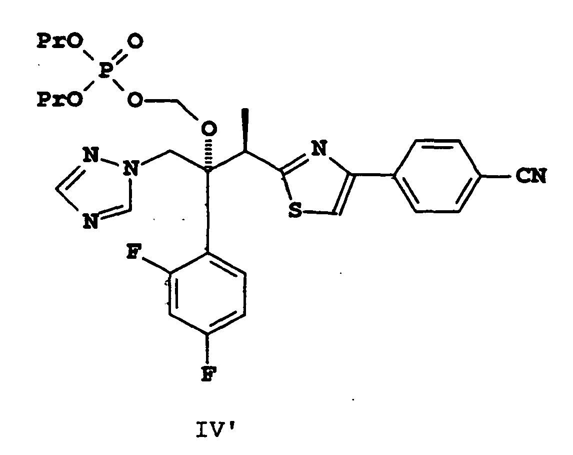 wherein Pr of formula IV' represents a hydroxyl-protecting group;
(b) removing the protecting groups Pr of formula IV' with organic solvent to form a second intermediate of formula V': 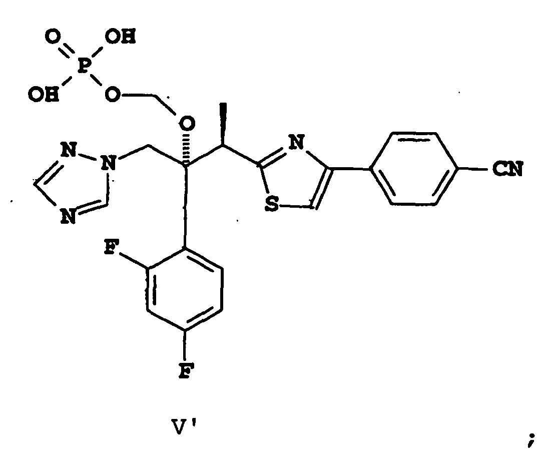 and
(c) reacting said second intermediate of formula V' with lysine in a solvent at a pH in the range of 4.2-5.5 to produce said mono-lysine salt.

35. The process according to claim 34, wherein the protecting group of Pr is tertiary-butyl.

36. The process according to claim 34 or 35, wherein the solvent in step (a) is tetrahydrofuran.

37. The process according to any one of claims 34 to 36, wherein the base used in step (a) is sodium hydride.

38. A process for the preparation of a water-soluble solvate of a mono-lysine salt, said mono-lysine salt having the formula: 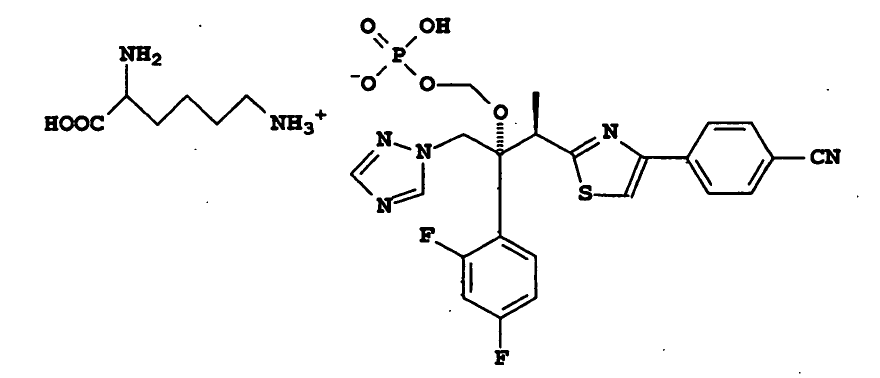 said method comprising:
(a) reacting a compound of formula B: 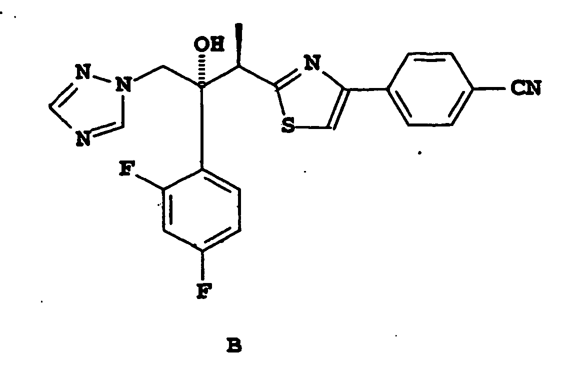 with a compound of formula III': 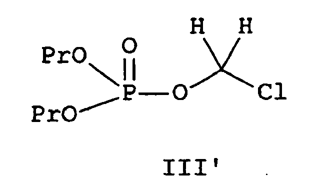 wherein Pr of formula III' represents a hydroxyl-protecting group; said reaction is in an inert organic solvent in the presence of base at a temperature of from about 25°C to 50°C to form a first intermediate of formula IV': 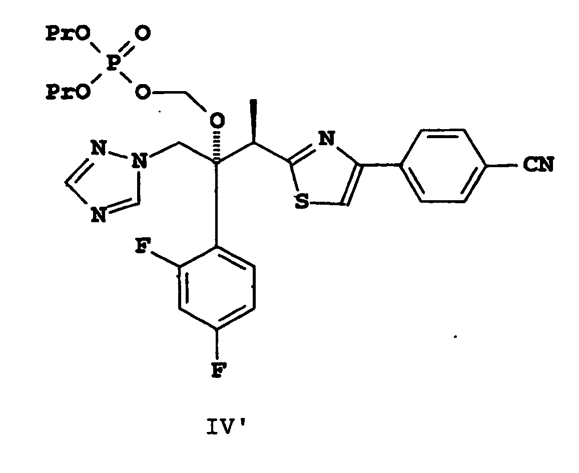 wherein Pr of formula IV' represents a hydroxyl-protecting group;
(b) removing the protecting groups Pr of formula IV' with organic solvent to form a second intermediate of formula V': 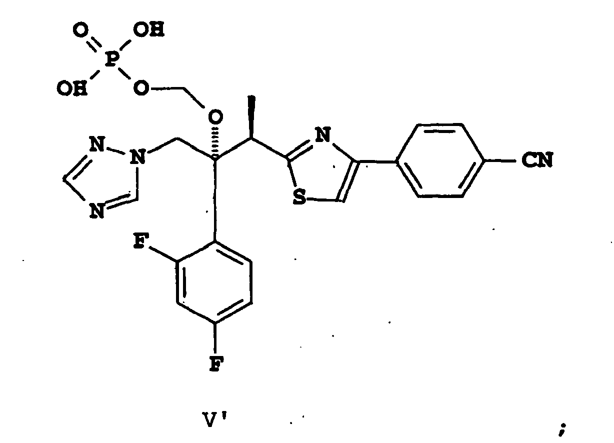
(c) reacting said second intermediate of formula V' with lysine in a solvent at a pH in the range of 4.2-5.5 to produce the mono-lysine salt; and
(d) crystallizing said mono-lysine salt in a solvent to produce the solvate of said mono-lysine salt.

39. The process according to claim 38, wherein the solvent in step (d) is aqueous ethanol.

40. The process according to claim 38 or 39, wherein the solvent in step (d) is aqueous isopropyl alcohol.

41. The process according to any one of claims 38 to 40, wherein the solvent in step (d) is aqueous n-propyl alcohol.

42. A use of the mono-lysine salt according to any one of claims 1 to 9 or a pharmaceutically acceptable solvate thereof for the manufacture of an agent for treating fungal infections.

43. Mono-lysine salt according to claim 24 or a pharmaceutically acceptable solvate thereof for use in a method for the treatment of fungal infections.

## Patentansprüche

1. Monolysinsalz einer Verbindung der Formel (I) oder ein Solvat davon: 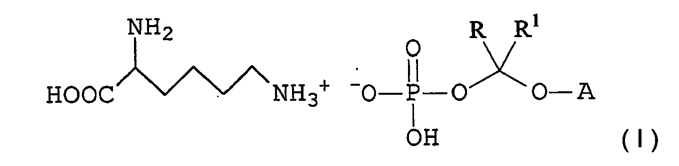 worin jedes von R und R¹ Wasserstoff oder (C₁₋₆)-Alkyl ist; und
A ausgewählt ist aus der Gruppe bestehend aus: 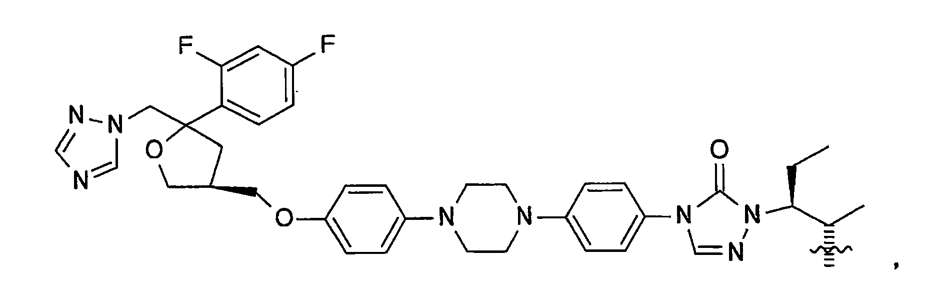 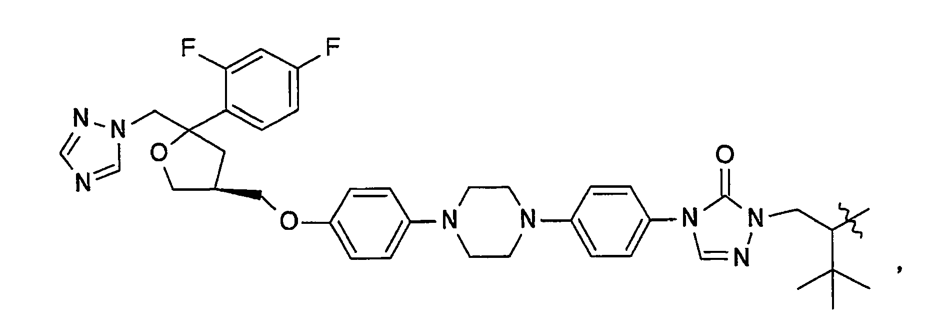 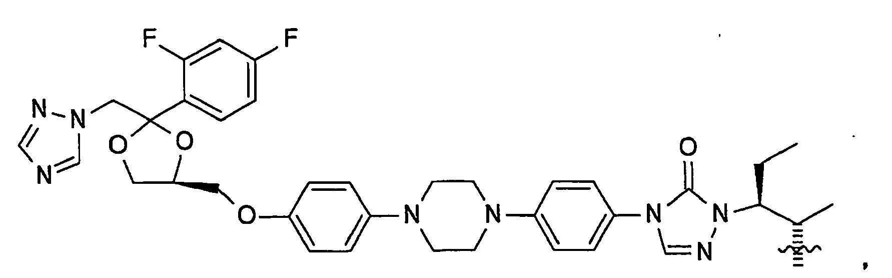 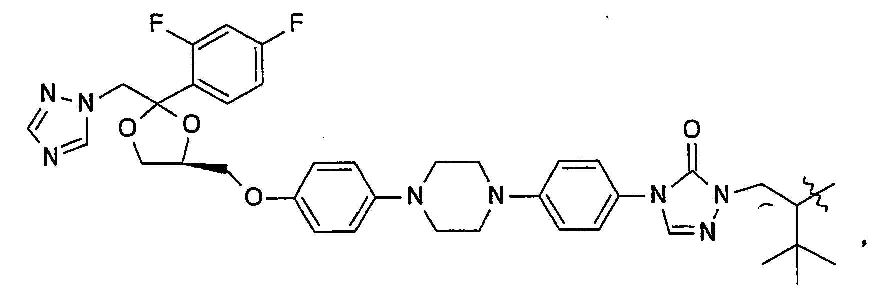 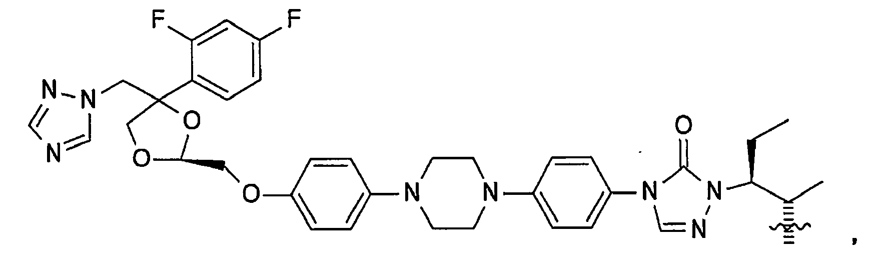 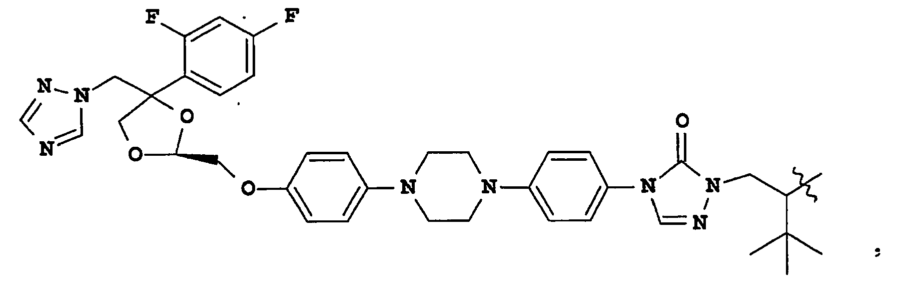 und einer Formel (i): 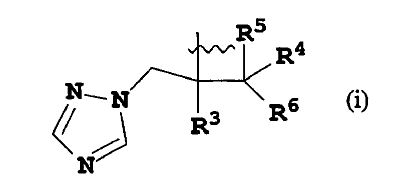 worin in Formel (i)
R³ eine Phenylgruppe, substituiert mit einem oder mehreren Halogenatomen, darstellt;
R⁴ Wasserstoff oder CH₃ darstellt;
R⁵ Wasserstoff oder zusammen mit R⁴ =CH₂ darstellt; und
R⁶ Thiazolyl, Pyrimidinyl oder Triazolyl darstellt, worin jeder Ring gegebenenfalls mit einer oder mehreren Gruppen, ausgewählt aus der Gruppe bestehend aus Halogen, =O, CH=CH-(C₆H₄₎-OCH₂CF₂CHF₂ und Phenyl, substituiert mit einer oder mehreren Gruppen, ausgewählt aus der Gruppe bestehend aus CN und OCH₂CF₂CHF₂, oder Phenyl, substituiert mit einer oder mehreren Gruppen, ausgewählt aus der Gruppe bestehend aus Halogen und Methylpyrazolyl, substituiert ist.

2. Monolysinsalz oder Solvat davon gemäss Anspruch 1, worin A die Formel (i) darstellt: 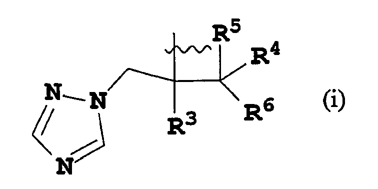 worin in Formel (i)
R³ eine Phenylgruppe, substituiert mit einem oder mehreren Halogenatomen, darstellt;
R⁴ Wasserstoff oder CH₃ darstellt;
R⁵ Wasserstoff oder zusammen mit R⁴ =CH₂ darstellt; und
R⁶ Thiazolyl, Pyrimidinyl oder Triazolyl darstellt, worin jeder Ring gegebenenfalls mit einer oder mehreren Gruppen, ausgewählt aus der Gruppe bestehend aus Halogen, =O, CH=CH-(C₆H₄₎-OCH₂CF₂CHF₂ und Phenyl, substituiert mit einer oder mehreren Gruppen, ausgewählt aus der Gruppe bestehend aus CN und OCH₂CF₂CHF₂, oder Phenyl, substituiert mit einer oder mehreren Gruppen, ausgewählt aus der Gruppe bestehend aus Halogen und Methylpyrazolyl, substituiert ist.

3. Monolysinsalz oder Solvat davon gemäss Anspruch 1 oder 2, worin R³ in Formel (i) 2,3-Difluorphenyl ist.

4. Monolysinsalz oder Solvat davon gemäss irgendeinem der Ansprüche 1 bis 3, worin R⁴ in Formel (i) Methyl ist und R⁵ Wasserstoff ist.

5. Monolysinsalz oder Solvat davon gemäss irgendeinem der Ansprüche 1 bis 4, worin R⁶ von Formel (i) 4-(4-Cyanophenyl)-thiazol-2-yl ist.

6. Monolysinsalz oder Solvat davon gemäss irgendeinem der Ansprüche 1 bis 5, worin jedes R und R¹ von Formel (I) Wasserstoff ist.

7. Monolysinsalz oder Solvat davon gemäss Anspruch 1 oder 2, worin A 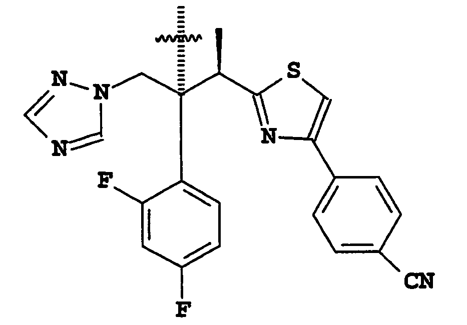 oder 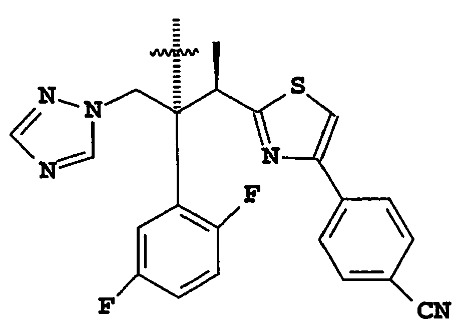 ist.

8. Monolysinsalz oder Solvat davon gemäss Anspruch 1, worin A ausgewählt ist aus der Gruppe bestehend aus: 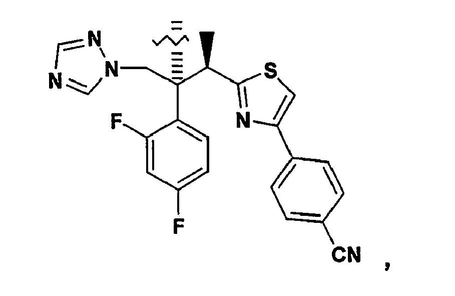 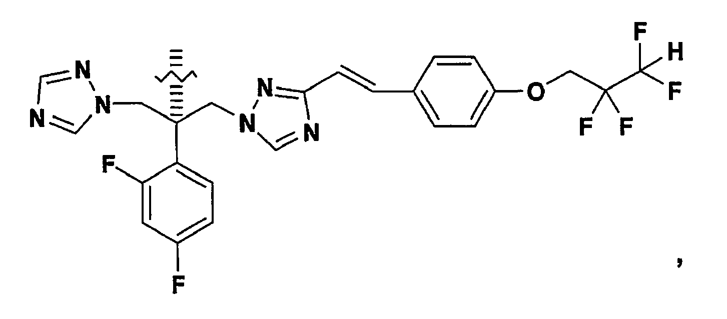 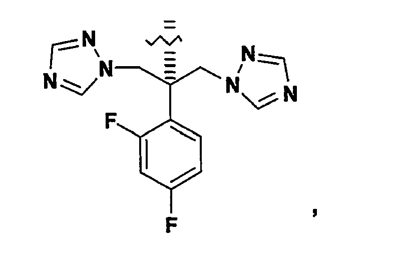 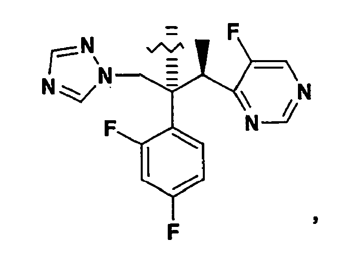 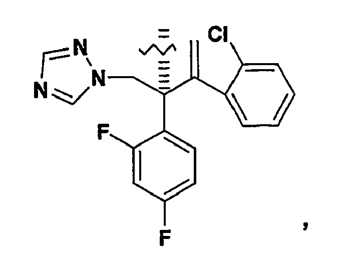 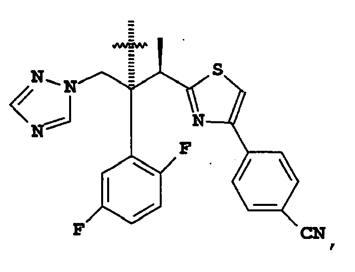 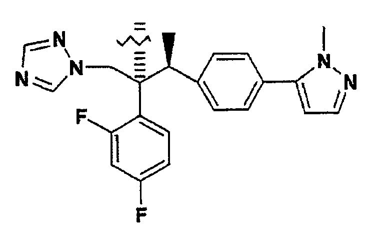 und

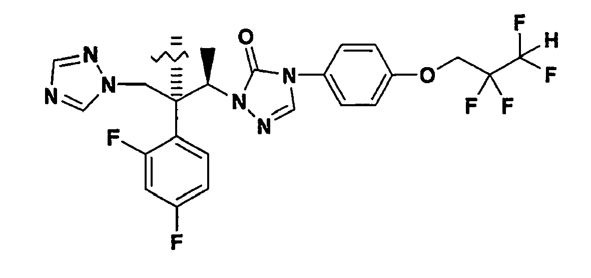

9. Monolysinsalz oder Solvat davon gemäss Anspruch 1, worin A ausgewählt ist aus der Gruppe bestehend aus: 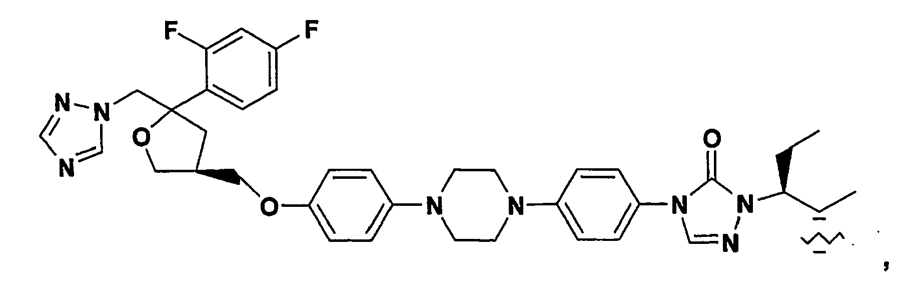 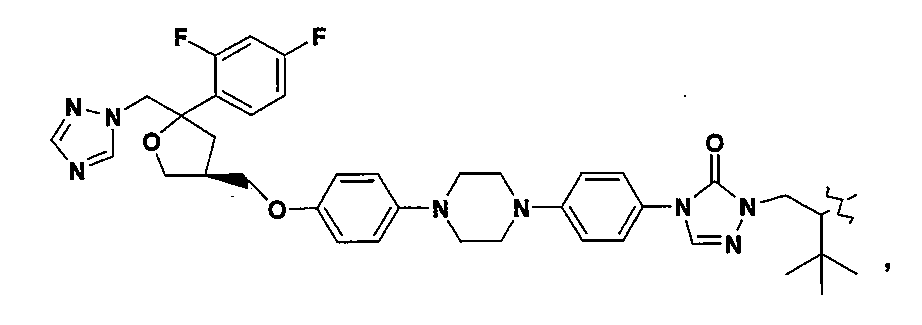 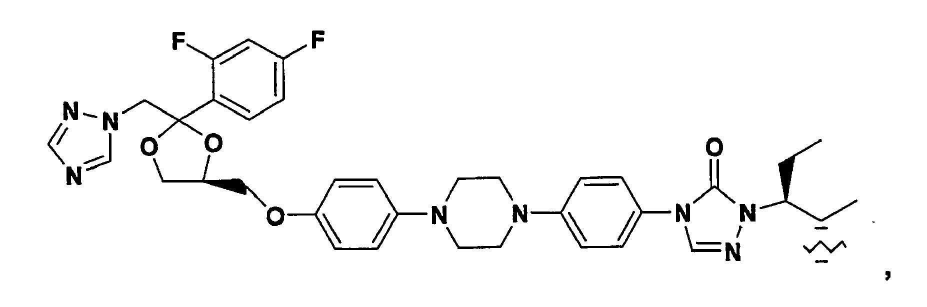 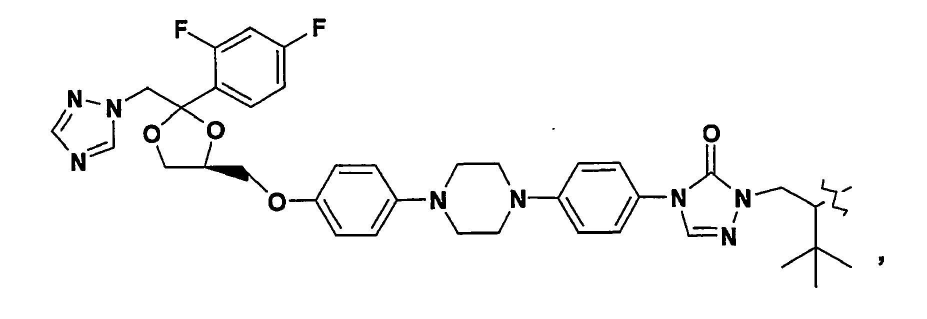 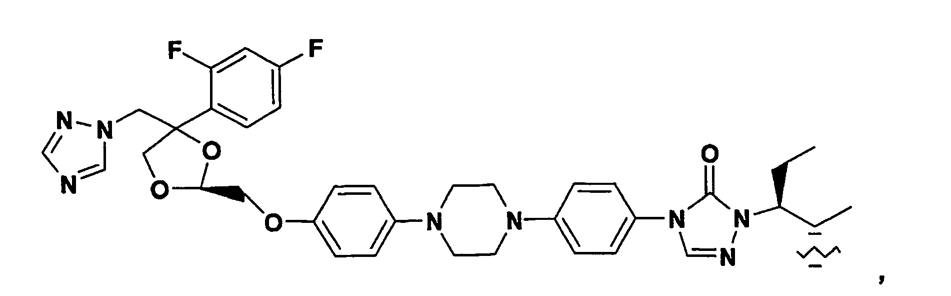 und

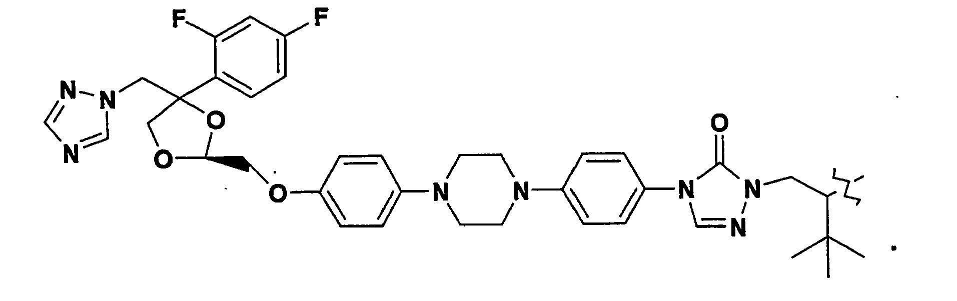

10. Solvat des Monolysinsalzes gemäss irgendeinem der Ansprüche 1 bis 9, worin das Solvat davon ein Ethanolsolvat ist.

11. Solvat des Monolysinsalzes gemäss irgendeinem der Ansprüche 1 bis 9, worin das Solvat davon ein Isopropylalkoholsolvat ist.

12. Solvat des Monolysinsalzes gemäss irgendeinem der Ansprüche 1 bis 9, worin das Solvat davon ein n-Propylalkoholsolvat ist.

13. Pharmazeutische Zusammensetzung, umfassend:
das Monolysinsalz gemäss irgendeinem der Ansprüche 1 bis 9 oder ein pharmazeutisch annehmbares Solvat davon; und
ein(en) pharmazeutisch annehmbare(s/n) Adjuvans, Verdünnungsmittel oder Träger.

14. Monolysinsalz gemäss irgendeinem der Ansprüche 1 bis 9 oder ein pharmazeutisch annehmbares Solvat davon zur Verwendung in einem Verfahren zur Behandlung von Pilzinfektionen.

15. Verfahren zur Herstellung eines wasserlöslichen Monolysinsalzes der Formel (I): 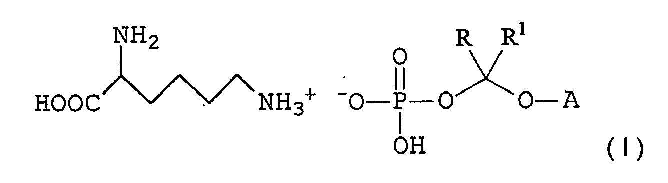 worin jedes von R und R¹ Wasserstoff oder (C₁₋₆)-Alkyl ist; und
A ausgewählt ist aus der Gruppe bestehend aus: 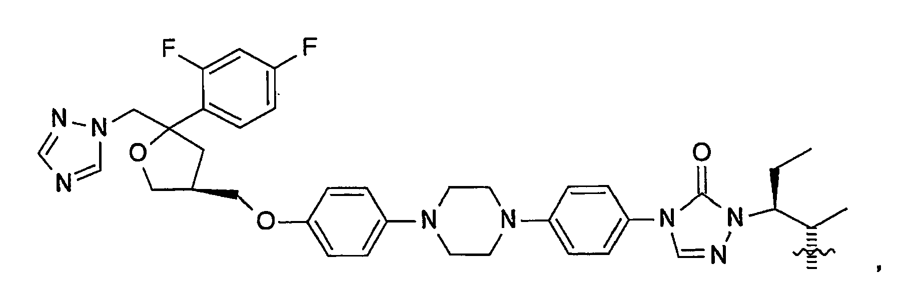 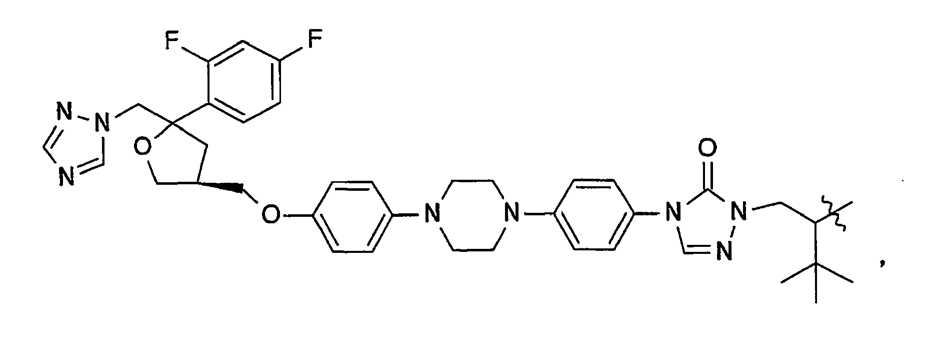 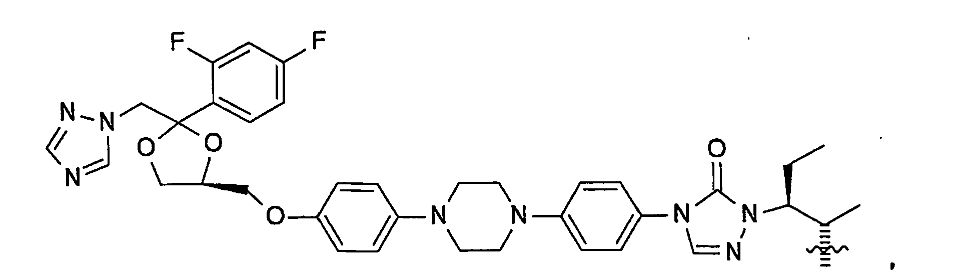 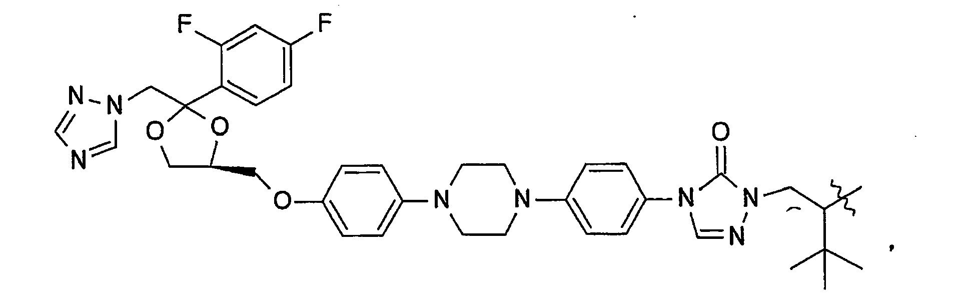 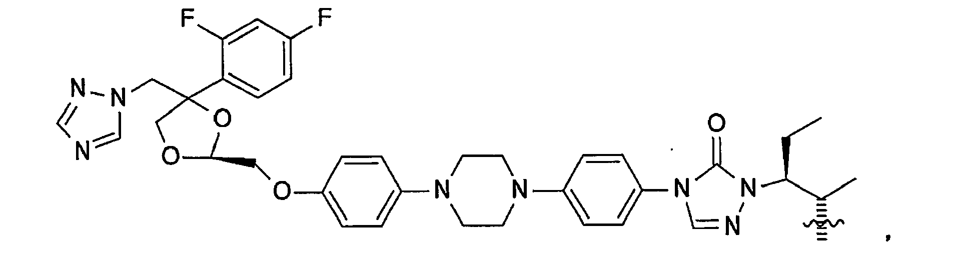 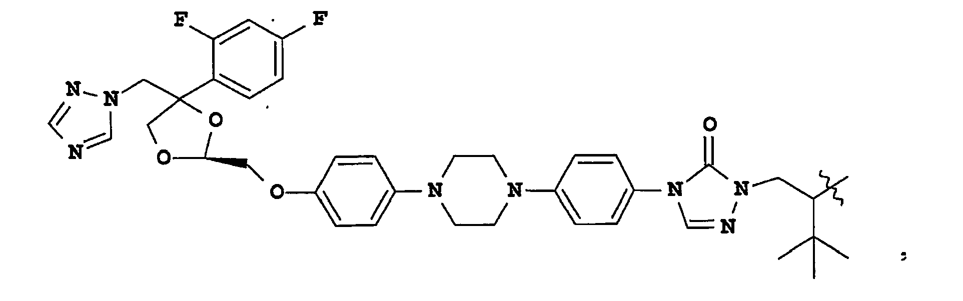 und einer Formel (i): 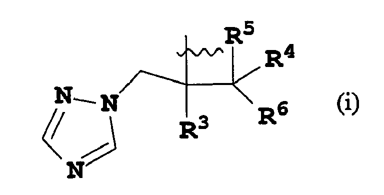 worin in Formel (i)
R³ eine Phenylgruppe, substituiert mit einem oder mehreren Halogenatomen, darstellt;
R⁴ Wasserstoff oder CH₃ darstellt;
R⁵ Wasserstoff oder zusammen mit R⁴ =CH₂ darstellt; und
R⁶ Thiazolyl, Pyrimidinyl oder Triazolyl darstellt, worin jeder Ring gegebenenfalls mit einer oder mehreren Gruppen, ausgewählt aus der Gruppe bestehend aus Halogen, =0, CH=CH-(C₆H₄)-OCH₂CF₂CHF₂ und Phenyl, substituiert mit einer oder mehreren Gruppen, ausgewählt aus der Gruppe bestehend aus CN und OCH₂CF₂CHF₂, oder Phenyl, substituiert mit einer oder mehreren Gruppen, ausgewählt aus der Gruppe bestehend aus Halogen und Methylpyrazolyl, substituiert ist;
wobei das Verfahren umfasst:
(a) Umsetzen einer Verbindung der Formel A-OH, worin A wie oben in Formel (I) definiert ist, mit einer Verbindung der Formel (III): 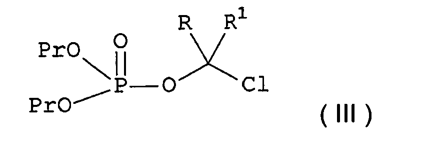 worin R und R¹ in Formel (III) jeweils unabhängig voneinander Wasserstoff oder (C₁₋₆)-Alkyl sind und Pr eine Hydroxyl-Schutzgruppe darstellt; wobei die Umsetzung in einem inerten organischen Lösungsmittel in Gegenwart einer Base bei einer Temperatur von etwa 25 bis 50°C durchgeführt wird, um ein erstes Intermediat der Formel (IV) zu bilden: 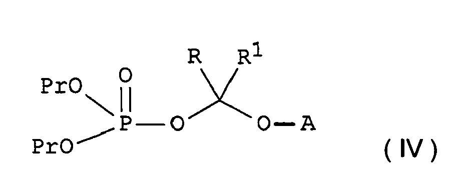 worin R und R¹ in Formel (IV) jeweils unabhängig voneinander Wasserstoff oder (C₁₋₆)-Alkyl sind, Pr eine Hydroxyl-Schutzgruppe darstellt und A wie für Formel (I) definiert ist;
(b) Entfernen der Schutzgruppen Pr in Formel (IV) mit einem organischen Lösungsmittel, um ein zweites Intermediat der Formel (V) zu bilden: 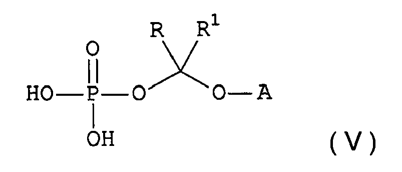 worin R und R¹ in Formel (V) jeweils unabhängig voneinander Wasserstoff oder (C₁₋₆)-Alkyl sind und A wie in Formel (I) definiert ist; und
(c) Umsetzen des zweiten Intermediats der Formel (V) mit Lysin in einem Lösungsmittel bei einem pH im Bereich von 4,2 bis 5,5, um das Monolysinsalz der Formel (I) herzustellen.

16. Verfahren gemäss Anspruch 15, worin die Schutzgruppe Pr tert-Butyl ist.

17. Verfahren gemäss Anspruch 15 oder 16, worin das Lösungsmittel von Schritt (a) Tetrahydrofuran ist.

18. Verfahren gemäss irgendeinem der Ansprüche 15 bis 17, worin die in Schritt (a) verwendete Base Natriumhydrid ist.

19. Verfahren zur Herstellung eines wasserlöslichen Solvats eines Monolysinsalzes, wobei das Monolysinsalz die Formel (I) hat: 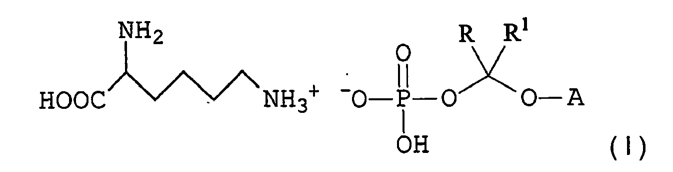 worin jedes von R und R¹ Wasserstoff oder (C₁₋₆)-Alkyl ist; und
A ausgewählt ist aus der Gruppe bestehend aus: 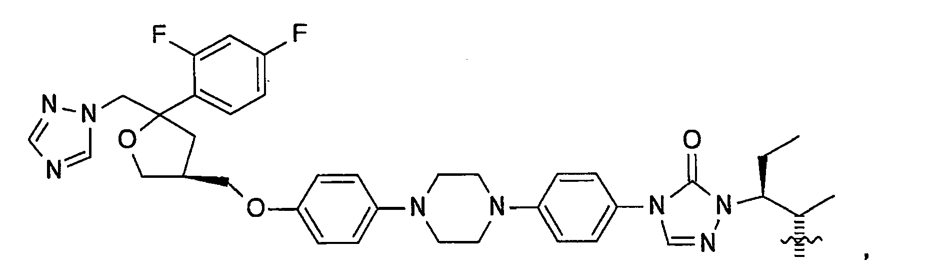 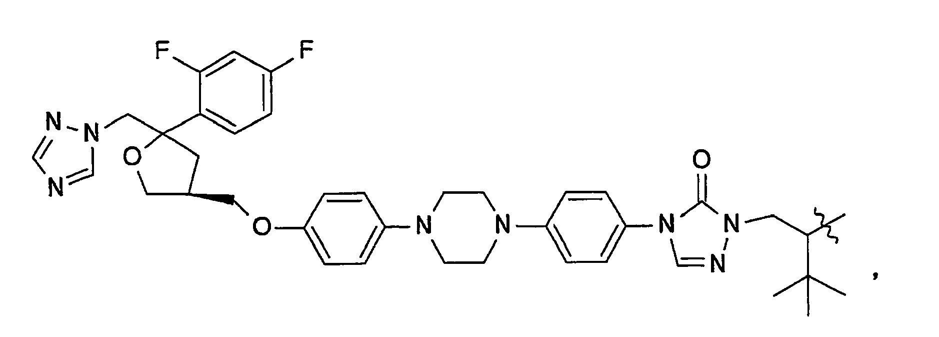 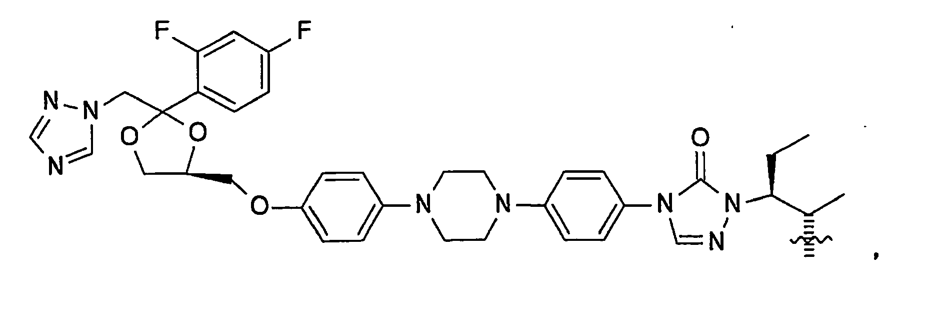 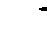 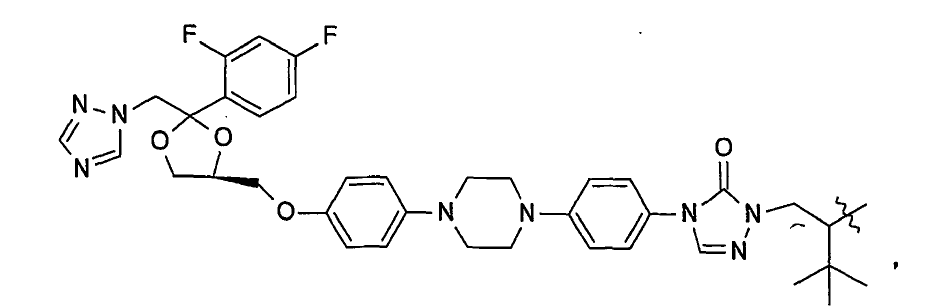 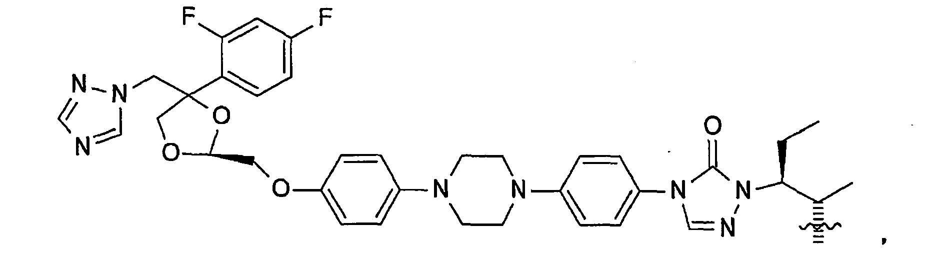 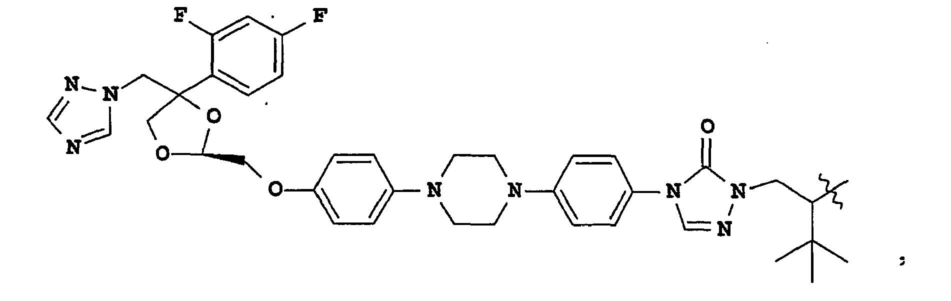 und einer Formel (i): 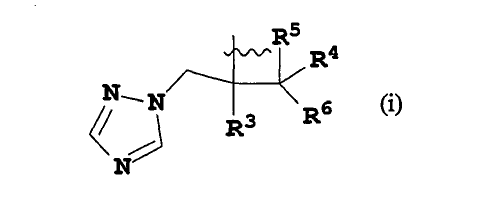 worin in Formel (i)
R³ eine Phenylgruppe, substituiert mit einem oder mehreren Halogenatomen, darstellt;
R⁴ Wasserstoff oder CH₃ darstellt;
R⁵ Wasserstoff oder zusammen mit R⁴ =CH₂ darstellt; und
R⁶ Thiazolyl, Pyrimidinyl oder Triazolyl darstellt, worin jeder Ring gegebenenfalls mit einer oder mehreren Gruppen, ausgewählt aus der Gruppe bestehend aus Halogen, =O, CH=CH-(C₆H₄)-OCH₂CF₂CHF₂ und Phenyl, substituiert mit einer oder mehreren Gruppen, ausgewählt aus der Gruppe bestehend aus CN und OCH₂CF₂CHF₂, oder Phenyl, substituiert mit einer oder mehreren Gruppen, ausgewählt aus der Gruppe bestehend aus Halogen und Methylpyrazolyl, substituiert ist;
wobei das Verfahren umfasst:
(a) Umsetzen einer Verbindung der Formel A-OH, worin A wie oben in Formel (I) definiert ist, mit einer Verbindung der Formel (III): 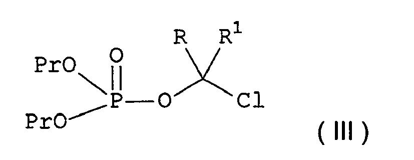 worin R und R¹ in Formel (III) jeweils unabhängig voneinander Wasserstoff oder (C₁₋₆)-Alkyl sind und Pr eine Hydroxyl-Schutzgruppe darstellt; wobei die Umsetzung in einem inerten organischen Lösungsmittel in Gegenwart einer Base bei einer Temperatur von etwa 25 bis 50°C durchgeführt wird, um ein erstes Intermediat der Formel (IV) zu bilden: 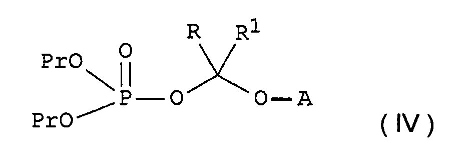 worin R und R¹ in Formel (IV) jeweils unabhängig voneinander Wasserstoff oder (C₁₋₆)-Alkyl sind, Pr eine Hydroxyl-Schutzgruppe darstellt und A wie für Formel (I) definiert ist;
(b) Entfernen der Schutzgruppen Pr in Formel (IV) mit einem organischen Lösungsmittel, um ein zweites Intermediat der Formel (V) zu bilden: 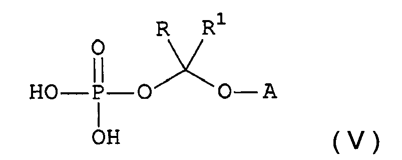 worin R und R¹ in Formel (V) jeweils unabhängig voneinander Wasserstoff oder (C₁₋₆)-Alkyl sind und A wie in Formel (I) definiert ist;
(c) Umsetzen des zweiten Intermediats der Formel (V) mit Lysin in einem Lösungsmittel bei einem pH im Bereich von 4,2 bis 5,5, um das Monolysinsalz der Formel (I) herzustellen; und
(d) Kristallisieren des Monolysinsalzes in einem Lösungsmittel, um das Solvat des Monolysinsalzes herzustellen.

20. Verfahren gemäss Anspruch 19, worin das Lösungsmittel in Schritt (d) wässriges Ethanol ist.

21. Verfahren gemäss Anspruch 19 oder 20, worin das Lösungsmittel in Schritt (d) wässriger Isopropylalkohol ist.

22. Verfahren gemäss irgendeinem der Ansprüche 19 bis 21, worin das Lösungsmittel in Schritt (d) wässriger n-Propylalkohol ist.

23. Verfahren gemäss irgendeinem der Ansprüche 15 bis 22, worin A des Ausgangsmaterials A-OH die Formel (i) ist:

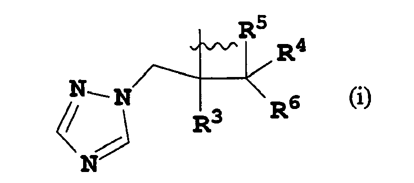

24. Monolysinsalz von ((2R,3R)-3-(4-(4-Cyanophenyl)thiazol-2-yl)-2-(2,4-difluorphenyl)-1-(1H-1,2,4-triazol-1-yl)butan-2-yloxy)methyl-dihydrogenphosphat mit der Struktur: 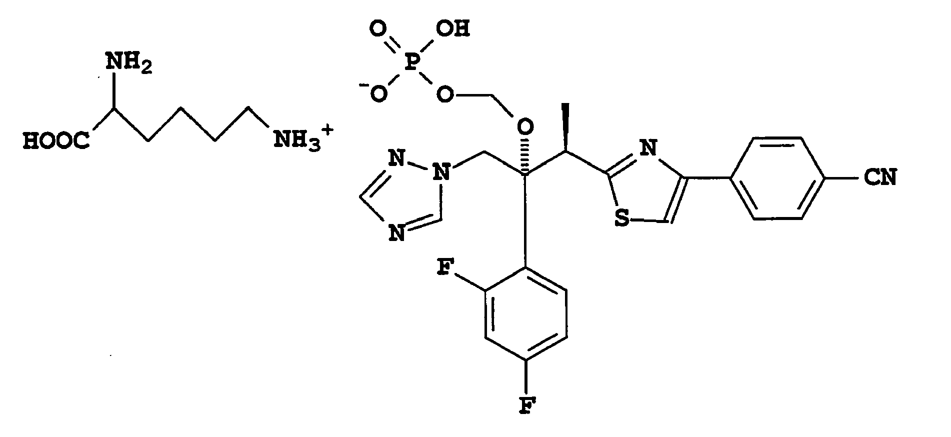 oder ein pharmazeutisch annehmbares Solvat davon.

25. Monolysinsalz oder Solvat davon gemäss Anspruch 24, wobei das Salz oder Solvat davon in kristalliner Form ist.

26. Solvat des Monolysinsalzes gemäss Anspruch 24, worin das Solvat davon ein Ethanolsolvat ist.

27. Solvat des Monolysinsalzes gemäss Anspruch 24, worin das Solvat davon ein Isopropylalkoholsolvat ist.

28. Solvat des Monolysinsalzes gemäss Anspruch 24, worin das Solvat davon ein n-Propylalkoholsolvat ist.

29. Pharmazeutische Zusammensetzung, umfassend:
eine wirksame Menge des Monolysinsalzes gemäss Anspruch 24 oder des pharmazeutisch annehmbaren Solvats davon; und
ein(en) pharmazeutisch annehmbare(s/n) Adjuvans, Verdünnungsmittel oder Träger.

30. Pharmazeutische Zusammensetzung gemäss Anspruch 29, wobei die Zusammensetzung eine Tablette, eine Kapsel, ein Pulver, eine Lösung, eine Suspension, eine Emulsion, eine Salbe, eine Lotion, eine Creme oder ein Spray ist.

31. Verwendung des Monolysinsalzes gemäss Anspruch 24 oder des pharmazeutisch annehmbaren Solvats davon zur Herstellung eines Medikaments zur Behandlung einer Pilzinfektion.

32. Verwendung gemäss Anspruch 31, wobei nur das Medikament verabreicht wird.

33. Verwendung gemäss Anspruch 31, wobei das Medikament parenteral verabreicht wird.

34. Verfahren zur Herstellung eines wasserlöslichen Monolysinsalzes der folgenden Formel: 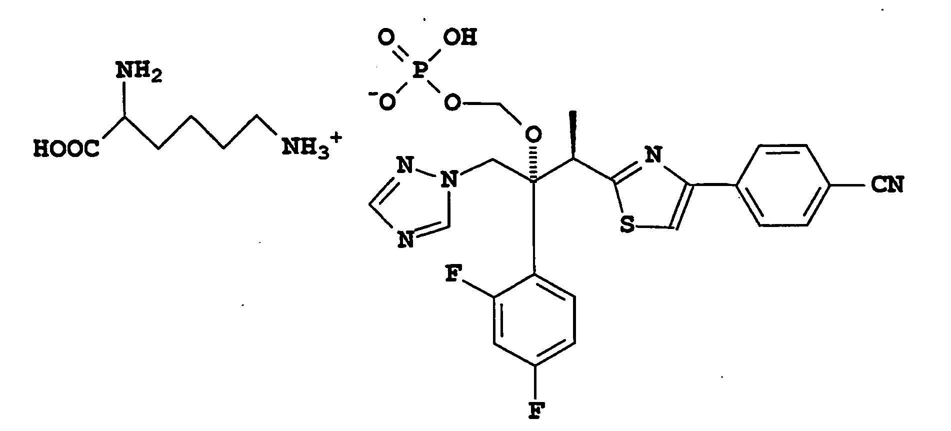 wobei das Verfahren umfasst:
(a) Umsetzen einer Verbindung der Formel (B): 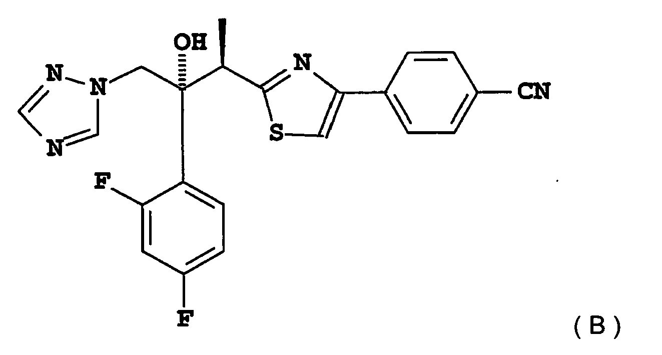 mit einer Verbindung der Formel (III'): 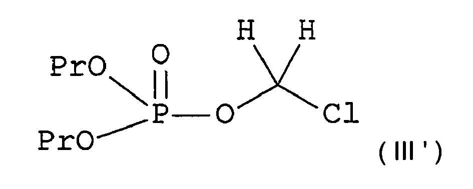 worin Pr in Formel (III') eine Hydroxyl-Schutzgruppe darstellt;
die Umsetzung wird in einem inerten organischen Lösungsmittel in Gegenwart einer Base bei einer Temperatur von etwa 25 bis 50°C durchgeführt, um ein erstes Intermediat der Formel (IV') zu bilden: 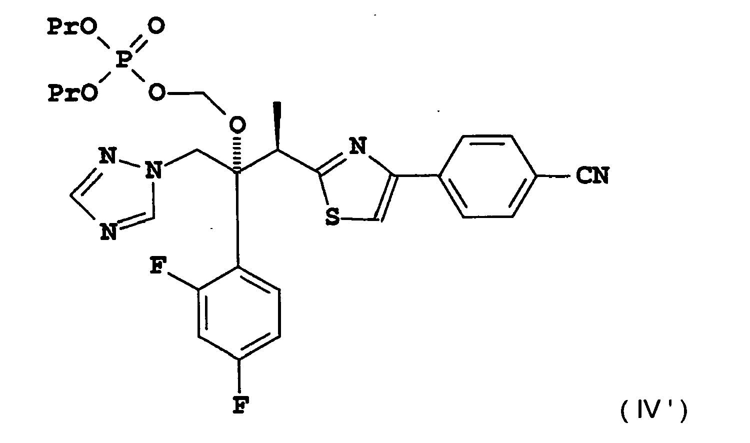 worin Pr in Formel (IV') eine Hydroxyl-Schutzgruppe darstellt;
(b) Entfernen der Schutzgruppe Pr von Formel (IV') mit einem organischen Lösungsmittel, um ein zweites Intermediat der Formel (V') zu bilden: 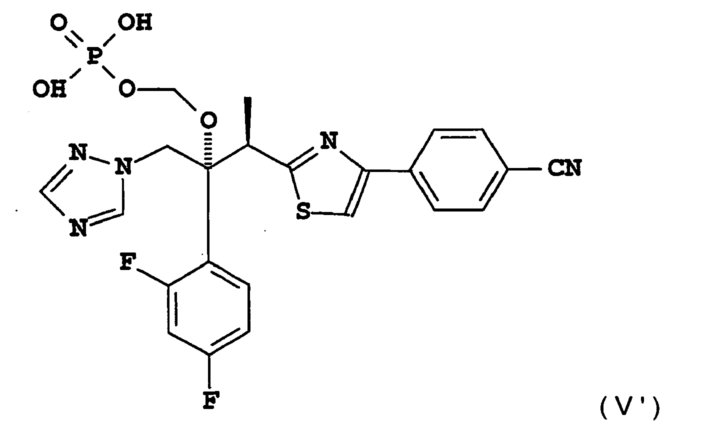 und
(c) Umsetzen des zweiten Intermediats der Formel (V') mit Lysin in einem Lösungsmittel bei einem pH im Bereich von 4,2 bis 5,5, um das Monolysinsalz herzustellen.

35. Verfahren gemäss Anspruch 34, worin die Schutzgruppe Pr tert-Butyl ist.

36. Verfahren gemäss Anspruch 34 oder 35, worin das Lösungsmittel in Schritt (a) Tetrahydrofuran ist.

37. Verfahren gemäss irgendeinem der Ansprüche 34 bis 36, worin die in Schritt (a) verwendete Base Natriumhydrid ist.

38. Verfahren zur Herstellung eines wasserlöslichen Solvats eines Monolysinsalzes, wobei das Monolysinsalz die Formel hat: 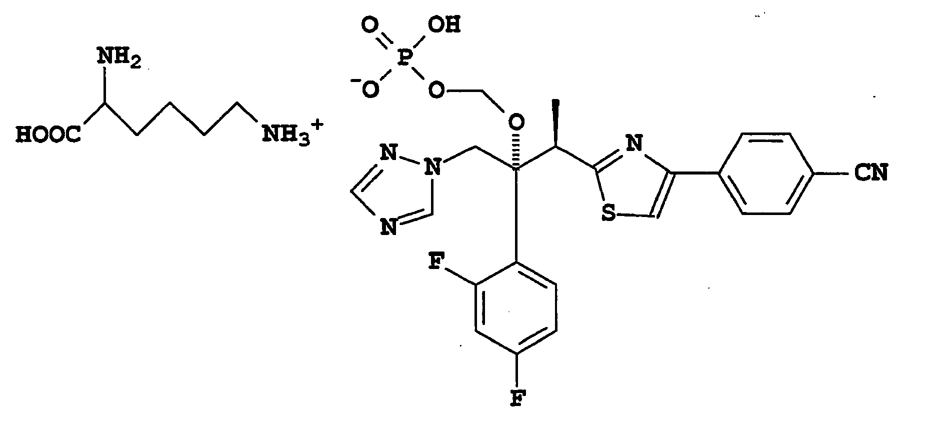 wobei das Verfahren umfasst:
(a) Umsetzen einer Verbindung der Formel (B): 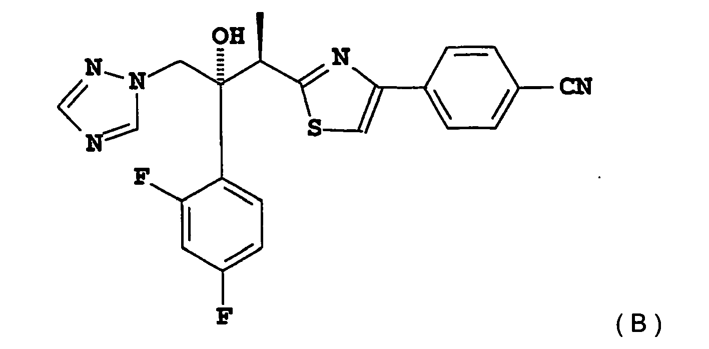 mit einer Verbindung der Formel (III'): 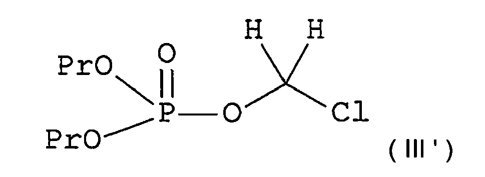 worin Pr in Formel (III') eine Hydroxyl-Schutzgruppe darstellt; die Umsetzung wird in einem inerten organischen Lösungsmittel in Gegenwart einer Base bei einer Temperatur von etwa 25 bis 50°C durchgeführt, um ein erstes Intermediat der Formel (IV') zu bilden: 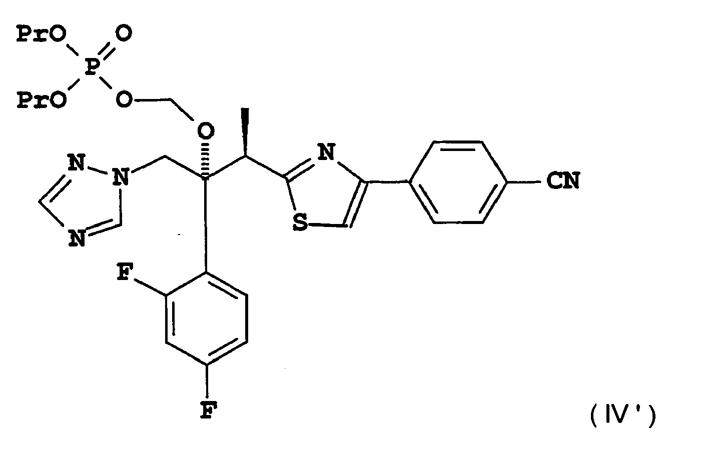 worin Pr in Formel (IV') eine Hydroxyl-Schutzgruppe darstellt;
(b) Entfernen der Schutzgruppe Pr von Formel (IV') mit einem organischen Lösungsmittel, um ein zweites Intermediat der Formel (V') zu bilden: 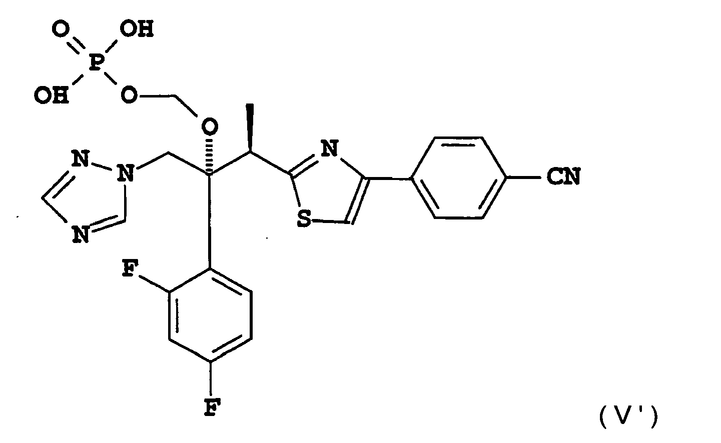
(c) Umsetzen des zweiten Intermediats der Formel (V') mit Lysin in einem Lösungsmittel bei einem pH im Bereich von 4,2 bis 5,5, um das Monolysinsalz herzustellen; und
(d) Kristallisieren des Monolysinsalzes in einem Lösungsmittel, um das Solvat des Monolysinsalzes herzustellen.

39. Verfahren gemäss Anspruch 38, worin das Lösungsmittel in Schritt (d) wässriges Ethanol ist.

40. Verfahren gemäss Anspruch 38 oder 39, worin das Lösungsmittel in Schritt (d) wässriger Isopropylalkohol ist.

41. Verfahren gemäss irgendeinem der Ansprüche 38 bis 40, worin das Lösungsmittel in Schritt (d) wässriger n-Propylalkohol ist.

42. Verwendung des Monolysinsalzes gemäss irgendeinem der Ansprüche 1 bis 9 oder eines pharmazeutisch annehmbaren Solvats davon zur Herstellung eines Mittels zur Behandlung von Pilzinfektionen.

43. Monolysinsalz gemäss Anspruch 24 oder ein pharmazeutisch annehmbares Solvat davon zur Verwendung in einem Verfahren zur Behandlung von Pilzinfektionen.

## Revendications

1. Sel de mono-lysine d'un composé de formule I, ou solvate de celui-ci : 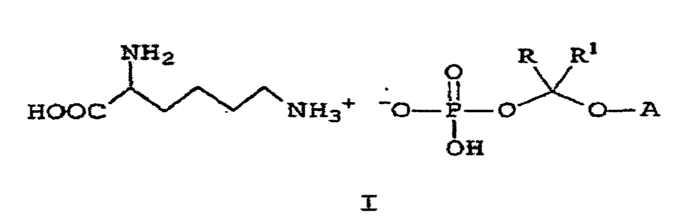 dans lequel chacun de R et R¹ est un hydrogène ou un alkyle en C₁-C₆ ; et
A est choisi parmi le groupe consistant en : 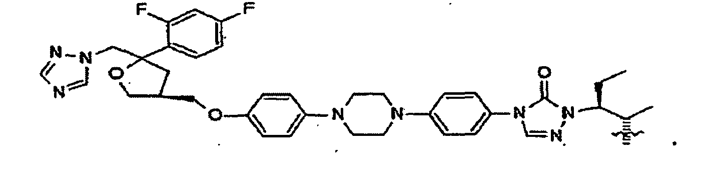 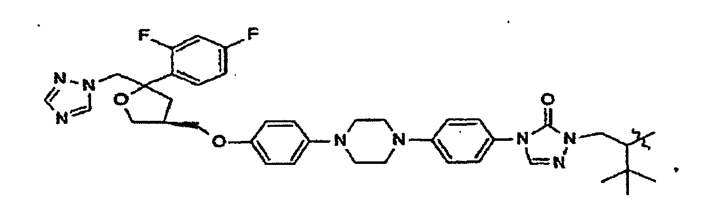 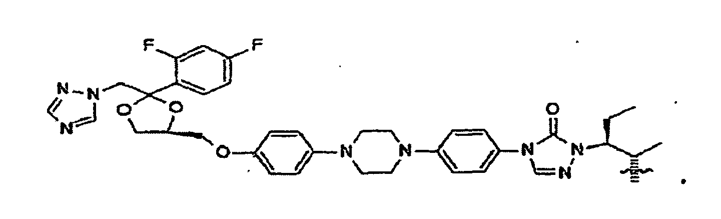 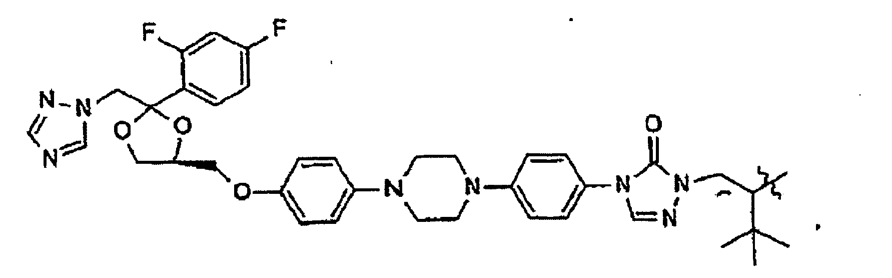 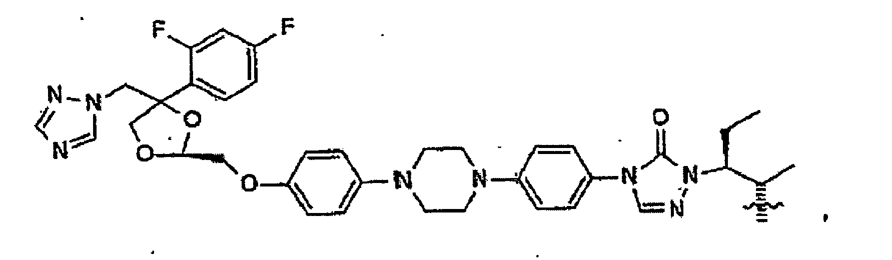 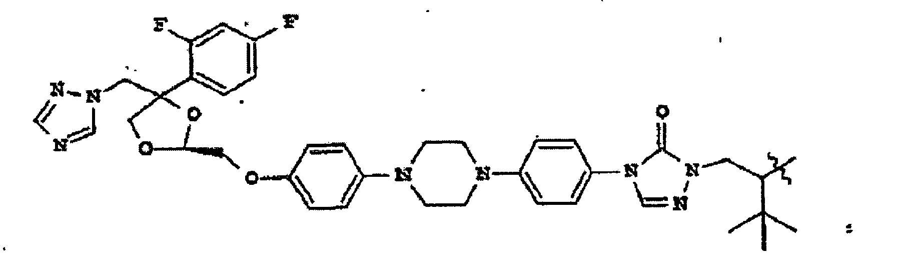 et une formule (i) : 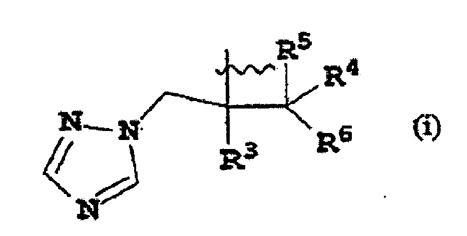 dans lequel, dans la formule (i)
R³ représente un groupe phényle substitué par un ou plusieurs atomes d'halogène ;
R⁴ représente un hydrogène ou CH₃;
R⁵ représente un hydrogène, ou pris conjointement avec R⁴ représente =CH₂ ; et
R⁶ représente un thiazolyle, un pyrimidinyle ou un triazolyle dans lequel chaque cycle est facultativement substitué par un ou plusieurs groupes choisis parmi le groupe consistant en un halogène, =O, CH=CH-(C₆H₄)-OCH₂CF₂CHF₂, et un phényle substitué par un ou plusieurs groupes choisis parmi le groupe consistant en CN et OCH₂CF₂CHF₂, ou un phényle substitué par un ou plusieurs groupes choisis parmi le groupe consistant en un halogène et un méthylpyrazolyle.

2. Sel de mono-lysine ou solvate de celui-ci selon la revendication 1, dans lequel A représente la formule (i) : 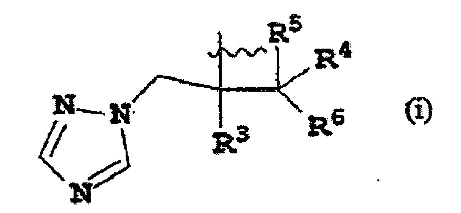 dans laquelle
R³ représente un groupe phényle substitué par un ou plusieurs atomes d'halogène ;
R⁴ représente un hydrogène ou CH₃;
R⁵ représente un hydrogène, ou pris conjointement avec R⁴ représente =CH₂ ; et
R⁶ représente un thiazolyle, un pyrimidinyle ou un triazolyle dans lequel chaque cycle est facultativement substitué par un ou plusieurs groupes choisis parmi le groupe consistant en un halogène, =O, CH=CH- (C₆H₄)-OCH₂CF₂CHF₂, et un phényle substitué par un ou plusieurs groupes choisis parmi le groupe consistant en CN et OCH₂CF₂CHF₂, ou un phényle substitué par un ou plusieurs groupes choisis parmi le groupe consistant en un halogène et un méthylpyrazolyle.

3. Sel de mono-lysine ou solvate de celui-ci selon la revendication 1 ou 2, dans lequel R³ de la formule (i) est un 2,4-difluorophényle.

4. Sel de mono-lysine ou solvate de celui-ci selon l'une quelconque des revendications 1 à 3, dans lequel R⁴ de la formule (i) est un méthyle et R⁵ est un hydrogène.

5. Sel de mono-lysine ou solvate de celui-ci selon l'une quelconque des revendications 1 à 4, dans lequel R⁶ de la formule (i) est un 4-(4-cyanophényl)-thiazol-2-yle.

6. Sel de mono-lysine ou solvate de celui-ci selon l'une quelconque des revendications 1 à 5, dans lequel chacun de R et R¹ de la formule I est un hydrogène.

7. Sel de mono-lysine ou solvate de celui-ci selon la revendication 1 ou 2, dans lequel A est 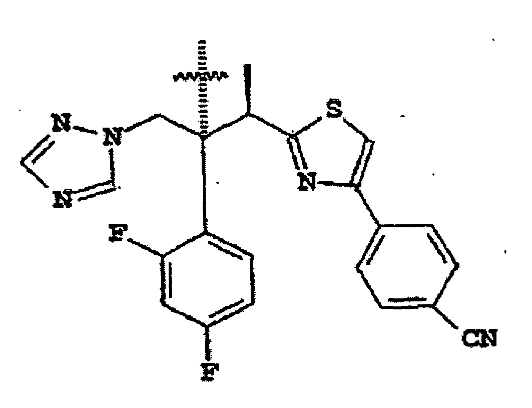 ou

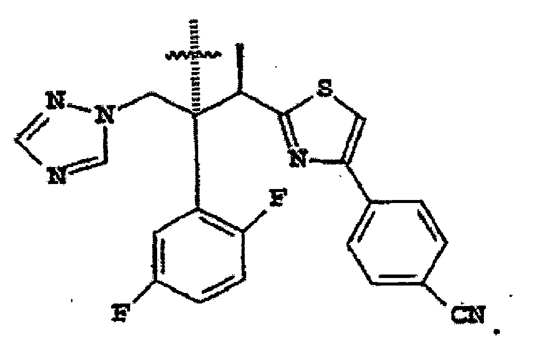

8. Sel de mono-lysine ou solvate de celui-ci selon la revendication 1, dans lequel A est choisi parmi le groupe consistant en 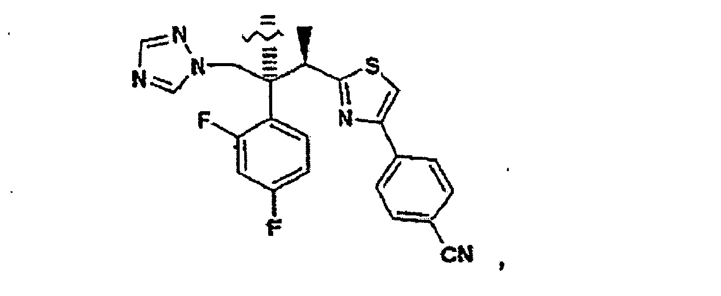 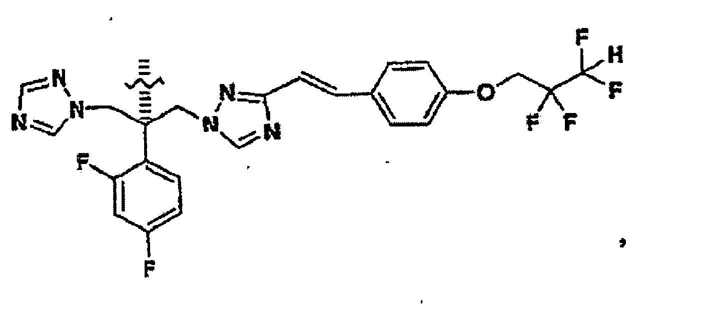 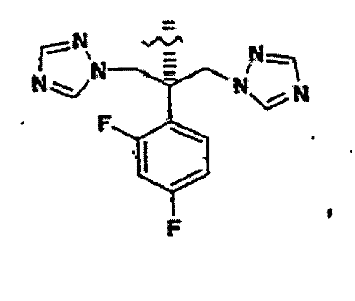 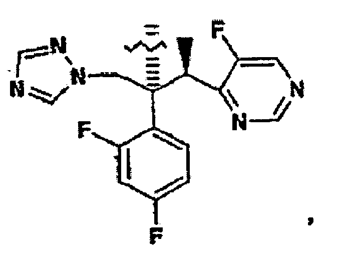 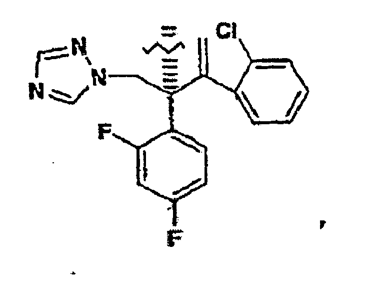 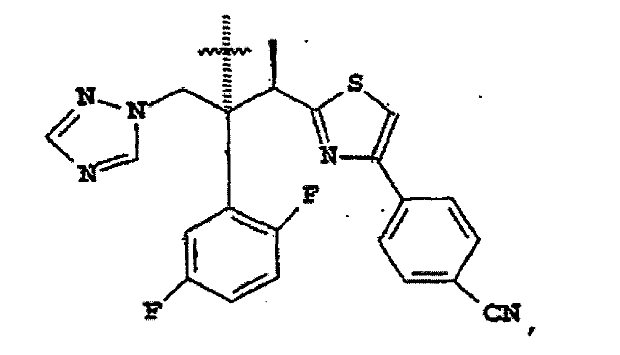 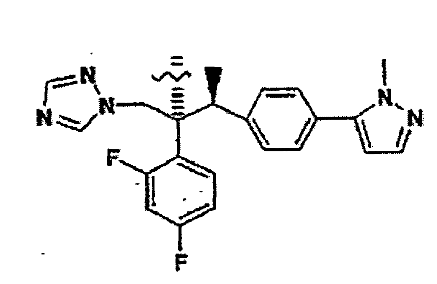 et

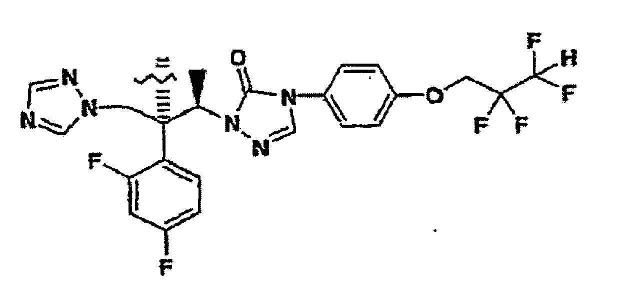

9. Sel de mono-lysine ou solvate de celui-ci selon la revendication 1, dans lequel ledit A est choisi parmi le groupe consistant en : 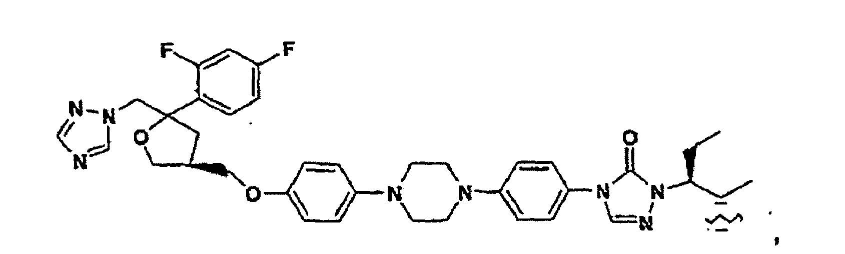 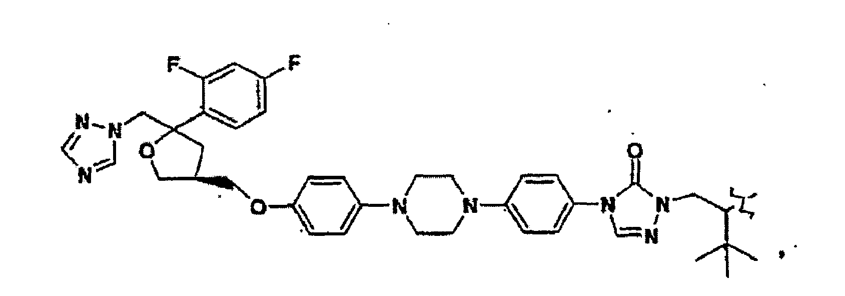 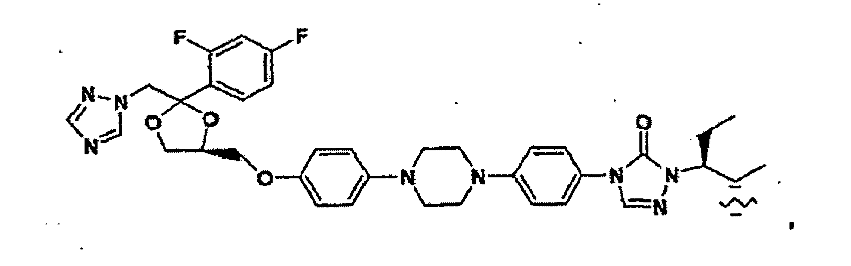 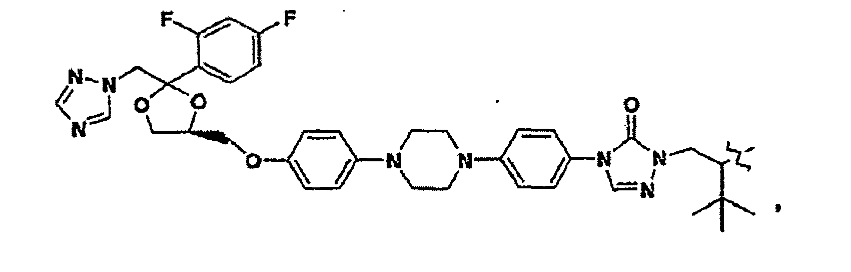 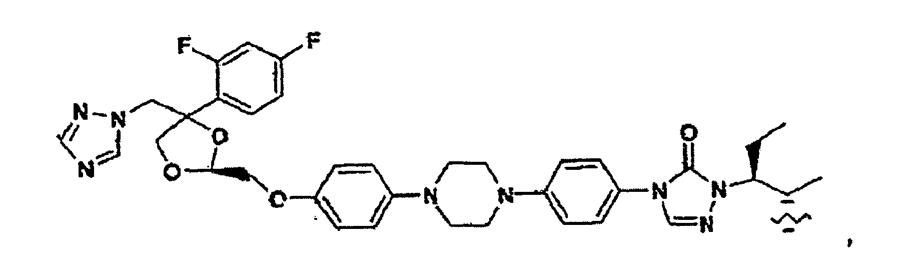 et

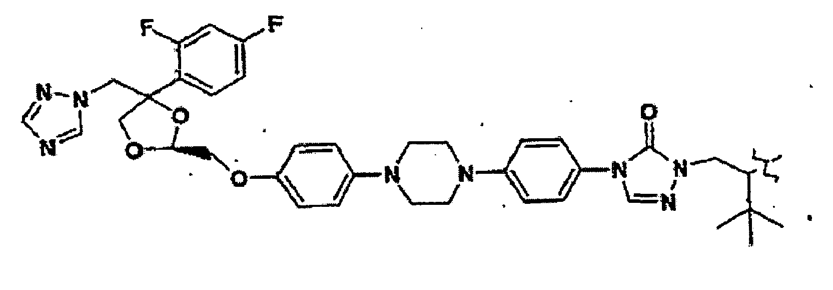

10. Solvate du sel de mono-lysine selon l'une quelconque des revendications 1 à 9, dans lequel ledit solvate de celui-ci est un solvate d'éthanol.

11. Solvate du sel de mono-lysine selon l'une quelconque des revendications 1 à 9, dans lequel ledit solvate de celui-ci est un solvate d'alcool isopropylique.

12. Solvate du sel de mono-lysine selon l'une quelconque des revendications 1 à 9, dans lequel ledit solvate de celui-ci est un solvate d'alcool n-propylique.

13. Composition pharmaceutique comprenant :
le sel de mono-lysine selon l'une quelconque des revendications 1 à 9, ou un solvate pharmaceutiquement acceptable de celui-ci ; et
un adjuvant, diluant, ou support pharmaceutiquement acceptable.

14. Sel de mono-lysine selon l'une quelconque des revendications 1 à 9, ou solvate pharmaceutiquement acceptable de celui-ci pour une utilisation dans un procédé pour le traitement d'infections fongiques.

15. Procédé pour la préparation d'un sel de mono-lysine soluble dans l'eau de la formule I : 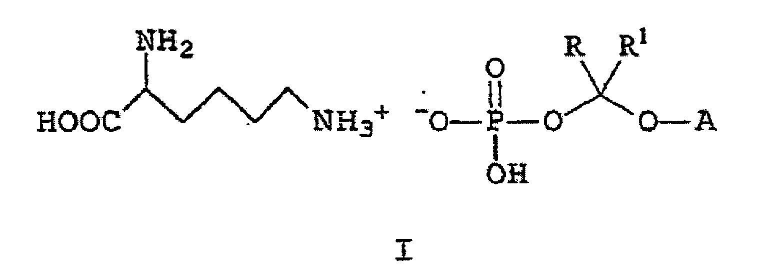 dans lequel chacun de R et R¹ est un hydrogène ou un alkyle en C₁-C₆ ; et
A est choisi parmi le groupe consistant en : 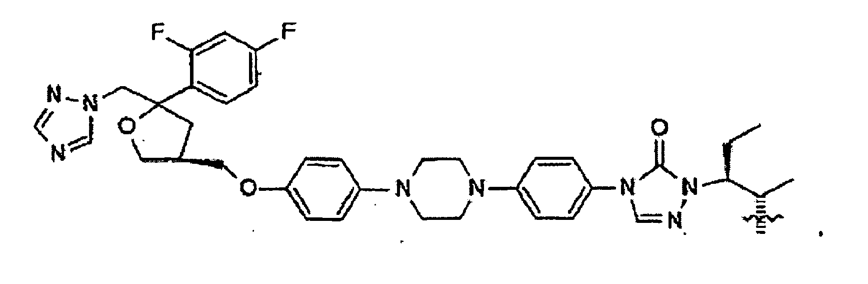 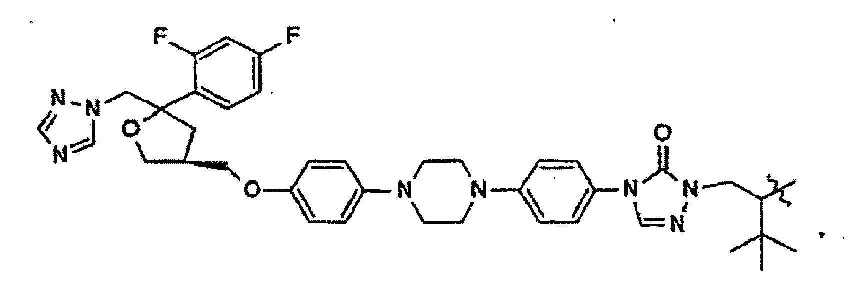 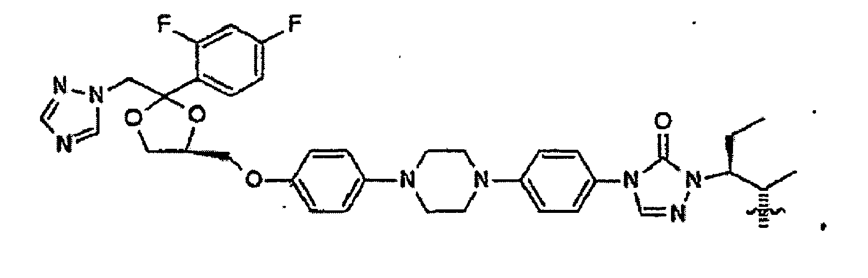 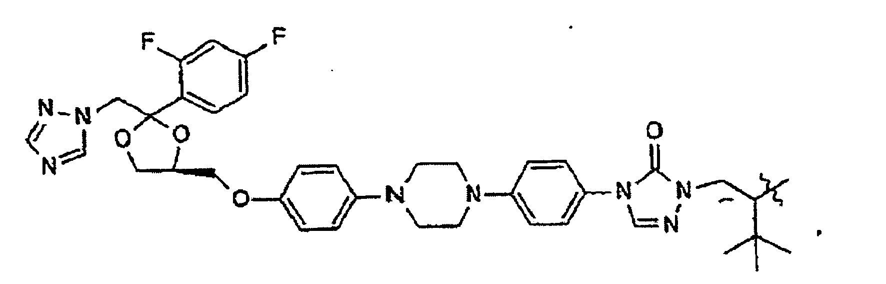 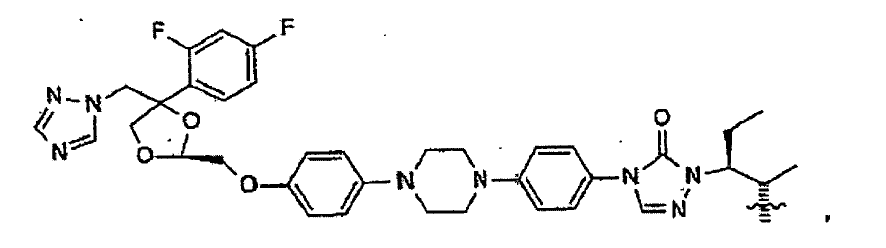 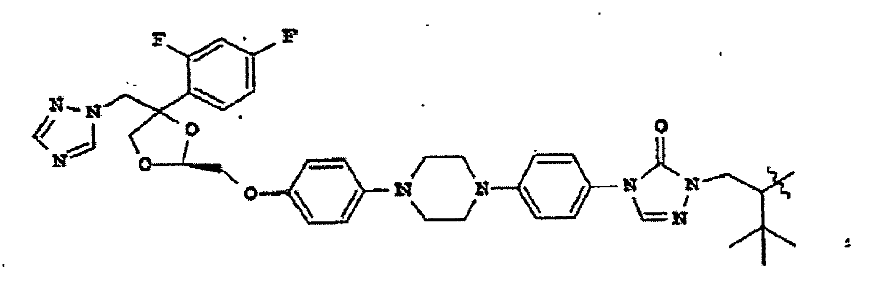 et une formule (i) : 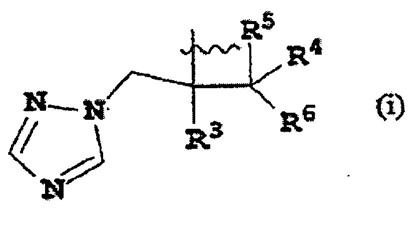 dans lequel, dans la formule (i)
R³ représente un groupe phényle substitué par un ou plusieurs atomes d'halogène ;
R⁹ représente un hydrogène ou CH₃ ;
R⁵ représente un hydrogène, ou pris conjointement avec R⁴ représente =CH₂ ; et
R⁶ représente un thiazolyle, un pyrimidinyle ou un triazolyle dans lequel chaque cycle est facultativement substitué par un ou plusieurs groupes choisis parmi le groupe consistant en un halogène, =O, CH=CH- (C₆H₄) -OCH₂CF₂CHF₂, et un phényle substitué par un ou plusieurs groupes choisis parmi le groupe consistant en CN et OCH₂CF₂CHF₂, ou un phényle substitué par un ou plusieurs groupes choisis parmi le groupe consistant en un halogène et un méthylpyrazolyle ;
ledit procédé comprenant :
(a) la réaction d'un composé de formule A-OH dans lequel A est tel que défini ci-dessus dans la formule I avec un composé de formule III : 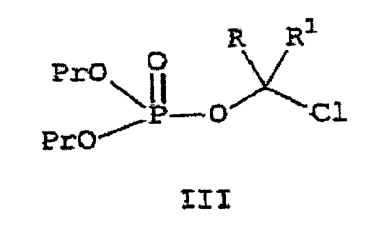 dans lequel R et R¹ dans la formule III sont chacun indépendamment un hydrogène ou un alkyle en C₁-C₆, et Pr représente un groupe de protection de l'hydroxyle ; ladite réaction est dans un solvant organique inerte en présence d'une base à une température d'environ 25°C à 50°C pour former un premier intermédiaire de formule IV : 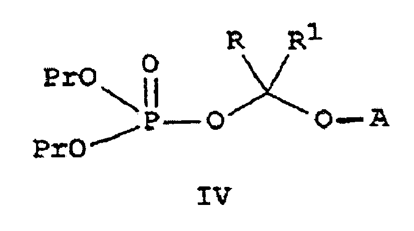 dans lequel R et R¹ dans la formule IV sont chacun indépendamment un hydrogène ou un alkyle en C₁-C₆, Pr représente un groupe de protection de l'hydroxyle, et A est tel que défini dans la formule I ;
(b) la suppression des groupes de protection Pr de la formule IV avec un solvant organique pour former un deuxième intermédiaire de formule V : 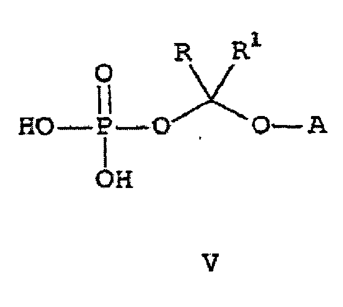 dans lequel R et R¹ dans la formule V sont chacun indépendamment un hydrogène ou un alkyle en C₁-C₆, et A est tel que défini dans la formule I ; et
(c) la réaction dudit deuxième intermédiaire de formule V avec de la lysine dans un solvant à un pH dans la plage de 4,2 à 5,5 pour produire ledit sel de mono-lysine de formule I.

16. Procédé selon la revendication 15, dans lequel le groupe de protection Pr est un butyle tertiaire.

17. Procédé selon la revendication 15 ou 16, dans lequel le solvant à l'étape (a) est le tétrahydrofurane.

18. Procédé selon l'une quelconque des revendications 15 à 17, dans lequel la base utilisée à l'étape (a) est l'hydrure de sodium.

19. Procédé pour la préparation d'un solvate soluble dans l'eau d'un sel de mono-lysine, ledit sel de mono-lysine ayant la formule I : 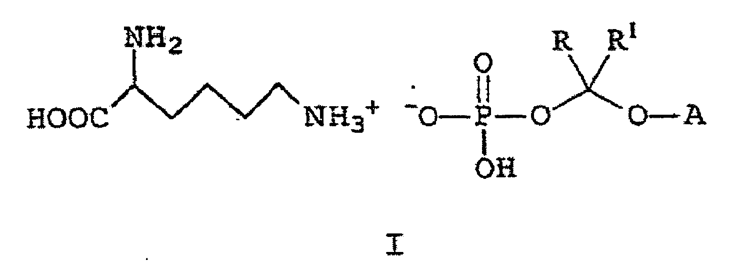 dans lequel chacun de R et R¹ est un hydrogène ou un alkyle en C₁-C₆ ; et
A est choisi parmi le groupe consistant en : 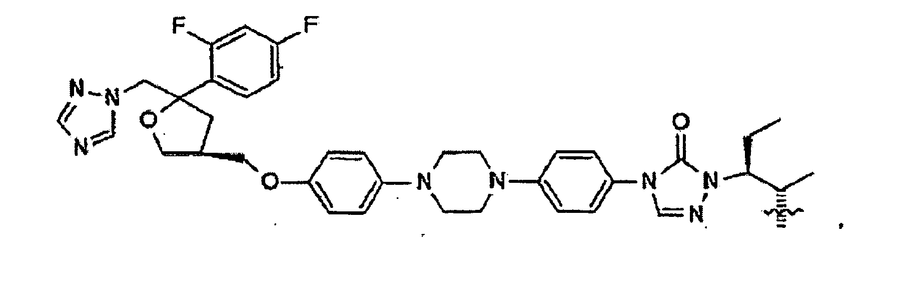 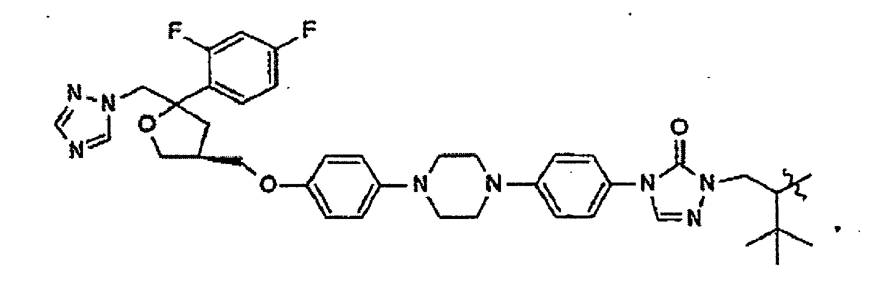 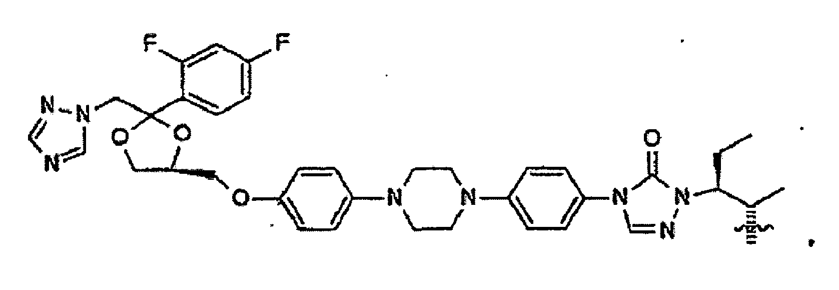 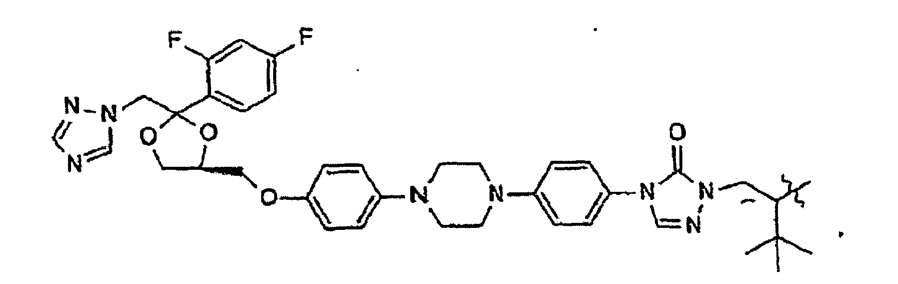 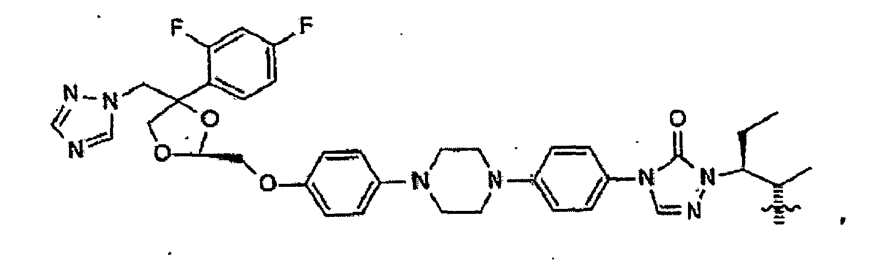 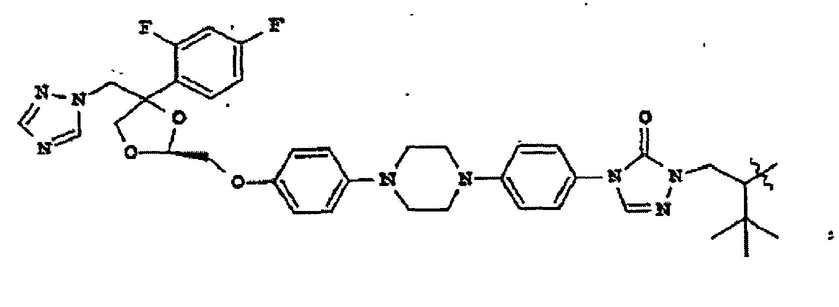 et une formule (i) : 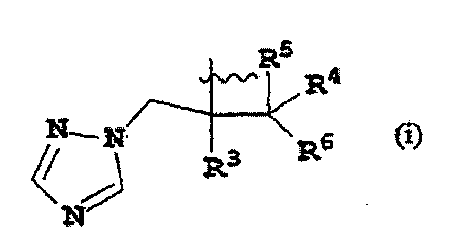 dans lequel, dans la formule (i)
R³ représente un groupe phényle substitué par un ou plusieurs atomes d'halogène ;
R⁴ représente un hydrogène ou CH₃ ;
R⁵ représente un hydrogène, ou pris conjointement avec R⁴ représente =CH₂ ; et
R⁶ représente un thiazolyle, un pyrimidinyle ou un triazolyle dans lequel chaque cycle est facultativement substitué par un ou plusieurs groupes choisis parmi le groupe consistant en un halogène, =O, CH=CH-(C₆H₄)-OCH₂CF₂CHF₂, et un phényle substitué par un ou plusieurs groupes choisis parmi le groupe consistant en CN et OCH₂CF₂CHF₂, ou un phényle substitué par un ou plusieurs groupes choisis parmi le groupe consistant en un halogène et un méthylpyrazolyle ;
ledit procédé comprenant :
(a) la réaction d'un composé de formule A-OH dans lequel A est tel que défini ci-dessus dans la formule I avec un composé de formule III : 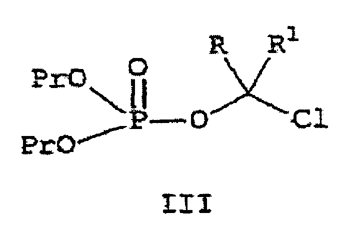 dans lequel R et R¹ dans la formule III sont chacun indépendamment un hydrogène ou un alkyle en C₁-C₆, et Pr représente un groupe de protection de l'hydroxyle ; ladite réaction est dans un solvant organique inerte en présence d'une base à une température d'environ 25°C à 50°C pour former un premier intermédiaire de formule IV : 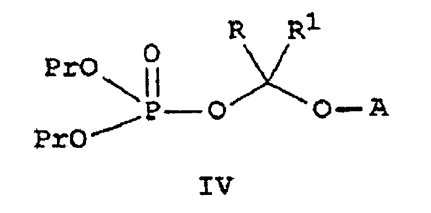 dans lequel R et R¹ dans la formule IV sont chacun indépendamment un hydrogène ou un alkyle en C₁-C₆, Pr représente un groupe de protection de l'hydroxyle, et A est tel que défini dans la formule I ;
(b) la suppression des groupes de protection Pr de la formule IV avec un solvant organique pour former un deuxième intermédiaire de formule V : 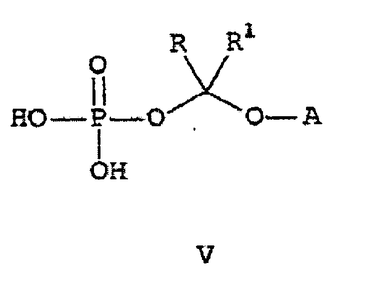 dans lequel R et R¹ dans la formule V sont chacun indépendamment un hydrogène ou un alkyle en C₁-C₆, et A est tel que défini dans la formule I ;
(c) la réaction dudit deuxième intermédiaire de formule V avec de la lysine dans un solvant à un pH dans la plage de 4,2 à 5,5 pour produire ledit sel de mono-lysine de formule I ; et
(d) la cristallisation dudit sel de mono-lysine dans un solvant pour produire le solvate dudit sel de mono-lysine.

20. Procédé selon la revendication 19, dans lequel le solvant à l'étape (d) est l'éthanol aqueux.

21. Procédé selon la revendication 19 ou 20, dans lequel le solvant à l'étape (d) est l'alcool isopropylique aqueux.

22. Procédé selon l'une quelconque des revendications 19 à 21, dans lequel le solvant à l'étape (d) est l'alcool n-propylique aqueux.

23. Procédé selon l'une quelconque des revendications 15 à 22, dans lequel A du matériau de départ A-OH est la formule (i) :

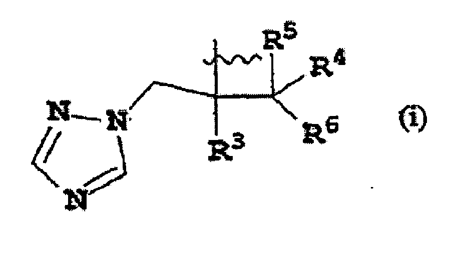

24. Sel de mono-lysine de phosphate dihydrogéné de ((2R, 3R)-3-(4-(4-cyanophényl)thiazol-2-yl)-2-(2,4-difluorophényl)-1-(1*H*-1,2,4-triazol-1-yl)butan-2-yloxy)méthyle ayant la structure : 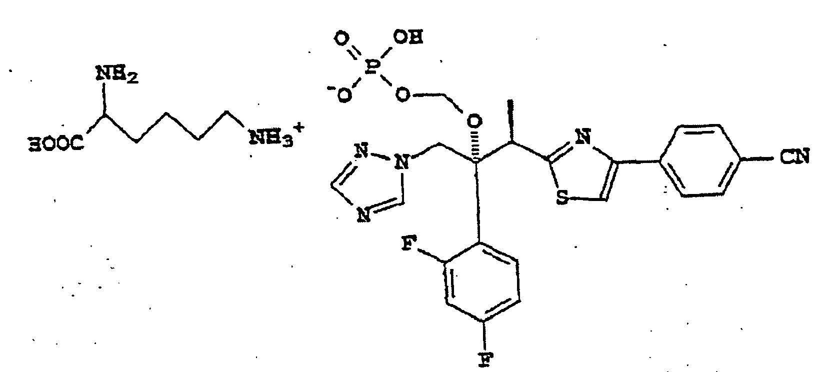 ou solvate pharmaceutiquement acceptable de celui-ci.

25. Sel de mono-lysine ou solvate de celui-ci selon la revendication 24, ledit sel ou solvate de celui-ci est sous une forme cristalline.

26. Solvate du sel de mono-lysine selon la revendication 24, dans lequel ledit solvate de celui-ci est un solvate d'éthanol.

27. Solvate du sel de mono-lysine selon la revendication 24, dans lequel ledit solvate de celui-ci est un solvate d'alcool isopropylique.

28. Solvate du sel de mono-lysine selon la revendication 24, dans lequel ledit solvate de celui-ci est un solvate d'alcool n-propylique.

29. Composition pharmaceutique comprenant :
une quantité efficace du sel de mono-lysine selon la revendication 24, ou du solvate pharmaceutiquement acceptable de celui-ci ; et
un adjuvant, diluant, ou support pharmaceutiquement acceptable.

30. Composition pharmaceutique selon la revendication 29, dans laquelle ladite composition est un comprimé, une gélule, une poudre, une solution, une suspension, une émulsion, un onguent, une lotion, une crème ou une pulvérisation.

31. Utilisation du sel de mono-lysine selon la revendication 24, ou du solvate pharmaceutiquement acceptable de celui-ci pour la préparation d'un médicament pour le traitement d'infections fongiques.

32. Utilisation selon la revendication 31, dans laquelle le médicament est administré par voie orale.

33. Utilisation selon la revendication 31, dans laquelle le médicament est administré par voie parentérale.

34. Procédé pour la préparation d'un sel de mono-lysine soluble dans l'eau de la formule suivante : 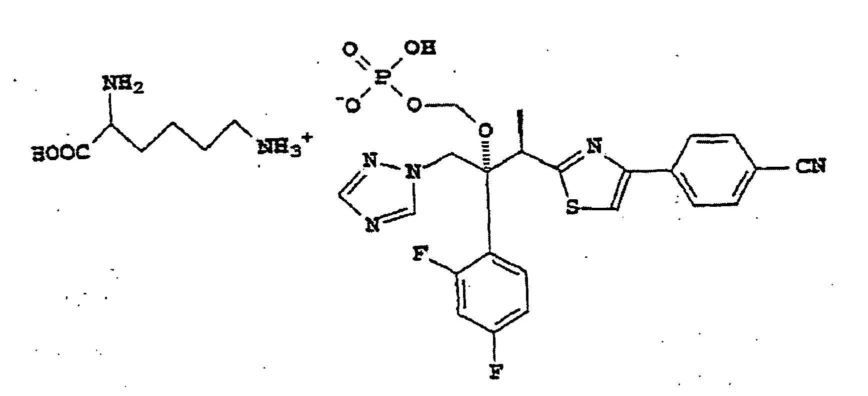 ledit procédé comprenant :
(a) la réaction d'un composé de formule B : 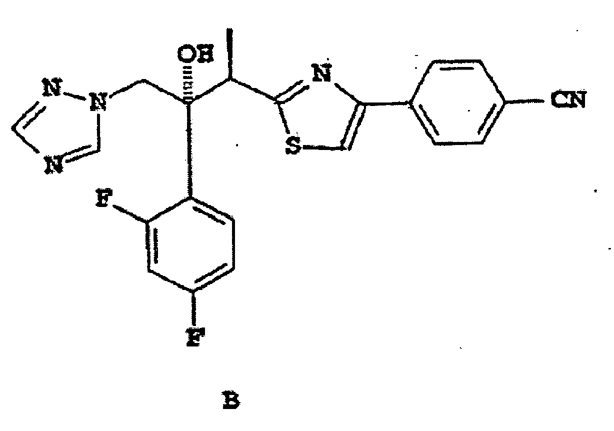 avec un composé de formule III' : 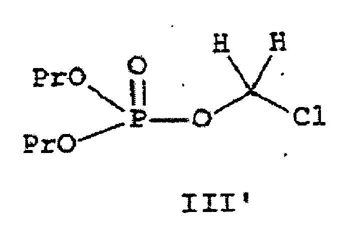 dans lequel Pr de la formule III' représente un groupe de protection de l'hydroxyle ;
ladite réaction est dans un solvant organique inerte en présence d'une base à une température d'environ 25°C à 50°C pour former un premier intermédiaire de formule IV' : 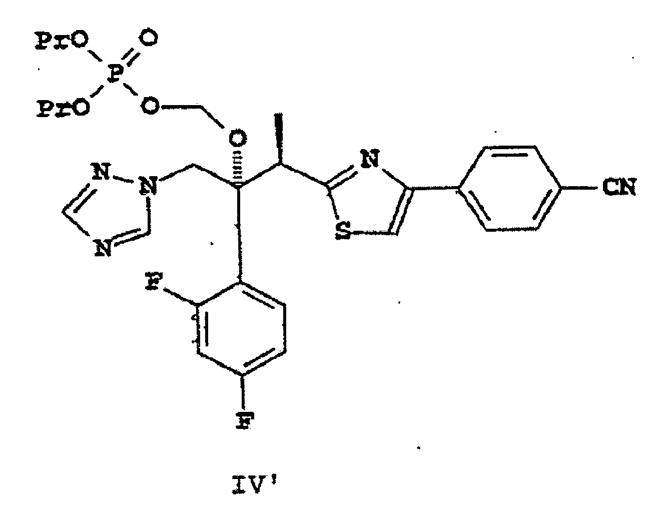 dans lequel Pr de la formule IV' représente un groupe de protection de l'hydroxyle ;
(b) la suppression des groupes de protection Pr de la formule IV' avec un solvant organique pour former un deuxième intermédiaire de formule V' : 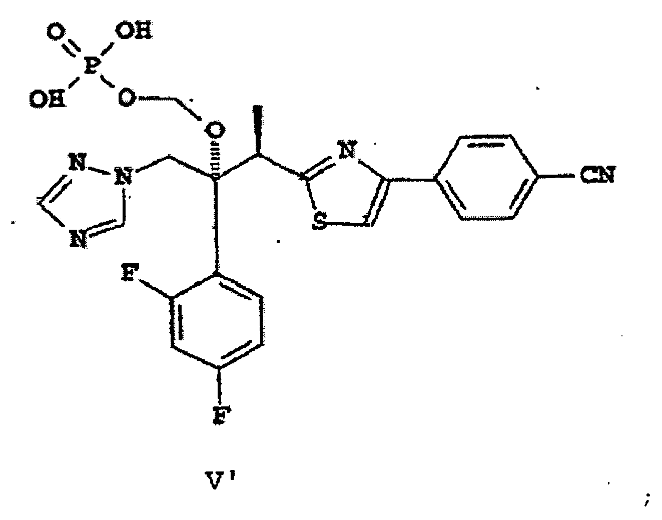 et
(c) la réaction dudit deuxième intermédiaire de formule V' avec de la lysine dans un solvant à un pH dans la plage de 4,2 à 5,5 pour produire ledit sel de mono-lysine.

35. Procédé selon la revendication 34, dans lequel le groupe de protection Pr est un butyle tertiaire.

36. Procédé selon la revendication 34 ou 35, dans lequel le solvant à l'étape (a) est le tétrahydrofurane.

37. Procédé selon l'une quelconque des revendications 34 à 36, dans lequel la base utilisée à l'étape (a) est l'hydrure de sodium.

38. Procédé pour la préparation d'un solvate soluble dans l'eau d'un sel de mono-lysine, ledit sel de mono-lysine ayant la formule : 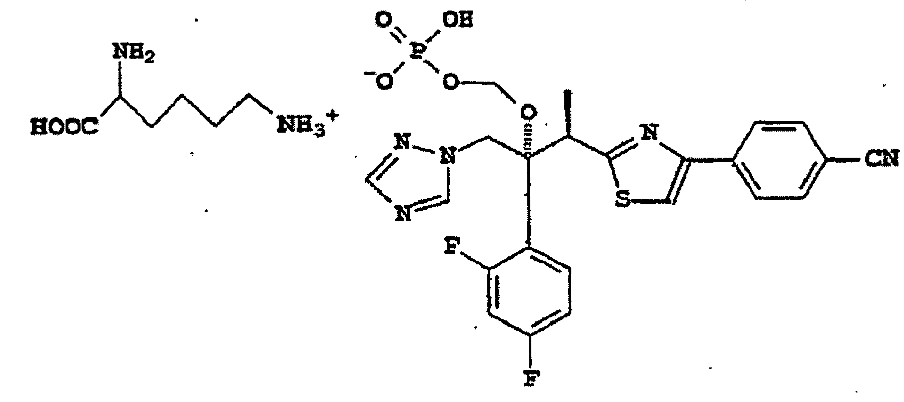 ledit procédé comprenant :
(a) la réaction d'un composé de formule B : 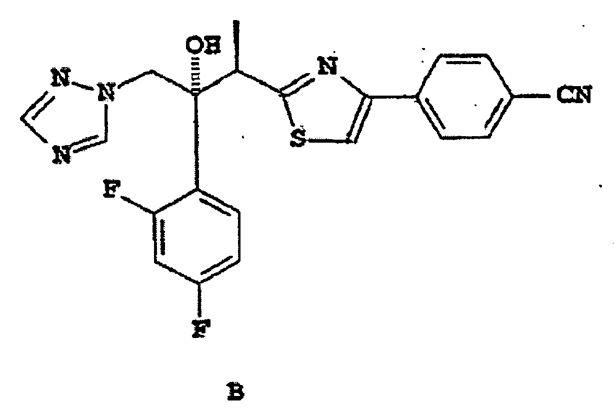 avec un composé de formule III' : 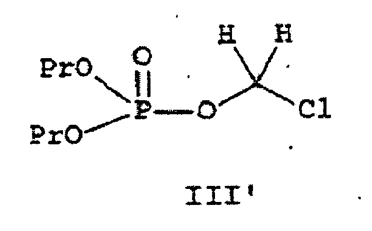 dans lequel Pr de la formule III' représente un groupe de protection de l'hydroxyle ; ladite réaction est dans un solvant organique inerte en présence d'une base à une température d'environ 25°C à 50°C pour former un premier intermédiaire de formule IV' : 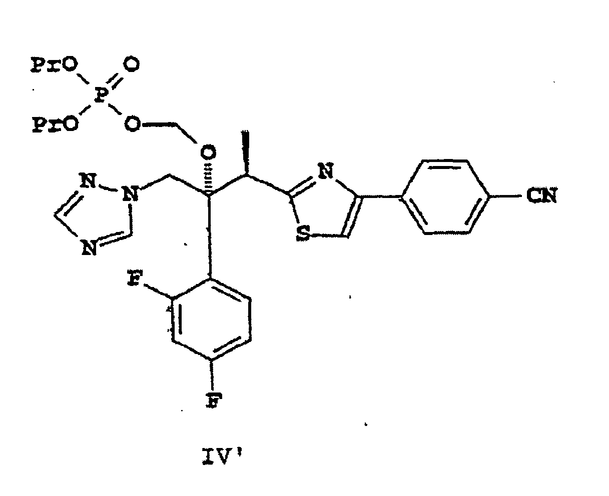 dans lequel Pr de la formule IV' représente un groupe de protection de l'hydroxyle ;
(b) la suppression des groupes de protection Pr de la formule IV' avec un solvant organique pour former un deuxième intermédiaire de formule V' : 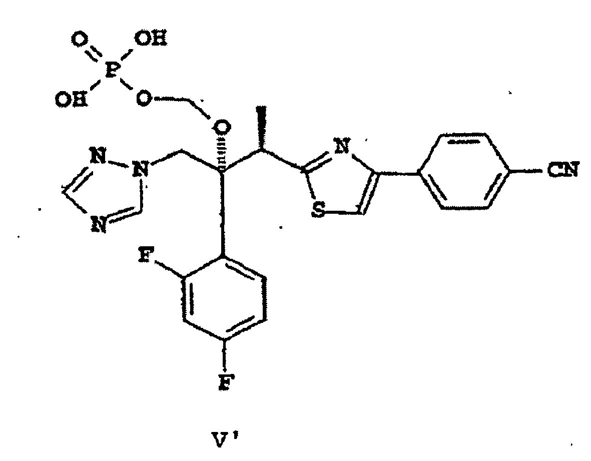
(c) la réaction dudit deuxième intermédiaire de formule V' avec de la lysine dans un solvant à un pH dans la plage de 4,2 à 5,5 pour produire ledit sel de mono-lysine ; et
(d) la cristallisation dudit sel de mono-lysine dans un solvant pour produire le solvate dudit sel de mono-lysine.

39. Procédé selon la revendication 38, dans lequel le solvant à l'étape (d) est l'éthanol aqueux.

40. Procédé selon la revendication 38 ou 39, dans lequel le solvant à l'étape (d) est l'alcool isopropylique aqueux.

41. Procédé selon l'une quelconque des revendications 38 à 40, dans lequel le solvant à l'étape (d) est l'alcool n-propylique aqueux.

42. Utilisation du sel de mono-lysine selon l'une quelconque des revendications 1 à 9 ou d'un solvate pharmaceutiquement acceptable de celui-ci pour la fabrication d'un agent pour traiter des infections fongiques.

43. Sel de mono-lysine selon la revendication 24 ou solvate pharmaceutiquement acceptable de celui-ci pour une utilisation dans un procédé pour le traitement d'infections fongiques.
